# EUROPÄISCHE PATENTSCHRIFT

(11) **EP 1 112 351 B1**
(45) Veröffentlichungstag und Bekanntmachung des Hinweises auf die Patenterteilung: **14.03.2007**
(21) Anmeldenummer: 99915607.8
(22) Anmeldetag: 15.03.1999
(51) Int. Cl.: C12N 9/00

(54) **POLYMERASENCHIMÄREN**
POLYMERASE CHIMERAS
CHIMERES DE POLYMERASES

(30) Priorität: 13.03.1998 DE 19810879
(43) Veröffentlichungstag der Anmeldung: 04.07.2001
(73) Patentinhaber: Roche Diagnostics GmbH, 68305 Mannheim (DE)
(72) Erfinder: FREY, Bruno, D-82377 Penzberg (DE); VILLBRANDT, Britta, D-38102 Braunschweig (DE); SCHOMBURG, Dietmar, D-50374 Erftstadt (DE); SOBEK, Harald, D-82377 Penzberg (DE); ANKENBAUER, Waltraud, D-82377 Penzberg (DE)
(86) Internationale Anmeldenummer: PCT/EP1999/001674
(87) Internationale Veröffentlichungsnummer: WO 1999/047649

(56) Entgegenhaltungen:
- EP-A- 0 482 714
- EP-A- 0 892 058
- WO-A-97/29209
- US-A- 5 466 591
- RUI SOUSA ET AL: "SINGLE CRYSTALS OF A CHIMERIC T7/T3 RNA POLYMERASE WITH T3 PROMOTER SPECIFICITY" JOURNAL OF CRYSTAL GROWTH, Bd. 122, Nr. 1 / 04, 2. August 1992, Seiten 366-374, XP000306506

### SEQUENZPROTOKOLL

(1) ALLGEMEINE ANGABEN:
   (i) ANMELDER:
      (A) NAME: Boehringer Mannheim GmbH
      (B) STRASSE: Sandhoferstr. 116
      (C) ORT: Mannheim
      (E) LAND: DE
      (F) POSTLEITZAHL: 68305
      (G) TELEFON: 06217595482
      (H) TELEFAX: 06217594457
   (ii) BEZEICHNUNG DER ERFINDUNG: Polymerasenchimaeren
   (iii) ANZAHL DER SEQUENZEN: 14
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.30 (EPA)
(2) ANGABEN ZU SEQ ID NO: 1:
   (i) SEQUENZKENNZEICHEN:
      (A) LÄNGE: 2733 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 1:
   ATGAGGGGCT CGCATCACCA TCACCATCAC GCTGCTGACG ATGACGATAA AATGAGGGGC   60
   ATGCTACCGC TATTTGAGCC CAAGGGCCGG GTCCTCCTGG TCGACGGCCA CCACCTGGCC  120
   TACCGCACCT TCCACGCCCT GAAGGGCCTC ACCACCAGCC GGGGGGAGCC GGTGCAGGCG  180
   GTCTACGGCT TCGCCAAGAG CCTCCTCAAG GCCCTCAAGG AGGACGGGGA CGCGGTGATC  240
   GTGGTCTTTG ACGCCAAGGC CCCCTCCTTC CGCCACGAGG CCTACGGGGG GTACAAGGCG  300
   GGCCGGGCCC CCACGCCGGA GGACTTTCCC CGGCAACTCG CCCTCATCAA GGAGCTGGTG  360
   GACCTCCTGG GGCTGGCGCG CCTCGAGGTC CCGGGCTACG AGGCGGACGA CGTCCTGGCC  420
   AGCCTGGCCA AGAAGGCGGA AAAGGAGGGC TACGAGGTCC GCATCCTCAC CGCCGACAAA 480
   GACCTTTACC AGCTCCTTTC CGACCGCATC CACGTCCTCC ACCCCGAGGG GTACCTCATC 540
   ACCCCGGCCT GGCTTTGGGA AAAGTACGGC CTGAGGCCCG ACCAGTGGGC CGACTACCGG 600
   GCCCTGACCG GGGACGAGTC CGACAACCTT CCCGGGGTCA AGGGCATCGG GGAGAAGACG 660
   GCGAGGAAGC TTCTGGAGGA GTGGGGGAGC CTGGAAGCCC TCCTCAAGAA CCTGGACCGG 720
   CTGAAGCCCG CCATCCGGGA GAAGATCCTG GCCCACATGG ACGATCTGAA GCTCTCCTGG 780
   GACCTGGCCA AGGTGCGCAC CGACCTGCCC CTGGAGGTGG ACTTCGCCAA AAGGCGGGAG 840
   CCCGACCGGG AGAGGCTTAG GGCCTTTCTG GAGAGGCTTG AGTTTGGCAG CCTCCTCCAC 900
   GAGTTCGGCC TTCTGGAAAG CCCCTATGAC AACTACGTCA CCATCCTTGA TGAAGAAACA 960
   CTGAAAGCGT GGATTGCGAA GCTGGAAAAA GCGCCGGTAT TTGCATTTGA TACCGAAACC 1020
   GACAGCCTTG ATAACATCTC TGCTAACCTG GTCGGGCTTT CTTTTGCTAT CGAGCCAGGC 1080
   GTAGCGGCAT ATATTCCGGT TGCTCATGAT TATCTTGATG CGCCCGATCA AATCTCTCGC 1140
   GAGCGTGCAC TCGAGTTGCT AAAACCGCTG CTGGAAGATG AAAAGGCGCT GAAGGTCGGG 1200
   CAAAACCTGA AATACGATCG CGGTATTCTG GCGAACTACG GCATTGAACT GCGTGGGATT 1260
   GCGTTTGATA CCATGCTGGA GTCCTACATT CTCAATAGCG TTGCCGGGCG TCACGATATG 1320
   GACAGCCTCG CGGAACGTTG GTTGAAGCAC AAAACCATCA CTTTTGAAGA GATTGCTGGT 1380
   AAAGGCAAAA ATCAACTGAC CTTTAACCAG ATTGCCCTCG AAGAAGCCGG ACGTTACGCC 1440
   GCCGAAGATG CAGATGTCAC CTTGCAGTTG CATCTGAAAA TGTGGCCGGA TCTGCAAAAA 1500
   CACGAGAGGC TCCTTTGGCT TTACCGGGAG GTGGAGAGGC CCCTTTCCGC TGTCCTGGCC 1560
   CACATGGAGG CCACGGGGGT GCGCCTGGAC GTGGCCTATC TCAGGGCCTT GTCCCTGGAG 1620
   GTGGCCGAGG AGGTCGCCCG CCTCGAGGCC GAGGTCTTCC GCCTGGCCGG CCACCCCTTC 1680
   AACCTCAACT CCCGGGACCA GCTGGAAAGG GTCCTCTTTG ACGAGCTAGG GCTTCCCGCC 1740
   ATCGGCAAGA CGGAGAAGAC CGGCAAGCGC TCCACCAGCG CCGCCGTCCT GGAGGCCCTC 1800
   CGCGAGGCCC ACCCCATCGT GGAGAAGATC CTGCAGTACC GGGAGCTCAC CAAGCTGAAG 1860
   AGCACCTACA TTGACCCCTT GCCGGACCTC ATCCACCCCA GGACGGGCCG CCTCCACACC 1920
   CGCTTCAACC AGACGGCCAC GGCCACGGGC AGGCTAAGTA GCTCCGATCC CAACCTCCAG 1980
   AACATCCCCG TCCGCACCCC GCTTGGGCAG AGGATCCGCC GGGCCTTCAT CGCCGAGGAG 2040
   GGGTGGCTAT TGGTGGCCCT GGACTATAGC CAGATAGAGC TCAGGGTGCT GGCCCACCTC 2100
   TCCGGCGACG AGAACCTGAT CCGGGTCTTC CAGGAGGGGC GGGACATCCA CACGGAGACC 2160
   GCCAGCTGGA TGTTCGGCGT CCCCCGGGAG GCCGTGGACC CCCTGATGCG CCGGGCGGCC 2220
   AAGACCATCA ACTTCGGGGT CCTCTACGGC ATGTCGGCCC ACCGCCTCTC CCAGGAGCTA 2280
   GCCATCCCTT ACGAGGAGGC CCAGGCCTTC ATTGAGCGCT ACTTTCAGAG CTTCCCCAAG 2340
   GTGCGGGCCT GGATTGAGAA GACCCTGGAG GAGGGCAGGA GGCGGGGGTA CGTGGAGACC 2400
   CTCTTCGGCC GCCGCCGCTA CGTGCCAGAC CTAGAGGCCC GGGTGAAGAG CGTGCGGGAG 2460
   GCGGCCGAGC GCATGGCCTT CAACATGCCC GTCCAGGGCA CCGCCGCCGA CCTCATGAAG 2520
   CTGGCTATGG TGAAGCTCTT CCCCAGGCTG GAGGAAATGG GGGCCAGGAT GCTCCTTCAG 2580
   GTCCACGACG AGCTGGTCCT CGAGGCCCCA AAAGAGAGGG CGGAGGCCGT GGCCCGGCTG 2640
   GCCAAGGAGG TCATGGAGGG GGTGTATCCC CTGGCCGTGC CCCTGGAGGT GGAGGTGGGG 2700
   ATAGGGGAGG ACTGGCTCTC CGCCAAGGAG TGA 2733
(2) ANGABEN ZU SEQ ID NO: 2:
   (i) SEQUENZKENNZEICHEN:
      (A) LÄNGE: 2733 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 2:
   ATGAGGGGCT CGCATCACCA TCACCATCAC GCTGCTGACG ATGACGATAA AATGAGGGGC 60
   ATGCTACCGC TATTTGAGCC CAAGGGCCGG GTCCTCCTGG TCGACGGCCA CCACCTGGCC 120
   TACCGCACCT TCCACGCCCT GAAGGGCCTC ACCACCAGCC GGGGGGAGCC GGTGCAGGCG 180
   GTCTACGGCT TCGCCAAGAG CCTCCTCAAG GCCCTCAAGG AGGACGGGGA CGCGGTGATC 240
   GTGGTCTTTG ACGCCAAGGC CCCCTCCTTC CGCCACGAGG CCTACGGGGG GTACAAGGCG 300
   GGCCGGGCCC CCACGCCGGA GGACTTTCCC CGGCAACTCG CCCTCATCAA GGAGCTGGTG  360
   GACCTCCTGG GGCTGGCGCG CCTCGAGGTC CCGGGCTACG AGGCGGACGA CGTCCTGGCC  420
   AGCCTGGCCA AGAAGGCGGA AAAGGAGGGC TACGAGGTCC GCATCCTCAC CGCCGACAAA  480
   GACCTTTACC AGCTCCTTTC CGACCGCATC CACGTCCTCC ACCCCGAGGG GTACCTCATC  540
   ACCCCGGCCT GGCTTTGGGA AAAGTACGGC CTGAGGCCCG ACCAGTGGGC CGACTACCGG  600
   GCCCTGACCG GGGACGAGTC CGACAACCTT CCCGGGGTCA AGGGCATCGG GGAGAAGACG  660
   GCGAGGAAGC TTCTGGAGGA GTGGGGGAGC CTGGAAGCCC TCCTCAAGAA CCTGGACCGG  720
   CTGAAGCCCG CCATCCGGGA GAAGATCCTG GCCCACATGG ACGATCTGAA GCTCTCCTGG  780
   GACCTGGCCA AGGTGCGCAC CGACCTGCCC CTGGAGGTGG ACTTCGCCAA AAGGCGGGAG  840
   CCCGACCGGG AGAGGCTTAG GGCCTTTCTG GAGAGGCTTG AGTTTGGCAG CCTCCTCCAC  900
   GAGTTCGGCC TTCTGGAAAG CCCCTATGAC AACTACGTCA CCATCCTTGA TGAAGAAACA  960
   CTGAAAGCGT GGATTGCGAA GCTGGAAAAA GCGCCGGTAT TTGCATTTGA TACCGAAACC 1020
   GACAGCCTTG ATAACATCTC TGCTAACCTG GTCGGGCTTT CTTTTGCTAT CGAGCCAGGC 1080
   GTAGCGGCAT ATATTCCGGT TGCTCATGAT TATCTTGATG CGCCCGATCA AATCTCTCGC 1140
   GAGCGTGCAC TCGAGTTGCT AAAACCGCTG CTGGAAGATG AAAAGGCGCT GAAGGTCGGG 1200
   CAAAACCTGA AATACGATCG CGGTATTCTG GCGAACTACG GCATTGAACT GCGTGGGATT 1260
   GCGTTTGATA CCATGCTGGA GTCCTACATT CTCAATAGCG TTGCCGGGCG TCACGATATG 1320
   GACAGCCTCG CGGAACGTTG GTTGAAGCAC AAAACCATCA CTTTTGAAGA GATTGCTGGT 1380
   AAAGGCAAAA ATCAACTGAC CTTTAACCAG ATTGCCCTCG AAGAAGCCGG ACGTTACGCC 1440
   GCCGAAGATG CAGATGTCAC CTTGCAGTTG CATCTGAAAA TGTGGCCGGA TCTGCAAAAA 1500
   CACAAAGGGC CGTTGAACGT CTTCGAGAAT ATCGAAATGC CGCTGGTGCC GGTGCTTTCA 1560
   CGCATTGAAC GTAACGGTGT GCGCCTGGAC GTGGCCTATC TCAGGGCCTT GTCCCTGGAG 1620
   GTGGCCGAGG AGATCGCCCG CCTCGAGGCC GAGGTCTTCC GCCTGGCCGG CCACCCCTTC 1680
   AACCTCAACT CCCGGGACCA GCTGGAAAGG GTCCTCTTTG ACGAGCTAGG GCTTCCCGCC 1740
   ATCGGCAAGA CGGAGAAGAC CGGCAAGCGC TCCACCAGCG CCGCCGTCCT GGAGGCCCTC 1800
   CGCGAGGCCC ACCCCATCGT GGAGAAGATC CTGCAGTACC GGGAGCTCAC CAAGCTGAAG 1860
   AGCACCTACA TTGACCCCTT GCCGGACCTC ATCCACCCCA GGACGGGCCG CCTCCACACC 1920
   CGCTTCAACC AGACGGCCAC GGCCACGGGC AGGCTAAGTA GCTCCGATCC CAACCTCCAG 1980
   AACATCCCCG TCCGCACCCC GCTTGGGCAG AGGATCCGCC GGGCCTTCAT CGCCGAGGAG 2040
   GGGTGGCTAT TGGTGGCCCT GGACTATAGC CAGATAGAGC TCAGGGTGCT GGCCCACCTC 2100
   TCCGGCGACG AGAACCTGAT CCGGGTCTTC CAGGAGGGGC GGGACATCCA CACGGAGACC 2160
   GCCAGCTGGA TGTTCGGCGT CCCCCGGGAG GCCGTGGACC CCCTGATGCG CCGGGCGGCC 2220
   AAGACCATCA ACTTCGGGGT CCTCTACGGC ATGTCGGCCC ACCGCCTCTC CCAGGAGCTA 2280
   GCCATCCCTT ACGAGGAGGC CCAGGCCTTC ATTGAGCGCT ACTTTCAGAG CTTCCCCAAG 2340
   GTGCGGGCCT GGATTGAGAA GACCCTGGAG GAGGGCAGGA GGCGGGGGTA CGTGGAGACC 2400
   CTCTTCGGCC GCCGCCGCTA CGTGCCAGAC CTAGAGGCCC GGGTGAAGAG CGTGCGGGAG 2460
   GCGGCCGAGC GCATGGCCTT CAACATGCCC GTCCAGGGCA CCGCCGCCGA CCTCATGAAG 2520
   CTGGCTATGG TGAAGCTCTT CCCCAGGCTG GAGGAAATGG GGGCCAGGAT GCTCCTTCAG 2580
   GTCCACGACG AGCTGGTCCT CGAGGCCCCA AAAGAGAGGG CGGAGGCCGT GGCCCGGCTG 2640
   GCCAAGGAGG TCATGGAGGG GGTGTATCCC CTGGCCGTGC CCCTGGAGGT GGAGGTGGGG 2700
   ATAGGGGAGG ACTGGCTCTC CGCCAAGGAG TGA 2733
(2) ANGABEN ZU SEQ ID NO: 3:
   (i) SEQUENZKENNZEICHEN:
      (A) LÄNGE: 2727 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 3:
   ATGAGGGGCT CGCATCACCA TCACCATCAC GCTGCTGACG ATGACGATAA AATGAGGGGC 60
   ATGCTACCGC TATTTGAGCC CAAGGGCCGG GTCCTCCTGG TCGACGGCCA CCACCTGGCC 120
   TACCGCACCT TCCACGCCCT GAAGGGCCTC ACCACCAGCC GGGGGGAGCC GGTGCAGGCG 180
   GTCTACGGCT TCGCCAAGAG CCTCCTCAAG GCCCTCAAGG AGGACGGGGA CGCGGTGATC 240
   GTGGTCTTTG ACGCCAAGGC CCCCTCCTTC CGCCACGAGG CCTACGGGGG GTACAAGGCG 300
   GGCCGGGCCC CCACGCCGGA GGACTTTCCC CGGCAACTCG CCCTCATCAA GGAGCTGGTG 360
   GACCTCCTGG GGCTGGCGCG CCTCGAGGTC CCGGGCTACG AGGCGGACGA CGTCCTGGCC 420
   AGCCTGGCCA AGAAGGCGGA AAAGGAGGGC TACGAGGTCC GCATCCTCAC CGCCGACAAA 480
   GACCTTTACC AGCTCCTTTC CGACCGCATC CACGTCCTCC ACCCCGAGGG GTACCTCATC 540
   ACCCCGGCCT GGCTTTGGGA AAAGTACGGC CTGAGGCCCG ACCAGTGGGC CGACTACCGG 600
   GCCCTGACCG GGGACGAGTC CGACAACCTT CCCGGGGTCA AGGGCATCGG GGAGAAGACG 660
   GCGAGGAAGC TTCTGGAGGA GTGGGGGAGC CTGGAAGCCC TCCTCAAGAA CCTGGACCGG 720
   CTGAAGCCCG CCATCCGGGA GAAGATCCTG GCCCACATGG ACGATCTGAA GCTCTCCTGG 780
   GACCTGGCCA AGGTGCGCAC CGACCTGCCC CTGGAGGTGG ACTTCGCCAA AAGGCGGGAG 840
   CCCGACCGGG AGAGGCTTAG GGCCTTTCTG GAGAGGCTTG AGTTTGGCAG CCTCCTCCAC 900
   GAGTTCGGCC TTCTGGAAAG CCCCCCCGTT GGATACAGAA TAGTGAAAGA CCTGGTGGAA 960
   TTTGAAAAAC TCATAGAGAA ACTGAGAGAA TCCCCTTCGT TCGCCATAGA TCTTGAGACG 1020
   TCTTCCCTCG ATCCTTTCGA CTGCGACATT GTCGGTATCT CTGTGTCTTT CAAACCAAAG 1080
   GAAGCGTACT ACATACCACT CCATCATAGA AACGCCCAGA ACCTGGATGA AAAAGAAGTT 1140
   CTGAAAAAGC TAAAAGAAAT CCTGGAGGAC CCCGGAGCAA AGATCGTTGG TCAGAATTTG 1200
   AAATTCGATT ACAAGGTGTT GATGGTAAAG GGTGTTGAAC CTGTCCCTCC TCACTTCGAC 1260
   ACGATGATAG CGGCTTACCT TCTTGAGCCG AACGAAAAGA AGTTCAATCT GGACGATCTC 1320
   GCATTGAAAT TTCTTGGATA CAAAATGACC TCTTACCAGG AACTCATGTC CTTCTCTTCT 1380
   CCGCTGTTTG GTTTCAGTTT TGCCGATGTT CCTGTAGAAA AAGCAGCGAA CTATTCCTGT 1440
   GAAGATGCCG ACATCACCTA CAGACTCTAC AAGATCCTGA GCTTAAAACT CCACGAGGAG 1500
   AGGCTCCTTT GGCTTTACCG GGAGGTGGAG AGGCCCCTTT CCGCTGTCCT GGCCCACATG 1560
   GAGGCCACGG GGGTGCGCCT GGACGTGGCC TATCTCAGGG CCTTGTCCCT GGAGGTGGCC 1620
   GAGGAGATCG CCCGCCTCGA GGCCGAGGTC TTCCGCCTGG CCGGCCACCC CTTCAACCTC 1680
   AACTCCCGGG ACCAGCTGGA AAGGGTCCTC TTTGACGAGC TAGGGCTTCC CGCCATCGGC 1740
   AAGACGGAGA AGACCGGCAA GCGCTCCACC AGCGCCGCCG TCCTGGAGGC CCTCCGCGAG 1800
   GCCCACCCCA TCGTGGAGAA GATCCTGCAG TACCGGGAGC TCACCAAGCT GAAGAGCACC 1860
   TACATTGACC CCTTGCCGGA CCTCATCCAC CCCAGGACGG GCCGCCTCCA CACCCGCTTC 1920
   AACCAGACGG CCACGGCCAC GGGCAGGCTA AGTAGCTCCG ATCCCAACCT CCAGAACATC 1980
   CCCGTCCGCA CCCCGCTTGG GCAGAGGATC CGCCGGGCCT TCATCGCCGA GGAGGGGTGG 2040
   CTATTGGTGG CCCTGGACTA TAGCCAGATA GAGCTCAGGG TGCTGGCCCA CCTCTCCGGC 2100
   GACGAGAACC TGATCCGGGT CTTCCAGGAG GGGCGGGACA TCCACACGGA GACCGCCAGC 2160
   TGGATGTTCG GCGTCCCCCG GGAGGCCGTG GACCCCCTGA TGCGCCGGGC GGCCAAGACC 2220
   ATCAACTTCG GGGTCCTCTA CGGCATGTCG GCCCACCGCC TCTCCCAGGA GCTAGCCATC 2280
   CCTTACGAGG AGGCCCAGGC CTTCATTGAG CGCTACTTTC AGAGCTTCCC CAAGGTGCGG 2340
   GCCTGGATTG AGAAGACCCT GGAGGAGGGC AGGAGGCGGG GGTACGTGGA GACCCTCTTC 2400
   GGCCGCCGCC GCTACGTGCC AGACCTAGAG GCCCGGGTGA AGAGCGTGCG GGAGGCGGCC 2460
   GAGCGCATGG CCTTCAACAT GCCCGTCCAG GGCACCGCCG CCGACCTCAT GAAGCTGGCT 2520
   ATGGTGAAGC TCTTCCCCAG GCTGGAGGAA ATGGGGGCCA GGATGCTCCT TCAGGTCCAC 2580
   GACGAGCTGG TCCTCGAGGC CCCAAAAGAG AGGGCGGAGG CCGTGGCCCG GCTGGCCAAG 2640
   GAGGTCATGG AGGGGGTGTA TCCCCTGGCC GTGCCCCTGG AGGTGGAGGT GGGGATAGGG 2700
   GAGGACTGGC TCTCCGCCAA GGAGTGA 2727
(2) ANGABEN ZU SEQ ID NO: 4:
   (i) SEQUENZKENNZEICHEN:
      (A) LÄNGE: 2727 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 4:
   ATGAGGGGCT CGCATCACCA TCACCATCAC GCTGCTGACG ATGACGATAA AATGAGGGGC 60
   ATGCTACCGC TATTTGAGCC CAAGGGCCGG GTCCTCCTGG TCGACGGCCA CCACCTGGCC 120
   TACCGCACCT TCCACGCCCT GAAGGGCCTC ACCACCAGCC GGGGGGAGCC GGTGCAGGCG 180
   GTCTACGGCT TCGCCAAGAG CCTCCTCAAG GCCCTCAAGG AGGACGGGGA CGCGGTGATC 240
   GTGGTCTTTG ACGCCAAGGC CCCCTCCTTC CGCCACGAGG CCTACGGGGG GTACAAGGCG 300
   GGCCGGGCCC CCACGCCGGA GGACTTTCCC CGGCAACTCG CCCTCATCAA GGAGCTGGTG 360
   GACCTCCTGG GGCTGGCGCG CCTCGAGGTC CCGGGCTACG AGGCGGACGA CGTCCTGGCC 420
   AGCCTGGCCA AGAAGGCGGA AAAGGAGGGC TACGAGGTCC GCATCCTCAC CGCCGACAAA 480
   GACCTTTACC AGCTCCTTTC CGACCGCATC CACGTCCTCC ACCCCGAGGG GTACCTCATC 540
   ACCCCGGCCT GGCTTTGGGA AAAGTACGGC CTGAGGCCCG ACCAGTGGGC CGACTACCGG 600
   GCCCTGACCG GGGACGAGTC CGACAACCTT CCCGGGGTCA AGGGCATCGG GGAGAAGACG 660
   GCGAGGAAGC TTCTGGAGGA GTGGGGGAGC CTGGAAGCCC TCCTCAAGAA CCTGGACCGG 720
   CTGAAGCCCG CCATCCGGGA GAAGATCCTG GCCCACATGG ACGATCTGAA GCTCTCCTGG 780
   GACCTGGCCA AGGTGCGCAC CGACCTGCCC CTGGAGGTGG ACTTCGCCAA AAGGCGGGAG 840
   CCCGACCGGG AGAGGCTTAG GGCCTTTCTG GAGAGGCTTG AGTTTGGCAG CCTCCTCCAC 900
   GAGTTCGGCC TTCTGGAAAG CCCCCCCGTT GGATACAGAA TAGTGAAAGA CCTGGTGGAA 960
   TTTGAAAAAC TCATAGAGAA ACTGAGAGAA TCCCCTTCGT TCGCCATAGA TCTTGAGACG 1020
   TCTTCCCTCG ATCCTTTCGA CTGCGACATT GTCGGTATCT CTGTGTCTTT CAAACCAAAG 1080
   GAAGCGTACT ACATACCACT CCATCATAGA AACGCCCAGA ACCTGGATGA AAAAGAAGTT 1140
   CTGAAAAAGC TAAAAGAAAT CCTGGAGGAC CCCGGAGCAA AGATCGTTGG TCAGAATTTG 1200
   AAATTCGATT ACAAGGTGTT GATGGTAAAG GGTGTTGAAC CTGTCCCTCC TCACTTCGAC 1260
   ACGATGATAG CGGCTTACCT TCTTGAGCCG AACGAAAAGA AGTTCAATCT GGACGATCTC 1320
   GCATTGAAAT TTCTTGGATA CAAAATGACC TCTTACCAGG AACTCATGTC CTTCTCTTCT 1380
   CCGCTGTTTG GTTTCAGTTT TGCCGATGTT CCTGTAGAAA AAGCAGCGAA CTATTCCTGT 1440
   GAAGATGCAG ACATCACCTA CAGACTCTAC AAGATCCTGA GCTTAAAACT CCACGAGGCA 1500
   GATCTGGAGA ACGTGTTCTA CAAGATAGAA ATGCCTCTTG TGAGCGTGCT TGCACGGATG 1560
   GAACTGAACG GTGTGCGCCT GGACGTGGCC TATCTCAGGG CCTTGTCCCT GGAGGTGGCC 1620
   GAGGAGATCG CCCGCCTCGA GGCCGAGGTC TTCCGCCTGG CCGGCCACCC CTTCAACCTC 1680
   AACTCCCGGG ACCAGCTGGA AAGGGTCCTC TTTGACGAGC TAGGGCTTCC CGCCATCGGC 1740
   AAGACGGAGA AGACCGGCAA GCGCTCTACC AGCGCCGCCG TCCTGGAGGC CCTCCGCGAG 1800
   GCCCACCCCA TCGTGGAGAA GATCCTGCAG TACCGGGAGC TCACCAAGCT GAAGAGCACC 1860
   TACATTGACC CCTTGCCGGA CCTCATCCAC CCCAGGACGG GCCGCCTCCA CACCCGCTTC 1920
   AACCAGACGG CCACGGCCAC GGGCAGGCTA AGTAGCTCCG ATCCCAACCT CCAGAACATC 1980
   CCCGTCCGCA CCCCGCTTGG GCAGAGGATC CGCCGGGCCT TCATCGCCGA GGAGGGGTGG 2040
   CTATTGGTGG CCCTGGACTA TAGCCAGATA GAGCTCAGGG TGCTGGCCCA CCTCTCCGGC 2100
   GACGAGAACC TGATCCGGGT CTTCCAGGAG GGGCGGGACA TCCACACGGA GACCGCCAGC 2160
   TGGATGTTCG GCGTCCCCCG GGAGGCCGTG GACCCCCTGA TGCGCCGGGC GGCCAAGACC 2220
   ATCAACTTCG GGGTCCTCTA CGGCATGTCG GCCCACCGCC TCTCCCAGGA GCTAGCCATC 2280
   CCTTACGAGG AGGCCCAGGC CTTCATTGAG CGCTACTTTC AGAGCTTCCC CAAGGTGCGG 2340
   GCCTGGATTG AGAAGACCCT GGAGGAGGGC AGGAGGCGGG GGTACGTGGA GACCCTCTTC 2400
   GGCCGCCGCC GCTACGTGCC AGACCTAGAG GCCCGGGTGA AGAGCGTGCG GGAGGCGGCC 2460
   GAGCGCATGG CCTTCAACAT GCCCGTCCAG GGCACCGCCG CCGACCTCAT GAAGCTGGCT 2520
   ATGGTGAAGC TCTTCCCCAG GCTGGAGGAA ATGGGGGCCA GGATGCTCCT TCAGGTCCAC 2580
   GACGAGCTGG TCCTCGAGGC CCCAAAAGAG AGGGCGGAGG CCGTGGCCCG GCTGGCCAAG 2640
   GAGGTCATGG AGGGGGTGTA TCCCCTGGCC GTGCCCCTGG AGGTGGAGGT GGGGATAGGG 2700
   GAGGACTGGC TCTCCGCCAA GGAGTGA                                    2727
(2) ANGABEN ZU SEQ ID NO: 5:
   (i) SEQUENZKENNZEICHEN:
      (A) LÄNGE: 2850 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 5:
   ATGAGGGGCT CGCATCACCA TCACCATCAC GCTGCTGACG ATGACGATAA AATGAGGGGC   60
   ATGCTACCGC TATTTGAGCC CAAGGGCCGG GTCCTCCTGG TCGACGGCCA CCACCTGGCC  120
   TACCGCACCT TCCACGCCCT GAAGGGCCTC ACCACCAGCC GGGGGGAGCC GGTGCAGGCG  180
   GTCTACGGCT TCGCCAAGAG CCTCCTCAAG GCCCTCAAGG AGGACGGGGA CGCGGTGATC  240
   GTGGTCTTTG ACGCCAAGGC CCCCTCCTTC CGCCACGAGG CCTACGGGGG GTACAAGGCG  300
   GGCCGGGCCC CCACGCCGGA GGACTTTCCC CGGCAACTCG CCCTCATCAA GGAGCTGGTG  360
   GACCTCCTGG GGCTGGCGCG CCTCGAGGTC CCGGGCTACG AGGCGGACGA CGTCCTGGCC  420
   AGCCTGGCCA AGAAGGCGGA AAAGGAGGGC TACGAGGTCC GCATCCTCAC CGCCGACAAA  480
   GACCTTTACC AGCTCCTTTC CGACCGCATC CACGTCCTCC ACCCCGAGGG GTACCTCATC  540
   ACCCCGGCCT GGCTTTGGGA AAAGTACGGC CTGAGGCCCG ACCAGTGGGC CGACTACCGG  600
   GCCCTGACCG GGGACGAGTC CGACAACCTT CCCGGGGTCA AGGGCATCGG GGAGAAGACG  660
   GCGAGGAAGC TTCTGGAGGA GTGGGGGAGC CTGGAAGCCC TCCTCAAGAA CCTGGACCGG  720
   CTGAAGCCCG CCATCCGGGA GAAGATCCTG GCCCACATGG ACGATCTGAA GCTCTCCTGG  780
   GACCTGGCCA AGGTGCGCAC CGACCTGCCC CTGGAGGTGG ACTTCGCCAA AAGGCGGGAG  840
   CCCGACCGGG AGAGGCTTAG GGCCTTTCTG GAGAGGCTTG AGTTTGGCAG CCTCCTCCAC  900
   GAGTTCGGCC TTCTGGAAAG CCCCCATCCA GCAGTTGTGG ACATCTTCGA ATACGATATT  960
   CCATTTGCAA AGAGATACCT CATCGACAAA GGCCTAATAC CAATGGAGGG GGAAGAAGAG 1020
   CTAAAGATTC TTGCCTTCGA TATAGAAACC CTCTATCACG AAGGAGAAGA GTTTGGAAAA 1080
   GGCCCAATTA TAATGATTAG TTATGCAGAT GAAAATGAAG CAAAGGTGAT TACTTGGAAA 1140
   AACATAGATC TTCCATACGT TGAGGTTGTA TCAAGCGAGA GAGAGATGAT AAAGAGATTT 1200
   CTCAGGATTA TCAGGGAGAA GGATCCTGAC ATTATAGTTA CTTATAATGG AGACTCATTC 1260
   GACTTCCCAT ATTTAGCGAA AAGGGCAGAA AAACTTGGGA TTAAATTAAC CATTGGAAGA 1320
   GATGGAAGCG AGCCCAAGAT GCAGAGAATA GGCGATATGA CGGCTGTAGA AGTCAAGGGA 1380
   AGAATACATT TCGACTTGTA TCATGTAATA ACAAGGACAA TAAATCTCCC AACATACACA 1440
   CTAGAGGCTG TATATGAAGC AATTTTTGGA AAGCCAAAGG AGAAGGTATA CGCCGACGAG 1500
   ATAGCAAAAG CCTGGGAAAG TGGAGAGAAC CTTGAGAGAG TTGCCAAATA CTCGATGGAA 1560
   GATGCAAAGG CAACTTATGA ACTCGGGAAA GAATTCCTTC CAATGGAAAT TCAGCTTTCA 1620
   GAGAGGCTCC TTTGGCTTTA CCGGGAGGTG GAGAGGCCCC TTTCCGCTGT CCTGGCCCAC 1680
   ATGGAGGCCA CGGGGGTGCG CCTGGACGTG GCCTATCTCA GGGCCTTGTC CCTGGAGGTG 1740
   GCCGAGGAGA TCGCCCGCCT CGAGGCCGAG GTCTTCCGCC TGGCCGGCCA CCCCTTCAAC 1800
   CTCAACTCCC GGGACCAGCT GGAAAGGGTC CTCTTTGACG AGCTAGGGCT TCCCGCCATC 1860
   GGCAAGACGG AGAAGACCGG CAAGCGCTCC ACCAGCGCCG CCGTCCTGGA GGCCCTCCGC 1920
   GAGGCCCACC CCATCGTGGA GAAGATCCTG CAGTACCGGG AGCTCACCAA GCTGAAGAGC 1980
   ACCTACATTG ACCCCTTGCC GGACCTCATC CACCCCAGGA CGGGCCGCCT CCACACCCGC 2040
   TTCAACCAGA CGGCCACGGC CACGGGCAGG CTAAGTAGCT CCGATCCCAA CCTCCAGAAC 2100
   ATCCCCGTCC GCACCCCGCT TGGGCAGAGG ATCCGCCGGG CCTTCATCGC CGAGGAGGGG 2160
   TGGCTATTGG TGGCCCTGGA CTATAGCCAG ATAGAGCTCA GGGTGCTGGC CCACCTCTCC 2220
   GGCGACGAGA ACCTGATCCG GGTCTTCCAG GAGGGGCGGG ACATCCACAC GGAGACCGCC 2280
   AGCTGGATGT TCGGCGTCCC CCGGGAGGCC GTGGACCCCC TGATGCGCCG GGCGGCCAAG 2340
   ACCATCAACT TCGGGGTCCT CTACGGCATG TCGGCCCACC GCCTCTCCCA GGAGCTAGCC 2400
   ATCCCTTACG AGGAGGCCCA GGCCTTCATT GAGCGCTACT TTCAGAGCTT CCCCAAGGTG 2460
   CGGGCCTGGA TTGAGAAGAC CCTGGAGGAG GGCAGGAGGC GGGGGTACGT GGAGACCCTC 2520
   TTCGGCCGCC GCCGCTACGT GCCAGACCTA GAGGCCCGGG TGAAGAGCGT GCGGGAGGCG 2580
   GCCGAGCGCA TGGCCTTCAA CATGCCCGTC CAGGGCACCG CCGCCGACCT CATGAAGCTG 2640
   GCTATGGTGA AGCTCTTCCC CAGGCTGGAG GAAATGGGGG CCAGGATGCT CCTTCAGGTC 2700
   CACGACGAGC TGGTCCTCGA GGCCCCAAAA GAGAGGGCGG AGGCCGTGGC CCGGCTGGCC 2760
   AAGGAGGTCA TGGAGGGGGT GTATCCCCTG GCCGTGCCCC TGGAGGTGGA GGTGGGGATA 2820
   GGGGAGGACT GGCTCTCCGC CAAGGAGTGA 2850
(2) ANGABEN ZU SEQ ID NO: 6:
   (i) SEQUENZKENNZEICHEN:
      (A) LÄNGE: 2949 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 6:
   ATGAGGGGCT CGCATCACCA TCACCATCAC GCTGCTGACG ATGACGATAA AATGAGGGGC   60
   ATGCTACCGC TATTTGAGCC CAAGGGCCGG GTCCTCCTGG TCGACGGCCA CCACCTGGCC  120
   TACCGCACCT TCCACGCCCT GAAGGGCCTC ACCACCAGCC GGGGGGAGCC GGTGCAGGCG  180
   GTCTACGGCT TCGCCAAGAG CCTCCTCAAG GCCCTCAAGG AGGACGGGGA CGCGGTGATC  240
   GTGGTCTTTG ACGCCAAGGC CCCCTCCTTC CGCCACGAGG CCTACGGGGG GTACAAGGCG  300
   GGCCGGGCCC CCACGCCGGA GGACTTTCCC CGGCAACTCG CCCTCATCAA GGAGCTGGTG  360
   GACCTCCTGG GGCTGGCGCG CCTCGAGGTC CCGGGCTACG AGGCGGACGA CGTCCTGGCC  420
   AGCCTGGCCA AGAAGGCGGA AAAGGAGGGC TACGAGGTCC GCATCCTCAC CGCCGACAAA  480
   GACCTTTACC AGCTCCTTTC CGACCGCATC CACGTCCTCC ACCCCGAGGG GTACCTCATC  540
   ACCCCGGCCT GGCTTTGGGA AAAGTACGGC CTGAGGCCCG ACCAGTGGGC CGACTACCGG  600
   GCCCTGACCG GGGACGAGTC CGACAACCTT CCCGGGGTCA AGGGCATCGG GGAGAAGACG  660
   GCGAGGAAGC TTCTGGAGGA GTGGGGGAGC CTGGAAGCCC TCCTCAAGAA CCTGGACCGG  720
   CTGAAGCCCG CCATCCGGGA GAAGATCCTG GCCCACATGG ACGATCTGAA GCTCTCCTGG  780
   GACCTGGCCA AGGTGCGCAC CGACCTGCCC CTGGAGGTGG ACTTCGCCAA AAGGCGGGAG  840
   CCCGACCGGG AGAGGCTTAG GGCCTTTCTG GAGAGGCTTG AGTTTGGCAG CCTCCTCCAC  900
   GAGTTCGGCC TTCTGGAAAG CCCCGTTAGA GAACATCCAG CAGTTGTGGA CATCTTCGAA  960
   TACGATATTC CATTTGCAAA GAGATACCTC ATCGACAAAG GCCTAATACC AATGGAGGGG 1020
   GAAGAAGAGC TAAAGATTCT TGCCTTCGAT ATAGAAACCC TCTATCACGA AGGAGAAGAG 1080
   TTTGGAAAAG GCCCAATTAT AATGATTAGT TATGCAGATG AAAATGAAGC AAAGGTGATT 1140
   ACTTGGAAAA ACATAGATCT TCCATACGTT GAGGTTGTAT CAAGCGAGAG AGAGATGATA 1200
   AAGAGATTTC TCAGGATTAT CAGGGAGAAG GATCCTGACA TTATAGTTAC TTATAATGGA 1260
   GACTCATTCG ACTTCCCATA TTTAGCGAAA AGGGCAGAAA AACTTGGGAT TAAATTAACC 1320
   ATTGGAAGAG ATGGAAGCGA GCCCAAGATG CAGAGAATAG GCGATATGAC GGCTGTAGAA 1380
   GTCAAGGGAA GAATACATTT CGACTTGTAT CATGTAATAA CAAGGACAAT AAATCTCCCA 1440
   ACATACACAC TAGAGGCTGT ATATGAAGCA ATTTTTGGAA AGCCAAAGGA GAAGGTATAC 1500
   GCCGACGAGA TAGCAAAAGC CTGGGAAAGT GGAGAGAACC TTGAGAGAGT TGCCAAATAC 1560
   TCGATGGAAG ATGCAAAGGC AACTTATGAA CTCGGGAAAG AATTCCTTCC AATGGAAATT 1620
   CAGCTTTCAA GATTAGTTGG ACAACCTTTA TGGGATGTTT CAAGGTCAAG CACAGGGAAC 1680
   CTTGTAGAGT GGTTCTTACT TAGGAAAGCC TACGAAAGAA ACGAAGTAGC TCCAAACAAG 1740
   CCAAGTGAAG AGGAGTATCA AAGAAGGCTC AGGGAGAGCT ACACAGGTGG ATTCGTGCGC 1800
   CTGGACGTGG CCTATCTCAG GGCCTTGTCC CTGGAGGTGG CCGAGGAGAT CGCCCGCCTC 1860
   GAGGCCGAGG TCTTCCGCCT GGCCGGCCAC CCCTTCAACC TCAACTCCCG GGACCAGCTG 1920
   GAAAGGGTCC TCTTTGACGA GCTAGGGCTT CCCGCCATCG GCAAGACGGA GAAGACCGGC 1980
   AAGCGCTCCA CCAGCGCCGC CGTCCTGGAG GCCCTCCGCG AGGCCCACCC CATCGTGGAG 2040
   AAGATCCTGC AGTACCGGGA GCTCACCAAG CTGAAGAGCA CCTACATTGA CCCCTTGCCG 2100
   GACCTCATCC ACCCCAGGAC GGGCCGCCTC CACACCCGCT TCAACCAGAC GGCCACGGCC 2160
   ACGGGCAGGC TAAGTAGCTC CGATCCCAAC CTCCAGAACA TCCCCGTCCG CACCCCGCTT 2220
   GGGCAGAGGA TCCGCCGGGC CTTCATCGCC GAGGAGGGGT GGCTATTGGT GGCCCTGGAC 2280
   TATAGCCAGA TAGAGCTCAG GGTGCTGGCC CACCTCTCCG GCGACGAGAA CCTGATCCGG 2340
   GTCTTCCAGG AGGGGCGGGA CATCCACACG GAGACCGCCA GCTGGATGTT CGGCGTCCCC 2400
   CGGGAGGCCG TGGACCCCCT GATGCGCCGG GCGGCCAAGA CCATCAACTT CGGGGTCCTC 2460
   TACGGCATGT CGGCCCACCG CCTCTCCCAG GAGCTAGCCA TCCCTTACGA GGAGGCCCAG 2520
   GCCTTCATTG AGCGCTACTT TCAGAGCTTC CCCAAGGTGC GGGCCTGGAT TGAGAAGACC 2580
   CTGGAGGAGG GCAGGAGGCG GGGGTACGTG GAGACCCTCT TCGGCCGCCG CCGCTACGTG 2640
   CCAGACCTAG AGGCCCGGGT GAAGAGCGTG CGGGAGGCGG CCGAGCGCAT GGCCTTCAAC 2700
   ATGCCCGTCC AGGGCACCGC CGCCGACCTC ATGAAGCTGG CTATGGTGAA GCTCTTCCCC 2760
   AGGCTGGAGG AAATGGGGGC CAGGATGCTC CTTCAGGTCC ACGACGAGCT GGTCCTCGAG 2820
   GCCCCAAAAG AGAGGGCGGA GGCCGTGGCC CGGCTGGCCA AGGAGGTCAT GGAGGGGGTG 2880
   TATCCCCTGG CCGTGCCCCT GGAGGTGGAG GTGGGGATAG GGGAGGACTG GCTCTCCGCC 2940
   AAGGAGTGA 2949
(2) ANGABEN ZU SEQ ID NO: 7:
   (i) SEQUENZKENNZEICHEN:
      (A) LÄNGE: 910 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 7:
   Met Arg Gly Ser His His His His His His Ala Ala Asp Asp Asp Asp
   1 5 10 15
   Lys Met Arg Gly Met Leu Pro Leu Phe Glu Pro Lys Gly Arg Val Leu
   20 25 30
   Leu Val Asp Gly His His Leu Ala Tyr Arg Thr Phe His Ala Leu Lys
   35 40 45
   Gly Leu Thr Thr Ser Arg Gly Glu Pro Val Gln Ala Val Tyr Gly Phe
   50 55 60
   Ala Lys Ser Leu Leu Lys Ala Leu Lys Glu Asp Gly Asp Ala Val Ile
   65 70 75 80
   Val Val Phe Asp Ala Lys Ala Pro Ser Phe Arg His Glu Ala Tyr Gly
   85 90 95
   Gly Tyr Lys Ala Gly Arg Ala Pro Thr Pro Glu Asp Phe Pro Arg Gln
   100 105 110
   Leu Ala Leu Ile Lys Glu Leu Val Asp Leu Leu Gly Leu Ala Arg Leu
   115 120 125
   Glu Val Pro Gly Tyr Glu Ala Asp Asp Val Leu Ala Ser Leu Ala Lys
   130 135 140
   Lys Ala Glu Lys Glu Gly Tyr Glu Val Arg Ile Leu Thr Ala Asp Lys
   145 150 155 160
   Asp Leu Tyr Gln Leu Leu Ser Asp Arg Ile His Val Leu His Pro Glu
   165 170 175
   Gly Tyr Leu Ile Thr Pro Ala Trp Leu Trp Glu Lys Tyr Gly Leu Arg
   180 185 190
   Pro Asp Gln Trp Ala Asp Tyr Arg Ala Leu Thr Gly Asp Glu Ser Asp
   195 200 205
   Asn Leu Pro Gly Val Lys Gly Ile Gly Glu Lys Thr Ala Arg Lys Leu
   210 215 220
   Leu Glu Glu Trp Gly Ser Leu Glu Ala Leu Leu Lys Asn Leu Asp Arg
   225 230 235 240
   Leu Lys Pro Ala Ile Arg Glu Lys Ile Leu Ala His Met Asp Asp Leu
   245 250 255
   Lys Leu Ser Trp Asp Leu Ala Lys Val Arg Thr Asp Leu Pro Leu Glu
   260 265 270
   Val Asp Phe Ala Lys Arg Arg Glu Pro Asp Arg Glu Arg Leu Arg Ala
   275 280 285
   Phe Leu Glu Arg Leu Glu Phe Gly Ser Leu Leu His Glu Phe Gly Leu
   290 295 300
   Leu Glu Ser Pro Tyr Asp Asn Tyr Val Thr Ile Leu Asp Glu Glu Thr
   305 310 315 320
   Leu Lys Ala Trp Ile Ala Lys Leu Glu Lys Ala Pro Val Phe Ala Phe
   325 330 335
   Asp Thr Glu Thr Asp Ser Leu Asp Asn Ile Ser Ala Asn Leu Val Gly
   340 345 350
   Leu Ser Phe Ala Ile Glu Pro Gly Val Ala Ala Tyr Ile Pro Val Ala
   355 360 365
   His Asp Tyr Leu Asp Ala Pro Asp Gln Ile Ser Arg Glu Arg Ala Leu
   370 375 380
   Glu Leu Leu Lys Pro Leu Leu Glu Asp Glu Lys Ala Leu Lys Val Gly
   385 390 395 400
   Gln Asn Leu Lys Tyr Asp Arg Gly Ile Leu Ala Asn Tyr Gly Ile Glu
                   405                 410                 415
   Leu Arg Gly Ile Ala Phe Asp Thr Met Leu Glu Ser Tyr Ile Leu Asn
               420                 425                 430
   Ser Val Ala Gly Arg His Asp Met Asp Ser Leu Ala Glu Arg Trp Leu
           435                 440                 445
   Lys His Lys Thr Ile Thr Phe Glu Glu Ile Ala Gly Lys Gly Lys Asn
       450                 455                 460
   Gln Leu Thr Phe Asn Gln Ile Ala Leu Glu Glu Ala Gly Arg Tyr Ala
   465                 470                 475                 480
   Ala Glu Asp Ala Asp Val Thr Leu Gln Leu His Leu Lys Met Trp Pro
                   485                 490                 495
   Asp Leu Gln Lys His Glu Arg Leu Leu Trp Leu Tyr Arg Glu Val Glu
               500                 505                 510
   Arg Pro Leu Ser Ala Val Leu Ala His Met Glu Ala Thr Gly Val Arg
           515                 520                 525
   Leu Asp Val Ala Tyr Leu Arg Ala Leu Ser Leu Glu Val Ala Glu Glu
       530                 535                 540
   Val Ala Arg Leu Glu Ala Glu Val Phe Arg Leu Ala Gly His Pro Phe
   545                 550                 555                 560
   Asn Leu Asn Ser Arg Asp Gln Leu Glu Arg Val Leu Phe Asp Glu Leu
                   565                 570                 575
   Gly Leu Pro Ala Ile Gly Lys Thr Glu Lys Thr Gly Lys Arg Ser Thr
               580                 585                 590
   Ser Ala Ala Val Leu Glu Ala Leu Arg Glu Ala His Pro Ile Val Glu
           595                 600                 605
   Lys Ile Leu Gln Tyr Arg Glu Leu Thr Lys Leu Lys Ser Thr Tyr Ile
       610                 615                 620
   Asp Pro Leu Pro Asp Leu Ile His Pro Arg Thr Gly Arg Leu His Thr
   625                 630                 635                 640
   Arg Phe Asn Gln Thr Ala Thr Ala Thr Gly Arg Leu Ser Ser Ser Asp
                   645                 650                 655
   Pro Asn Leu Gln Asn Ile Pro Val Arg Thr Pro Leu Gly Gln Arg Ile
               660                 665                 670
   Arg Arg Ala Phe Ile Ala Glu Glu Gly Trp Leu Leu Val Ala Leu Asp
           675                 680                 685
   Tyr Ser Gln Ile Glu Leu Arg Val Leu Ala His Leu Ser Gly Asp Glu
       690                 695                 700
   Asn Leu Ile Arg Val Phe Gln Glu Gly Arg Asp Ile His Thr Glu Thr
   705                 710                 715                 720
   Ala Ser Trp Met Phe Gly Val Pro Arg Glu Ala Val Asp Pro Leu Met
                   725                 730                 735
   Arg Arg Ala Ala Lys Thr Ile Asn Phe Gly Val Leu Tyr Gly Met Ser
               740                 745                 750
   Ala His Arg Leu Ser Gln Glu Leu Ala Ile Pro Tyr Glu Glu Ala Gln
           755                 760                 765
   Ala Phe Ile Glu Arg Tyr Phe Gln Ser Phe Pro Lys Val Arg Ala Trp
       770                 775                 780
   Ile Glu Lys Thr Leu Glu Glu Gly Arg Arg Arg Gly Tyr Val Glu Thr
   785                 790                 795                 800
   Leu Phe Gly Arg Arg Arg Tyr Val Pro Asp Leu Glu Ala Arg Val Lys
                   805                 810                 815
   Ser Val Arg Glu Ala Ala Glu Arg Met Ala Phe Asn Met Pro Val Gln
               820                 825                 830
   Gly Thr Ala Ala Asp Leu Met Lys Leu Ala Met Val Lys Leu Phe Pro
           835                 840                 845
   Arg Leu Glu Glu Met Gly Ala Arg Met Leu Leu Gln Val His Asp Glu
       850                 855                 860
   Leu Val Leu Glu Ala Pro Lys Glu Arg Ala Glu Ala Val Ala Arg Leu
   865                 870                 875                 880
   Ala Lys Glu Val Met Glu Gly Val Tyr Pro Leu Ala Val Pro Leu Glu
                   885                 890                 895
   Val Glu Val Gly Ile Gly Glu Asp Trp Leu Ser Ala Lys Glu
               900                 905                 910
(2) ANGABEN ZU SEQ ID NO: 8:
   (i) SEQUENZKENNZEICHEN:
      (A) LÄNGE: 910 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 8:
   Met Arg Gly Ser His His His His His His Ala Ala Asp Asp Asp Asp
   1               5                   10                  15
   Lys Met Arg Gly Met Leu Pro Leu Phe Glu Pro Lys Gly Arg Val Leu
               20                  25                  30
   Leu Val Asp Gly His His Leu Ala Tyr Arg Thr Phe His Ala Leu Lys
           35                  40                  45
   Gly Leu Thr Thr Ser Arg Gly Glu Pro Val Gln Ala Val Tyr Gly Phe
       50                  55                  60
   Ala Lys Ser Leu Leu Lys Ala Leu Lys Glu Asp Gly Asp Ala Val Ile
   65                  70                  75                  80
   Val Val Phe Asp Ala Lys Ala Pro Ser Phe Arg His Glu Ala Tyr Gly
                   85                  90                  95
   Gly Tyr Lys Ala Gly Arg Ala Pro Thr Pro Glu Asp Phe Pro Arg Gln
               100                 105                 110
   Leu Ala Leu Ile Lys Glu Leu Val Asp Leu Leu Gly Leu Ala Arg Leu
           115                 120                 125
   Glu Val Pro Gly Tyr Glu Ala Asp Asp Val Leu Ala Ser Leu Ala Lys
       130                 135                 140
   Lys Ala Glu Lys Glu Gly Tyr Glu Val Arg Ile Leu Thr Ala Asp Lys
   145                 150                 155                 160
   Asp Leu Tyr Gln Leu Leu Ser Asp Arg Ile His Val Leu His Pro Glu
                   165                 170                 175
   Gly Tyr Leu Ile Thr Pro Ala Trp Leu Trp Glu Lys Tyr Gly Leu Arg
               180                 185                 190
   Pro Asp Gln Trp Ala Asp Tyr Arg Ala Leu Thr Gly Asp Glu Ser Asp
           195                 200                 205
   Asn Leu Pro Gly Val Lys Gly Ile Gly Glu Lys Thr Ala Arg Lys Leu
   210 215 220
   Leu Glu Glu Trp Gly Ser Leu Glu Ala Leu Leu Lys Asn Leu Asp Arg
   225 230 235 240
   Leu Lys Pro Ala Ile Arg Glu Lys Ile Leu Ala His Met Asp Asp Leu
   245 250 255
   Lys Leu Ser Trp Asp Leu Ala Lys Val Arg Thr Asp Leu Pro Leu Glu
   260 265 270
   Val Asp Phe Ala Lys Arg Arg Glu Pro Asp Arg Glu Arg Leu Arg Ala
   275 280 285
   Phe Leu Glu Arg Leu Glu Phe Gly Ser Leu Leu His Glu Phe Gly Leu
   290 295 300
   Leu Glu Ser Pro Tyr Asp Asn Tyr Val Thr Ile Leu Asp Glu Glu Thr
   305 310 315 320
   Leu Lys Ala Trp Ile Ala Lys Leu Glu Lys Ala Pro Val Phe Ala Phe
   325 330 335
   Asp Thr Glu Thr Asp Ser Leu Asp Asn Ile Ser Ala Asn Leu Val Gly
   340 345 350
   Leu Ser Phe Ala Ile Glu Pro Gly Val Ala Ala Tyr Ile Pro Val Ala
   355 360 365
   His Asp Tyr Leu Asp Ala Pro Asp Gln Ile Ser Arg Glu Arg Ala Leu
   370 375 380
   Glu Leu Leu Lys Pro Leu Leu Glu Asp Glu Lys Ala Leu Lys Val Gly
   385 390 395 400
   Gln Asn Leu Lys Tyr Asp Arg Gly Ile Leu Ala Asn Tyr Gly Ile Glu
   405 410 415
   Leu Arg Gly Ile Ala Phe Asp Thr Met Leu Glu Ser Tyr Ile Leu Asn
   420 425 430
   Ser Val Ala Gly Arg His Asp Met Asp Ser Leu Ala Glu Arg Trp Leu
   435 440 445
   Lys His Lys Thr Ile Thr Phe Glu Glu Ile Ala Gly Lys Gly Lys Asn
   450 455 460
   Gln Leu Thr Phe Asn Gln Ile Ala Leu Glu Glu Ala Gly Arg Tyr Ala
   465 470 475 480
   Ala Glu Asp Ala Asp Val Thr Leu Gln Leu His Leu Lys Met Trp Pro
   485 490 495
   Asp Leu Gln Lys His Lys Gly Pro Leu Asn Val Phe Glu Asn Ile Glu
   500 505 510
   Met Pro Leu Val Pro Val Leu Ser Arg Ile Glu Arg Asn Gly Val Arg
   515 520 525
   Leu Asp Val Ala Tyr Leu Arg Ala Leu Ser Leu Glu Val Ala Glu Glu
   530 535 540
   Ile Ala Arg Leu Glu Ala Glu Val Phe Arg Leu Ala Gly His Pro Phe
   545 550 555 560
   Asn Leu Asn Ser Arg Asp Gln Leu Glu Arg Val Leu Phe Asp Glu Leu
   565 570 575
   Gly Leu Pro Ala Ile Gly Lys Thr Glu Lys Thr Gly Lys Arg Ser Thr
   580 585 590
   Ser Ala Ala Val Leu Glu Ala Leu Arg Glu Ala His Pro Ile Val Glu
   595 600 605
   Lys Ile Leu Gln Tyr Arg Glu Leu Thr Lys Leu Lys Ser Thr Tyr Ile
   610 615 620
   Asp Pro Leu Pro Asp Leu Ile His Pro Arg Thr Gly Arg Leu His Thr
   625 630 635 640
   Arg Phe Asn Gln Thr Ala Thr Ala Thr Gly Arg Leu Ser Ser Ser Asp
   645 650 655
   Pro Asn Leu Gln Asn Ile Pro Val Arg Thr Pro Leu Gly Gln Arg Ile
   660 665 670
   Arg Arg Ala Phe Ile Ala Glu Glu Gly Trp Leu Leu Val Ala Leu Asp
   675 680 685
   Tyr Ser Gln Ile Glu Leu Arg Val Leu Ala His Leu Ser Gly Asp Glu
   690 695 700
   Asn Leu Ile Arg Val Phe Gln Glu Gly Arg Asp Ile His Thr Glu Thr
   705 710 715 720
   Ala Ser Trp Met Phe Gly Val Pro Arg Glu Ala Val Asp Pro Leu Met
   725 730 735
   Arg Arg Ala Ala Lys Thr Ile Asn Phe Gly Val Leu Tyr Gly Met Ser
   740 745 750
   Ala His Arg Leu Ser Gln Glu Leu Ala Ile Pro Tyr Glu Glu Ala Gln
   755 760 765
   Ala Phe Ile Glu Arg Tyr Phe Gln Ser Phe Pro Lys Val Arg Ala Trp
   770 775 780
   Ile Glu Lys Thr Leu Glu Glu Gly Arg Arg Arg Gly Tyr Val Glu Thr
   785 790 795 800
   Leu Phe Gly Arg Arg Arg Tyr Val Pro Asp Leu Glu Ala Arg Val Lys
   805 810 815
   Ser Val Arg Glu Ala Ala Glu Arg Met Ala Phe Asn Met Pro Val Gln
   820 825 830
   Gly Thr Ala Ala Asp Leu Met Lys Leu Ala Met Val Lys Leu Phe Pro
   835 840 845
   Arg Leu Glu Glu Met Gly Ala Arg Met Leu Leu Gln Val His Asp Glu
   850 855 860
   Leu Val Leu Glu Ala Pro Lys Glu Arg Ala Glu Ala Val Ala Arg Leu
   865 870 875 880
   Ala Lys Glu Val Met Glu Gly Val Tyr Pro Leu Ala Val Pro Leu Glu
   885 890 895
   Val Glu Val Gly Ile Gly Glu Asp Trp Leu Ser Ala Lys Glu
   900 905 910
(2) ANGABEN ZU SEQ ID NO: 9:
   (i) SEQUENZKENNZEICHEN:
      (A) LÄNGE: 908 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 9:
   Met Arg Gly Ser His His His His His His Ala Ala Asp Asp Asp Asp
   1 5 10 15
   Lys Met Arg Gly Met Leu Pro Leu Phe Glu Pro Lys Gly Arg Val Leu
   20 25 30
   Leu Val Asp Gly His His Leu Ala Tyr Arg Thr Phe His Ala Leu Lys
   35 40 45
   Gly Leu Thr Thr Ser Arg Gly Glu Pro Val Gln Ala Val Tyr Gly Phe
   50 55 60
   Ala Lys Ser Leu Leu Lys Ala Leu Lys Glu Asp Gly Asp Ala Val Ile
   65 70 75 80
   Val Val Phe Asp Ala Lys Ala Pro Ser Phe Arg His Glu Ala Tyr Gly
   85 90 95
   Gly Tyr Lys Ala Gly Arg Ala Pro Thr Pro Glu Asp Phe Pro Arg Gln
   100 105 110
   Leu Ala Leu Ile Lys Glu Leu Val Asp Leu Leu Gly Leu Ala Arg Leu
   115 120 125
   Glu Val Pro Gly Tyr Glu Ala Asp Asp Val Leu Ala Ser Leu Ala Lys
   130 135 140
   Lys Ala Glu Lys Glu Gly Tyr Glu Val Arg Ile Leu Thr Ala Asp Lys
   145 150 155 160
   Asp Leu Tyr Gln Leu Leu Ser Asp Arg Ile His Val Leu His Pro Glu
   165 170 175
   Gly Tyr Leu Ile Thr Pro Ala Trp Leu Trp Glu Lys Tyr Gly Leu Arg
   180 185 190
   Pro Asp Gln Trp Ala Asp Tyr Arg Ala Leu Thr Gly Asp Glu Ser Asp
   195 200 205
   Asn Leu Pro Gly Val Lys Gly Ile Gly Glu Lys Thr Ala Arg Lys Leu
   210 215 220
   Leu Glu Glu Trp Gly Ser Leu Glu Ala Leu Leu Lys Asn Leu Asp Arg
   225 230 235 240
   Leu Lys Pro Ala Ile Arg Glu Lys Ile Leu Ala His Met Asp Asp Leu
   245 250 255
   Lys Leu Ser Trp Asp Leu Ala Lys Val Arg Thr Asp Leu Pro Leu Glu
   260 265 270
   Val Asp Phe Ala Lys Arg Arg Glu Pro Asp Arg Glu Arg Leu Arg Ala
   275 280 285
   Phe Leu Glu Arg Leu Glu Phe Gly Ser Leu Leu His Glu Phe Gly Leu
   290 295 300
   Leu Glu Ser Pro Pro Val Gly Tyr Arg Ile Val Lys Asp Leu Val Glu
   305 310 315 320
   Phe Glu Lys Leu Ile Glu Lys Leu Arg Glu Ser Pro Ser Phe Ala Ile
   325 330 335
   Asp Leu Glu Thr Ser Ser Leu Asp Pro Phe Asp Cys Asp Ile Val Gly
   340 345 350
   Ile Ser Val Ser Phe Lys Pro Lys Glu Ala Tyr Tyr Ile Pro Leu His
   355 360 365
   His Arg Asn Ala Gln Asn Leu Asp Glu Lys Glu Val Leu Lys Lys Leu
   370 375 380
   Lys Glu Ile Leu Glu Asp Pro Gly Ala Lys Ile Val Gly Gln Asn Leu
   385 390 395 400
   Lys Phe Asp Tyr Lys Val Leu Met Val Lys Gly Val Glu Pro Val Pro
   405 410 415
   Pro His Phe Asp Thr Met Ile Ala Ala Tyr Leu Leu Glu Pro Asn Glu
   420 425 430
   Lys Lys Phe Asn Leu Asp Asp Leu Ala Leu Lys Phe Leu Gly Tyr Lys
   435 440 445
   Met Thr Ser Tyr Gln Glu Leu Met Ser Phe Ser Ser Pro Leu Phe Gly
   450 455 460
   Phe Ser Phe Ala Asp Val Pro Val Glu Lys Ala Ala Asn Tyr Ser Cys
   465 470 475 480
   Glu Asp Ala Asp Ile Thr Tyr Arg Leu Tyr Lys Ile Leu Ser Leu Lys
   485 490 495
   Leu His Glu Glu Arg Leu Leu Trp Leu Tyr Arg Glu Val Glu Arg Pro
   500 505 510
   Leu Ser Ala Val Leu Ala His Met Glu Ala Thr Gly Val Arg Leu Asp
   515 520 525
   Val Ala Tyr Leu Arg Ala Leu Ser Leu Glu Val Ala Glu Glu Ile Ala
   530 535 540
   Arg Leu Glu Ala Glu Val Phe Arg Leu Ala Gly His Pro Phe Asn Leu
   545 550 555 560
   Asn Ser Arg Asp Gln Leu Glu Arg Val Leu Phe Asp Glu Leu Gly Leu
   565 570 575
   Pro Ala Ile Gly Lys Thr Glu Lys Thr Gly Lys Arg Ser Thr Ser Ala
               580                 585                 590
   Ala Val Leu Glu Ala Leu Arg Glu Ala His Pro Ile Val Glu Lys Ile
           595                 600                 605
   Leu Gln Tyr Arg Glu Leu Thr Lys Leu Lys Ser Thr Tyr Ile Asp Pro
       610                 615                 620
   Leu Pro Asp Leu Ile His Pro Arg Thr Gly Arg Leu His Thr Arg Phe
   625                 630                 635                 640
   Asn Gln Thr Ala Thr Ala Thr Gly Arg Leu Ser Ser Ser Asp Pro Asn
                   645                 650                 655
   Leu Gln Asn Ile Pro Val Arg Thr Pro Leu Gly Gln Arg Ile Arg Arg
               660                 665                 670
   Ala Phe Ile Ala Glu Glu Gly Trp Leu Leu Val Ala Leu Asp Tyr Ser
           675                 680                 685
   Gln Ile Glu Leu Arg Val Leu Ala His Leu Ser Gly Asp Glu Asn Leu
       690                 695                 700
   Ile Arg Val Phe Gln Glu Gly Arg Asp Ile His Thr Glu Thr Ala Ser
   705                 710                 715                 720
   Trp Met Phe Gly Val Pro Arg Glu Ala Val Asp Pro Leu Met Arg Arg
                   725                 730                 735
   Ala Ala Lys Thr Ile Asn Phe Gly Val Leu Tyr Gly Met Ser Ala His
               740                 745                 750
   Arg Leu Ser Gln Glu Leu Ala Ile Pro Tyr Glu Glu Ala Gln Ala Phe
           755                 760                 765
   Ile Glu Arg Tyr Phe Gln Ser Phe Pro Lys Val Arg Ala Trp Ile Glu
       770                 775                 780
   Lys Thr Leu Glu Glu Gly Arg Arg Arg Gly Tyr Val Glu Thr Leu Phe
   785                 790                 795                 800
   Gly Arg Arg Arg Tyr Val Pro Asp Leu Glu Ala Arg Val Lys Ser Val
                   805                 810                 815
   Arg Glu Ala Ala Glu Arg Met Ala Phe Asn Met Pro Val Gln Gly Thr
               820                 825                 830
   Ala Ala Asp Leu Met Lys Leu Ala Met Val Lys Leu Phe Pro Arg Leu
           835                 840                 845
   Glu Glu Met Gly Ala Arg Met Leu Leu Gln Val His Asp Glu Leu Val
   850 855 860
   Leu Glu Ala Pro Lys Glu Arg Ala Glu Ala Val Ala Arg Leu Ala Lys
   865 870 875 880
   Glu Val Met Glu Gly Val Tyr Pro Leu Ala Val Pro Leu Glu Val Glu
   885 890 895
   Val Gly Ile Gly Glu Asp Trp Leu Ser Ala Lys Glu
   900 905
(2) ANGABEN ZU SEQ ID NO: 10:
   (i) SEQUENZKENNZEICHEN:
      (A) LÄNGE: 908 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 10:
   Met Arg Gly Ser His His His His His His Ala Ala Asp Asp Asp Asp
   1 5 10 15
   Lys Met Arg Gly Met Leu Pro Leu Phe Glu Pro Lys Gly Arg Val Leu
   20 25 30
   Leu Val Asp Gly His His Leu Ala Tyr Arg Thr Phe His Ala Leu Lys
   35 40 45
   Gly Leu Thr Thr Ser Arg Gly Glu Pro Val Gln Ala Val Tyr Gly Phe
   50 55 60
   Ala Lys Ser Leu Leu Lys Ala Leu Lys Glu Asp Gly Asp Ala Val Ile
   65 70 75 80
   Val Val Phe Asp Ala Lys Ala Pro Ser Phe Arg His Glu Ala Tyr Gly
   85 90 95
   Gly Tyr Lys Ala Gly Arg Ala Pro Thr Pro Glu Asp Phe Pro Arg Gln
   100 105 110
   Leu Ala Leu Ile Lys Glu Leu Val Asp Leu Leu Gly Leu Ala Arg Leu
   115 120 125
   Glu Val Pro Gly Tyr Glu Ala Asp Asp Val Leu Ala Ser Leu Ala Lys
   130 135 140
   Lys Ala Glu Lys Glu Gly Tyr Glu Val Arg Ile Leu Thr Ala Asp Lys
   145 150 155 160
   Asp Leu Tyr Gln Leu Leu Ser Asp Arg Ile His Val Leu His Pro Glu
   165 170 175
   Gly Tyr Leu Ile Thr Pro Ala Trp Leu Trp Glu Lys Tyr Gly Leu Arg
   180 185 190
   Pro Asp Gln Trp Ala Asp Tyr Arg Ala Leu Thr Gly Asp Glu Ser Asp
   195 200 205
   Asn Leu Pro Gly Val Lys Gly Ile Gly Glu Lys Thr Ala Arg Lys Leu
   210 215 220
   Leu Glu Glu Trp Gly Ser Leu Glu Ala Leu Leu Lys Asn Leu Asp Arg
   225 230 235 240
   Leu Lys Pro Ala Ile Arg Glu Lys Ile Leu Ala His Met Asp Asp Leu
   245 250 255
   Lys Leu Ser Trp Asp Leu Ala Lys Val Arg Thr Asp Leu Pro Leu Glu
   260 265 270
   Val Asp Phe Ala Lys Arg Arg Glu Pro Asp Arg Glu Arg Leu Arg Ala
   275 280 285
   Phe Leu Glu Arg Leu Glu Phe Gly Ser Leu Leu His Glu Phe Gly Leu
   290 295 300
   Leu Glu Ser Pro Pro Val Gly Tyr Arg Ile Val Lys Asp Leu Val Glu
   305 310 315 320
   Phe Glu Lys Leu Ile Glu Lys Leu Arg Glu Ser Pro Ser Phe Ala Ile
   325 330 335
   Asp Leu Glu Thr Ser Ser Leu Asp Pro Phe Asp Cys Asp Ile Val Gly
   340 345 350
   Ile Ser Val Ser Phe Lys Pro Lys Glu Ala Tyr Tyr Ile Pro Leu His
   355 360 365
   His Arg Asn Ala Gln Asn Leu Asp Glu Lys Glu Val Leu Lys Lys Leu
   370 375 380
   Lys Glu Ile Leu Glu Asp Pro Gly Ala Lys Ile Val Gly Gln Asn Leu
   385 390 395 400
   Lys Phe Asp Tyr Lys Val Leu Met Val Lys Gly Val Glu Pro Val Pro
                   405                 410                 415
   Pro His Phe Asp Thr Met Ile Ala Ala Tyr Leu Leu Glu Pro Asn Glu
               420                 425                 430
   Lys Lys Phe Asn Leu Asp Asp Leu Ala Leu Lys Phe Leu Gly Tyr Lys
           435                 440                 445
   Met Thr Ser Tyr Gln Glu Leu Met Ser Phe Ser Ser Pro Leu Phe Gly
       450                 455                 460
   Phe Ser Phe Ala Asp Val Pro Val Glu Lys Ala Ala Asn Tyr Ser Cys
   465                 470                 475                 480
   Glu Asp Ala Asp Ile Thr Tyr Arg Leu Tyr Lys Ile Leu Ser Leu Lys
                   485                 490                 495
   Leu His Glu Ala Asp Leu Glu Asn Val Phe Tyr Lys Ile Glu Met Pro
               500                 505                 510
   Leu Val Ser Val Leu Ala Arg Met Glu Leu Asn Gly Val Arg Leu Asp
           515                 520                 525
   Val Ala Tyr Leu Arg Ala Leu Ser Leu Glu Val Ala Glu Glu Ile Ala
       530                 535                 540
   Arg Leu Glu Ala Glu Val Phe Arg Leu Ala Gly His Pro Phe Asn Leu
   545                 550                 555                 560
   Asn Ser Arg Asp Gln Leu Glu Arg Val Leu Phe Asp Glu Leu Gly Leu
                   565                 570                 575
   Pro Ala Ile Gly Lys Thr Glu Lys Thr Gly Lys Arg Ser Thr Ser Ala
               580                 585                 590
   Ala Val Leu Glu Ala Leu Arg Glu Ala His Pro Ile Val Glu Lys Ile
           595                 600                 605
   Leu Gln Tyr Arg Glu Leu Thr Lys Leu Lys Ser Thr Tyr Ile Asp Pro
       610                 615                 620
   Leu Pro Asp Leu Ile His Pro Arg Thr Gly Arg Leu His Thr Arg Phe
   625                 630                 635                 640
   Asn Gln Thr Ala Thr Ala Thr Gly Arg Leu Ser Ser Ser Asp Pro Asn
                   645                 650                 655
   Leu Gln Asn Ile Pro Val Arg Thr Pro Leu Gly Gln Arg Ile Arg Arg
               660                 665                 670
   Ala Phe Ile Ala Glu Glu Gly Trp Leu Leu Val Ala Leu Asp Tyr Ser
           675                 680                 685
   Gln Ile Glu Leu Arg Val Leu Ala His Leu Ser Gly Asp Glu Asn Leu
       690                 695                 700
   Ile Arg Val Phe Gln Glu Gly Arg Asp Ile His Thr Glu Thr Ala Ser
   705                 710                 715                 720
   Trp Met Phe Gly Val Pro Arg Glu Ala Val Asp Pro Leu Met Arg Arg
                   725                 730                 735
   Ala Ala Lys Thr Ile Asn Phe Gly Val Leu Tyr Gly Met Ser Ala His
               740                 745                 750
   Arg Leu Ser Gln Glu Leu Ala Ile Pro Tyr Glu Glu Ala Gln Ala Phe
           755                 760                 765
   Ile Glu Arg Tyr Phe Gln Ser Phe Pro Lys Val Arg Ala Trp Ile Glu
       770                 775                 780
   Lys Thr Leu Glu Glu Gly Arg Arg Arg Gly Tyr Val Glu Thr Leu Phe
   785                 790                 795                 800
   Gly Arg Arg Arg Tyr Val Pro Asp Leu Glu Ala Arg Val Lys Ser Val
                   805                 810                 815
   Arg Glu Ala Ala Glu Arg Met Ala Phe Asn Met Pro Val Gln Gly Thr
               820                 825                 830
   Ala Ala Asp Leu Met Lys Leu Ala Met Val Lys Leu Phe Pro Arg Leu
           835                 840                 845
   Glu Glu Met Gly Ala Arg Met Leu Leu Gln Val His Asp Glu Leu Val
       850                 855                 860
   Leu Glu Ala Pro Lys Glu Arg Ala Glu Ala Val Ala Arg Leu Ala Lys
   865                 870                 875                 880
   Glu Val Met Glu Gly Val Tyr Pro Leu Ala Val Pro Leu Glu Val Glu
                   885                 890                 895
   Val Gly Ile Gly Glu Asp Trp Leu Ser Ala Lys Glu
               900                 905
(2) ANGABEN ZU SEQ ID NO: 11:
   (i) SEQUENZKENNZEICHEN:
      (A) LÄNGE: 949 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 11:
   Met Arg Gly Ser His His His His His His Ala Ala Asp Asp Asp Asp
   1               5                   10                  15
   Lys Met Arg Gly Met Leu Pro Leu Phe Glu Pro Lys Gly Arg Val Leu
               20                  25                  30
   Leu Val Asp Gly His His Leu Ala Tyr Arg Thr Phe His Ala Leu Lys
           35                  40                  45
   Gly Leu Thr Thr Ser Arg Gly Glu Pro Val Gln Ala Val Tyr Gly Phe
       50                  55                  60
   Ala Lys Ser Leu Leu Lys Ala Leu Lys Glu Asp Gly Asp Ala Val Ile
   65                  70                  75                  80
   Val Val Phe Asp Ala Lys Ala Pro Ser Phe Arg His Glu Ala Tyr Gly
                   85                  90                  95
   Gly Tyr Lys Ala Gly Arg Ala Pro Thr Pro Glu Asp Phe Pro Arg Gln
               100                 105                 110
   Leu Ala Leu Ile Lys Glu Leu Val Asp Leu Leu Gly Leu Ala Arg Leu
           115                 120                 125
   Glu Val Pro Gly Tyr Glu Ala Asp Asp Val Leu Ala Ser Leu Ala Lys
       130                 135                 140
   Lys Ala Glu Lys Glu Gly Tyr Glu Val Arg Ile Leu Thr Ala Asp Lys
   145                 150                 155                 160
   Asp Leu Tyr Gln Leu Leu Ser Asp Arg Ile His Val Leu His Pro Glu
                   165                 170                 175
   Gly Tyr Leu Ile Thr Pro Ala Trp Leu Trp Glu Lys Tyr Gly Leu Arg
               180                 185                 190
   Pro Asp Gln Trp Ala Asp Tyr Arg Ala Leu Thr Gly Asp Glu Ser Asp
           195                 200                 205
   Asn Leu Pro Gly Val Lys Gly Ile Gly Glu Lys Thr Ala Arg Lys Leu
       210                 215                 220
   Leu Glu Glu Trp Gly Ser Leu Glu Ala Leu Leu Lys Asn Leu Asp Arg
   225                 230                 235                 240
   Leu Lys Pro Ala Ile Arg Glu Lys Ile Leu Ala His Met Asp Asp Leu
                   245                 250                 255
   Lys Leu Ser Trp Asp Leu Ala Lys Val Arg Thr Asp Leu Pro Leu Glu
               260                 265                 270
   Val Asp Phe Ala Lys Arg Arg Glu Pro Asp Arg Glu Arg Leu Arg Ala
           275                 280                 285
   Phe Leu Glu Arg Leu Glu Phe Gly Ser Leu Leu His Glu Phe Gly Leu
       290                 295                 300
   Leu Glu Ser Pro His Pro Ala Val Val Asp Ile Phe Glu Tyr Asp Ile
   305                 310                 315                 320
   Pro Phe Ala Lys Arg Tyr Leu Ile Asp Lys Gly Leu Ile Pro Met Glu
                   325                 330                 335
   Gly Glu Glu Glu Leu Lys Ile Leu Ala Phe Asp Ile Glu Thr Leu Tyr
               340                 345                 350
   His Glu Gly Glu Glu Phe Gly Lys Gly Pro Ile Ile Met Ile Ser Tyr
           355                 360                 365
   Ala Asp Glu Asn Glu Ala Lys Val Ile Thr Trp Lys Asn Ile Asp Leu
       370                 375                 380
   Pro Tyr Val Glu Val Val Ser Ser Glu Arg Glu Met Ile Lys Arg Phe
   385                 390                 395                 400
   Leu Arg Ile Ile Arg Glu Lys Asp Pro Asp Ile Ile Val Thr Tyr Asn
                   405                 410                 415
   Gly Asp Ser Phe Asp Phe Pro Tyr Leu Ala Lys Arg Ala Glu Lys Leu
               420                 425                 430
   Gly Ile Lys Leu Thr Ile Gly Arg Asp Gly Ser Glu Pro Lys Met Gln
           435                 440                 445
   Arg Ile Gly Asp Met Thr Ala Val Glu Val Lys Gly Arg Ile His Phe
       450                 455                 460
   Asp Leu Tyr His Val Ile Thr Arg Thr Ile Asn Leu Pro Thr Tyr Thr
   465                 470                 475                 480
   Leu Glu Ala Val Tyr Glu Ala Ile Phe Gly Lys Pro Lys Glu Lys Val
   485 490 495
   Tyr Ala Asp Glu Ile Ala Lys Ala Trp Glu Ser Gly Glu Asn Leu Glu
   500 505 510
   Arg Val Ala Lys Tyr Ser Met Glu Asp Ala Lys Ala Thr Tyr Glu Leu
   515 520 525
   Gly Lys Glu Phe Leu Pro Met Glu Ile Gln Leu Ser Glu Arg Leu Leu
   530 535 540
   Trp Leu Tyr Arg Glu Val Glu Arg Pro Leu Ser Ala Val Leu Ala His
   545 550 555 560
   Met Glu Ala Thr Gly Val Arg Leu Asp Val Ala Tyr Leu Arg Ala Leu
   565 570 575
   Ser Leu Glu Val Ala Glu Glu Ile Ala Arg Leu Glu Ala Glu Val Phe
   580 585 590
   Arg Leu Ala Gly His Pro Phe Asn Leu Asn Ser Arg Asp Gln Leu Glu
   595 600 605
   Arg Val Leu Phe Asp Glu Leu Gly Leu Pro Ala Ile Gly Lys Thr Glu
   610 615 620
   Lys Thr Gly Lys Arg Ser Thr Ser Ala Ala Val Leu Glu Ala Leu Arg
   625 630 635 640
   Glu Ala His Pro Ile Val Glu Lys Ile Leu Gln Tyr Arg Glu Leu Thr
   645 650 655
   Lys Leu Lys Ser Thr Tyr Ile Asp Pro Leu Pro Asp Leu Ile His Pro
   660 665 670
   Arg Thr Gly Arg Leu His Thr Arg Phe Asn Gln Thr Ala Thr Ala Thr
   675 680 685
   Gly Arg Leu Ser Ser Ser Asp Pro Asn Leu Gln Asn Ile Pro Val Arg
   690 695 700
   Thr Pro Leu Gly Gln Arg Ile Arg Arg Ala Phe Ile Ala Glu Glu Gly
   705 710 715 720
   Trp Leu Leu Val Ala Leu Asp Tyr Ser Gln Ile Glu Leu Arg Val Leu
   725 730 735
   Ala His Leu Ser Gly Asp Glu Asn Leu Ile Arg Val Phe Gln Glu Gly
   740 745 750
   Arg Asp Ile His Thr Glu Thr Ala Ser Trp Met Phe Gly Val Pro Arg
           755                 760                 765
   Glu Ala Val Asp Pro Leu Met Arg Arg Ala Ala Lys Thr Ile Asn Phe
       770                 775                 780
   Gly Val Leu Tyr Gly Met Ser Ala His Arg Leu Ser Gln Glu Leu Ala
   785                 790                 795                 800
   Ile Pro Tyr Glu Glu Ala Gln Ala Phe Ile Glu Arg Tyr Phe Gln Ser
                   805                 810                 815
   Phe Pro Lys Val Arg Ala Trp Ile Glu Lys Thr Leu Glu Glu Gly Arg
               820                 825                 830
   Arg Arg Gly Tyr Val Glu Thr Leu Phe Gly Arg Arg Arg Tyr Val Pro
           835                 840                 845
   Asp Leu Glu Ala Arg Val Lys Ser Val Arg Glu Ala Ala Glu Arg Met
       850                 855                 860
   Ala Phe Asn Met Pro Val Gln Gly Thr Ala Ala Asp Leu Met Lys Leu
   865                 870                 875                 880
   Ala Met Val Lys Leu Phe Pro Arg Leu Glu Glu Met Gly Ala Arg Met
                   885                 890                 895
   Leu Leu Gln Val His Asp Glu Leu Val Leu Glu Ala Pro Lys Glu Arg
               900                 905                 910
   Ala Glu Ala Val Ala Arg Leu Ala Lys Glu Val Met Glu Gly Val Tyr
           915                 920                 925
   Pro Leu Ala Val Pro Leu Glu Val Glu Val Gly Ile Gly Glu Asp Trp
       930                 935                 940
   Leu Ser Ala Lys Glu
   945
(2) ANGABEN ZU SEQ ID NO: 12:
   (i) SEQUENZKENNZEICHEN:
      (A) LÄNGE: 982 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 12:
   Met Arg Gly Ser His His His His His His Ala Ala Asp Asp Asp Asp
   1               5                   10                  15
   Lys Met Arg Gly Met Leu Pro Leu Phe Glu Pro Lys Gly Arg Val Leu
               20                  25                  30
   Leu Val Asp Gly His His Leu Ala Tyr Arg Thr Phe His Ala Leu Lys
           35                  40                  45
   Gly Leu Thr Thr Ser Arg Gly Glu Pro Val Gln Ala Val Tyr Gly Phe
       50                  55                  60
   Ala Lys Ser Leu Leu Lys Ala Leu Lys Glu Asp Gly Asp Ala Val Ile
   65                  70                  75                  80
   Val Val Phe Asp Ala Lys Ala Pro Ser Phe Arg His Glu Ala Tyr Gly
                   85                  90                  95
   Gly Tyr Lys Ala Gly Arg Ala Pro Thr Pro Glu Asp Phe Pro Arg Gln
               100                 105                 110
   Leu Ala Leu Ile Lys Glu Leu Val Asp Leu Leu Gly Leu Ala Arg Leu
           115                 120                 125
   Glu Val Pro Gly Tyr Glu Ala Asp Asp Val Leu Ala Ser Leu Ala Lys
       130                 135                 140
   Lys Ala Glu Lys Glu Gly Tyr Glu Val Arg Ile Leu Thr Ala Asp Lys
   145                 150                 155                 160
   Asp Leu Tyr Gln Leu Leu Ser Asp Arg Ile His Val Leu His Pro Glu
                   165                 170                 175
   Gly Tyr Leu Ile Thr Pro Ala Trp Leu Trp Glu Lys Tyr Gly Leu Arg
               180                 185                 190
   Pro Asp Gln Trp Ala Asp Tyr Arg Ala Leu Thr Gly Asp Glu Ser Asp
           195                 200                 205
   Asn Leu Pro Gly Val Lys Gly Ile Gly Glu Lys Thr Ala Arg Lys Leu
       210                 215                 220
   Leu Glu Glu Trp Gly Ser Leu Glu Ala Leu Leu Lys Asn Leu Asp Arg
   225                 230                 235                 240
   Leu Lys Pro Ala Ile Arg Glu Lys Ile Leu Ala His Met Asp Asp Leu
                   245                 250                 255
   Lys Leu Ser Trp Asp Leu Ala Lys Val Arg Thr Asp Leu Pro Leu Glu
               260                 265                 270
   Val Asp Phe Ala Lys Arg Arg Glu Pro Asp Arg Glu Arg Leu Arg Ala
           275                 280                 285
   Phe Leu Glu Arg Leu Glu Phe Gly Ser Leu Leu His Glu Phe Gly Leu
       290                 295                 300
   Leu Glu Ser Pro Val Arg Glu His Pro Ala Val Val Asp Ile Phe Glu
   305                 310                 315                 320
   Tyr Asp Ile Pro Phe Ala Lys Arg Tyr Leu Ile Asp Lys Gly Leu Ile
                   325                 330                 335
   Pro Met Glu Gly Glu Glu Glu Leu Lys Ile Leu Ala Phe Asp Ile Glu
               340                 345                 350
   Thr Leu Tyr His Glu Gly Glu Glu Phe Gly Lys Gly Pro Ile Ile Met
           355                 360                 365
   Ile Ser Tyr Ala Asp Glu Asn Glu Ala Lys Val Ile Thr Trp Lys Asn
       370                 375                 380
   Ile Asp Leu Pro Tyr Val Glu Val Val Ser Ser Glu Arg Glu Met Ile
   385                 390                 395                 400
   Lys Arg Phe Leu Arg Ile Ile Arg Glu Lys Asp Pro Asp Ile Ile Val
                   405                 410                 415
   Thr Tyr Asn Gly Asp Ser Phe Asp Phe Pro Tyr Leu Ala Lys Arg Ala
               420                 425                 430
   Glu Lys Leu Gly Ile Lys Leu Thr Ile Gly Arg Asp Gly Ser Glu Pro
           435                 440                 445
   Lys Met Gln Arg Ile Gly Asp Met Thr Ala Val Glu Val Lys Gly Arg
       450                 455                 460
   Ile His Phe Asp Leu Tyr His Val Ile Thr Arg Thr Ile Asn Leu Pro
   465                 470                 475                 480
   Thr Tyr Thr Leu Glu Ala Val Tyr Glu Ala Ile Phe Gly Lys Pro Lys
                   485                 490                 495
   Glu Lys Val Tyr Ala Asp Glu Ile Ala Lys Ala Trp Glu Ser Gly Glu
               500                 505                 510
   Asn Leu Glu Arg Val Ala Lys Tyr Ser Met Glu Asp Ala Lys Ala Thr
           515                 520                 525
   Tyr Glu Leu Gly Lys Glu Phe Leu Pro Met Glu Ile Gln Leu Ser Arg
   530 535 540
   Leu Val Gly Gln Pro Leu Trp Asp Val Ser Arg Ser Ser Thr Gly Asn
   545 550 555 560
   Leu Val Glu Trp Phe Leu Leu Arg Lys Ala Tyr Glu Arg Asn Glu Val
   565 570 575
   Ala Pro Asn Lys Pro Ser Glu Glu Glu Tyr Gln Arg Arg Leu Arg Glu
   580 585 590
   Ser Tyr Thr Gly Gly Phe Val Arg Leu Asp Val Ala Tyr Leu Arg Ala
   595 600 605
   Leu Ser Leu Glu Val Ala Glu Glu Ile Ala Arg Leu Glu Ala Glu Val
   610 615 620
   Phe Arg Leu Ala Gly His Pro Phe Asn Leu Asn Ser Arg Asp Gln Leu
   625 630 635 640
   Glu Arg Val Leu Phe Asp Glu Leu Gly Leu Pro Ala Ile Gly Lys Thr
   645 650 655
   Glu Lys Thr Gly Lys Arg Ser Thr Ser Ala Ala Val Leu Glu Ala Leu
   660 665 670
   Arg Glu Ala His Pro Ile Val Glu Lys Ile Leu Gln Tyr Arg Glu Leu
   675 680 685
   Thr Lys Leu Lys Ser Thr Tyr Ile Asp Pro Leu Pro Asp Leu Ile His
   690 695 700
   Pro Arg Thr Gly Arg Leu His Thr Arg Phe Asn Gln Thr Ala Thr Ala
   705 710 715 720
   Thr Gly Arg Leu Ser Ser Ser Asp Pro Asn Leu Gln Asn Ile Pro Val
   725 730 735
   Arg Thr Pro Leu Gly Gln Arg Ile Arg Arg Ala Phe Ile Ala Glu Glu
   740 745 750
   Gly Trp Leu Leu Val Ala Leu Asp Tyr Ser Gln Ile Glu Leu Arg Val
   755 760 765
   Leu Ala His Leu Ser Gly Asp Glu Asn Leu Ile Arg Val Phe Gln Glu
   770 775 780
   Gly Arg Asp Ile His Thr Glu Thr Ala Ser Trp Met Phe Gly Val Pro
   785 790 795 800
   Arg Glu Ala Val Asp Pro Leu Met Arg Arg Ala Ala Lys Thr Ile Asn
                   805                 810                 815
   Phe Gly Val Leu Tyr Gly Met Ser Ala His Arg Leu Ser Gln Glu Leu
               820                 825                 830
   Ala Ile Pro Tyr Glu Glu Ala Gln Ala Phe Ile Glu Arg Tyr Phe Gln
           835                 840                 845
   Ser Phe Pro Lys Val Arg Ala Trp Ile Glu Lys Thr Leu Glu Glu Gly
       850                 855                 860
   Arg Arg Arg Gly Tyr Val Glu Thr Leu Phe Gly Arg Arg Arg Tyr Val
   865                 870                 875                 880
   Pro Asp Leu Glu Ala Arg Val Lys Ser Val Arg Glu Ala Ala Glu Arg
                   885                 890                 895
   Met Ala Phe Asn Met Pro Val Gln Gly Thr Ala Ala Asp Leu Met Lys
               900                 905                 910
   Leu Ala Met Val Lys Leu Phe Pro Arg Leu Glu Glu Met Gly Ala Arg
           915                 920                 925
   Met Leu Leu Gln Val His Asp Glu Leu Val Leu Glu Ala Pro Lys Glu
       930                 935                 940
   Arg Ala Glu Ala Val Ala Arg Leu Ala Lys Glu Val Met Glu Gly Val
   945                 950                 955                 960
   Tyr Pro Leu Ala Val Pro Leu Glu Val Glu Val Gly Ile Gly Glu Asp
                   965                 970                 975
   Trp Leu Ser Ala Lys Glu
               980
(2) ANGABEN ZU SEQ ID NO: 13:
   (i) SEQUENZKENNZEICHEN:
      (A) LÄNGE: 66 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "oligonucleotide"
   (ix) MERKMAL:
      (A) NAME/SCHLÜSSEL: CDS
      (B) LAGE:1..66
   (xi) SEQUENZBESCHREIBUNG: SEQ ID NO: 13:
   GAA TTC ATG AGG GGC TCG CAT CAC CAT CAC CAT CAC GCT GCT GAC GAT
   48
   GAC GAT AAA ATG AGG GGC
   66
(2) ANGABEN ZU SEQ ID NO: 14:
   (i) SEQUENZKENNZEICHEN:
      (A) LÄNGE: 20 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 14:
   Met Arg Gly Ser His His His His His His Ala Ala Asp Asp Asp Asp
     1               5                  10                  15
   Lys Met Arg Gly
               20

## Beschreibung

Gegenstand der vorliegenden Erfindung sind Polymerasenchimären, die aus Aminosäurefragmenten, die Domänen repräsentieren, bestehen und die - im Hinblick auf eine bestimmte Verwendung- vorteilhafte Eigenschaften von natürlich vorkommenden Polymerasen in sich vereinen. Überraschenderweise konnte gezeigt werden, daß die Domänen aus den unterschiedlichen Enzymen in der Chimäre aktiv sind und ein kooperatives Verhalten zeigen. Insbesondere sind solche Polymerasen-Chimären Gegenstand der vorliegenden Erfindung, bei denen die Polymeraseaktivität aufweisende Domäne und die 3'-5'-Exonukleaseaktivität aufweisende Domäne aus verschiedenen Enzymen stammen. Derartige Chimäre können zusätzlich RT-Aktivität aufweisen. Weiterhin ist Gegenstand der vorliegenden Erfindung ein Verfahren zur Herstellung der erfindungsgemäßen Chimären sowie die Verwendung dieser Chimären bei der Synthese von Nukleinsäuren z.B. während der Polymerase-Ketten-Reaktion. Ein weiterer Gegenstand der vorliegenden Erfindung ist ein Kit, der die erfindungsgemäßen Polymerasenchimären enthält.

Nach Braithwaite, D.K. und Ito, J. (1993) Nucl. Acids Res. 21, 787-802 werden die DNA Polymerasen nach den Übereinstimmungen in ihren Aminosäuresequenzen in drei Hauptfamilien mit Unterklassen eingeteilt. Eine Zusammenfassung der gefundenen Motive und konservierten Aminosäuren geben Joyce, C.M. und Steitz, T.A. (1994) Annu. Rev. Biochem. 63, 777-822. In Prokaryoten werden hauptsächlich drei Polymerasen unterschieden: Polymerase I, II und III. Diese Polymerasen unterscheiden sich untereinander bezüglich ihrer Funktion in der Zelle und bezüglich ihrer Eigenschaften. Die DNA Polymerase I gilt als Reparaturenzym, besitzt häufig sowohl 5'-3'- als auch 3'-5'- Exonukleaseaktivität. Polymerase II scheint die DNA Synthese zu erleichtern, die von einem beschädigten Matrizenstrang ausgeht und bewahrt daher Mutationen. Die Polymerase III ist das Replikationsenzym der Zelle, sie synthetisiert Nukleotide mit hoher Geschwindigkeit (ca. 30,000 per Minute) und gilt als sehr prozessiv. Polymerase III besitzt keine 5'-3'-Exonukleaseaktivität. Andere Eigenschaften von Polymerasen werden bedingt durch ihre Herkunft wie z.B. die Thermostabilität oder auch die Prozessivität.

Je nach Anwendung sind bestimmte Eigenschaften von Polymerasen wünschenswert. Für die PCR z.B. werden thermostabile, fidele - d.h. Polymerasen mit proofreading-Aktivität - prozessive und schnellsynthetisierende Polymerasen bevorzugt. Bei der Sequenzierung werden Enzyme bevorzugt, die wenig zwischen Dideoxy- und Deoxy-Nukleotiden diskriminieren. Die Proofreading-Aktivität der Polymerasen hingegen, d.h. 3'-5'- Exonukleaseaktivität, ist bei der Sequenzierung nicht wünschenswert. Für manche Anwendungen, z.B. PCR , ist es wünschenswert, wenn die Polymerase keine oder wenig 5'-3'- Exonukleaseaktivität (5'-Nukleaseaktivität) aufweist.

Polymerasen können sich weiterhin unterscheiden in ihrer Fähigkeit RNA als Template zu akzeptieren, d.h. bezüglich ihrer Reversen Transkriptasen (RT) -Aktivität. Die RT-Aktivität kann von der Gegenwart an Mangan- und/oder Magnesium-Ionen abhängig sein. Oftmals ist es wünschenswert, wenn die RT-Aktivität der Polymerase von Mangan- Ionen - unabhängig ist, weil die Lesegenauigkeit der Polymerase in Gegenwart von Mangan - Ionen sinkt. Polymerasen unterscheiden sich weiterhin in ihrer Prozessivität, die ebenfalls eine für viele Anwendungen wünschenswerte Eigenschaft darstellt.

Es besteht daher das Bedürfnis, Polymerasen bezüglich ihrer Eigenschaften im Hinblick auf eine bestimmte Anwendung zu optimieren. Dies wurde in der Vergangenheit oftmals durch das Einführen von Mutationen oder durch Deletion von Funktionen der Polymerasen erreicht.

So wurde z.B. das Ausschalten der 5'-3'- Exonukleaseaktivität sowohl durch das Einführen von Mutationen (Merkens, L. S. (1995) *Biochem. Biophys. Acta* 1264,243-248) als auch durch Verkürzung erreicht (Jacobsen, H. (1974) *Eur. J. Biochem.* 45, 623-627; Barnes, W. M. (1992) *Gene* 112, 29-35). Die Diskriminierungsfähigkeit der Polymerasen gegenüber Dideoxy- und Deoxy-Nukleotiden wurde durch das Einführen von Punktmutationen herabgesetzt (Tabor S. und Richardson, C. C. (1995) *Proc. Natl. Acad. Sci.* 92, 6339-6343). Tabor und Richardson beschreiben die Konstruktion von Active-site-Hybriden. Ferner sind Polymerasenchimären mit verbesserter Prozessivität, welche durch Einbringen einer Thioredoxin-Bindestelle erreicht wird, bekannt (WO 97/29209). Darüber hinaus beschreiben Abramson, R.D. und Gelfand, D. Polymerasenchimäre basierend auf Schnittstellen im Loop zwischen den Polymerase- und 3'-5'-Exonukleasedomänen, die sich insbesondere durch geringere 5'-3'-Exonukleaseaktivität auszeichnen (US 5.466.591).

Die Aufgabe, Polymerasen mit optimierten Eigenschaften bereitzustellen, wurde durch die vorliegende Erfindung erstmals durch das Herstellen von Polymerasenchimären durch den Austausch strukturell und funktionell voneinander unabhängiger Domänen gelöst. Als Domäne im Sinne der vorliegenden Erfindung sind Bereiche zu verstehen, die alle essentiellen Zentren bzw. alle funktionell wichtigen Aminosäuren enthalten, so daß die Domäne ihre Funktion im wesentlichen behält. Es können daher auch nur Teile, d.h. funktionierende Fragmente von Domänen ausgetauscht werden. So können diese Domänen im Sinne der vorliegenden Erfindung als funktionelle Aminosäurefragmente bezeichnet werden. Die Chimäre kann darüber hinaus durch Mutationen oder Verkürzungen weiter verändert werden. Falls es vorteilhaft erscheint, können in die Chimäre Mutationen eingeführt werden, die ihre Eigenschaften im Hinblick auf die jeweilige Anwendung weiter optimieren. So können beispielsweise Mutationen eingeführt werden, die die Diskriminierungsfähigkeit der Polymerasen gegenüber Dideoxy- und Deoxy-Nukleotiden herabsetzt. Oder es können durch die Einführung von Mutationen oder durch Verkürzung erwünschte Eigenschaften wie z. B. die Prozessivität verstärkt oder eingeführt werden. Es können aber auch durch die Einführung von Mutationen oder durch Verkürzungen unerwünschte Eigenschaften ausgeschaltet werden, z.B. die 5'-Nukleaseaktivität.

Und somit sind Polymerasenchimären Gegenstand der vorliegenden Erfindung, die - im Hinblick auf eine bestimmte Verwendung - vorteilhafte Eigenschaften von natürlich vorkommenden Polymerasen in sich vereinen. Die erfindungsgemäßen Polymerasenchimären bestehen aus funktionellen Aminosäurefragmenten unterschiedlicher Enzyme, die vorzugsweise Domänen unterschiedlicher Enzyme repräsentieren. Überraschenderweise konnte gezeigt werden, daß die Domänen aus den unterschiedlichen Enzymen in der Chimäre aktiv sind und ein kooperatives Verhalten der Domänen untereinander zeigen. Gegenstand der vorliegenden Erfindung sind weiterhin generelle Verfahren zur Herstellung von Polymerasenchimären mit optimierten Eigenschaften. Durch diese erfindungsgemäßen Verfahren werden somit das Design einer Chimären aus einer beliebigen Kombinationen von Enzymen möglich, indem Domänen ausgetauscht werden. Weiterhin ist bevorzugt, daß die Wechselwirkungen in den Kontaktstellen der Domänen durch verschiedene Verfahren noch weiter aufeinander abgestimmt werden. Dadurch kann beispielsweise die Thermostabilität der Chimären erhöht werden. Ein weiterer Gegenstand der Erfindung ist ein Kit zur Synthese von Nukleinsäuren, der eine erfindungsgemäße Chimäre enthält.

Für die PCR werden in der Praxis zunehmend thermostabile DNA Polymerasen mit Korrekturlesefunktion eingesetzt. Zur Amplifikation langer DNA Moleküle hat sich insbesondere das Einsetzen von Mischungen aus *Taq* Polymerase und thermostabiler Korrekturlese-DNA Polymerase (wie *Pfu, Pwo, Vent* Polymerase) bewährt. Es war daher weiterhin Gegenstand der vorliegenden Erfindung , die hohe Prozessivität und Thermostabilität der *Taq* Polymerase mit der 3'-5'-Exonukleaseaktivität einer anderen DNA Polymerase in einem Enzym zu vereinen. Gegenstand der vorliegenden Erfindung sind daher insbesondere thermostabile Polymerasenchimäre, die eine Prozessivität aufweisen, die mindestens der der Taq Polymerase entspricht, sowie eine geringe Fehlerrate beim Einbau der Nukleotide in die Polymerkette während der Amplifikation aufweisen durch das Vorhandensein einer 3'-5'-Exonuklease-Aktivität (Proofreading-Aktivität). Durch die Kombination dieser beiden Eigenschaften kann beispielsweise eine Chimäre generiert werden, die in der Lage ist, lange PCR Produkte zu machen, d.h. Nukleinsäurefragmente die größer sind als 2 kb. Die erfindungsgemäße Chimäre eignet sich ebenfalls für die Vervielfältigung kürzerer Fragmente.

Gegenstand der vorliegenden Erfindung ist daher insbesondere eine Polymerasenchimäre, die zusammengesetzt ist aus funktionellen Aminosäurefragmenten von zwei unterschiedlichen Polymerasen, wobei die erste oder die zweite der Polymerasen 3'-5'-Exonukleaseaktivität aufweist und die Polymerasenchimäre sowohl 5'-3'-Polymeraseaktivität als auch 3'-5'-Exonukleaseaktivität aufweist. Die Polymerasen können natürlich vorkommende oder rekombinante Polymerasen sein. Die erfindungsgemäße Polymerasenchimäre kann aus funktionellen Aminosäurefragmenten von zwei oder mehr unterschiedlichen Polymerasen zusammengesetzt sein. Die erfindungsgemäße Polymerasenchimäre kann aus zwei oder mehreren funktionellen Aminosäurefragmenten der unterschiedlichen Polymerasen zusammengesetzt sein. Die Aminosäuresequenz des Fragmentes kann der natürlich vorkommenden Sequenz der Polymerase oder einer durch Mutationen veränderten Sequenz entsprechen.

Die Aminosäurefragmente, aus denen die Polymerasenchimäre zusammengesetzt ist, entsprechen bevorzugterweise jeweils funktionalen Polymerasendomänen der ersten oder zweiten Polymerase. Eine funktionale Polymerasendomäne im Sinne der vorliegenden Erfindung ist ein Bereich, der alle für die Aktivität essentiellen Aminosäuren beinhaltet, und wird in folgendem kurz Domäne genannt.

Insbesondere ist Gegenstand der vorliegenden Erfindung eine Polymerasenchimäre zusammengesetzt aus funktionellen Aminosäurefragmenten (hier kurz Domänen) von mindestens zwei unterschiedlichen Polymerasen, wobei die Polymeraseaktivität aufweisende Domäne von der ersten Polymerase und die 3'-Exonukleaseaktivität aufweisende Domäne von der zweiten Polymerase stammt und wobei die Aminosäuresequenz der Polymerasenchimären der SEQ ID NO: 8, 10 oder 12 entspricht. Des weiteren kann diese Chimäre noch 5'-Exonukleaseaktivität aufweisen, wobei die 5'-Exonukleaseaktivität aufweisende Domäne homolog zur ersten oder zur zweiten Polymerase sein kann. Jedoch ist es auch möglich, daß die 5'-Exonukleasedomäne teilweise oder vollständig deletiert ist bzw. Punktmutationen aufweist. Die erfindungsgemäße Polymerasenchimäre kann des weiteren Reverse Transkriptase (RT) Aktivität aufweisen.

Bevorzugt ist weiterhin, daß ein Teil der Aminosäurefragmente der Polymerasenchimären einem Teil der Aminosäuresequenz der Taq-Polymerase entspricht.

Die Polymerase, deren 3'-5'-Exonukleaseaktivität aufweisende Domäne bzw. Aminosäurefragment in die Chimäre eingebaut wurde, kann beispielsweise eine Pol-I-Typ-Polymerase oder auch eine Pol-II-Typ-Polymerase sein. Vertreter der Pol-I-Typ-Polymerase mit 3'-5'-Exonukleaseaktivität sind z.B. *Escherichia .coli* Polymerase I (Ec.1), *Salmonella* Polymerase 1, *Bacillus* Polymerase I, *Thermosiphon* Polymerase I sowie die *Thermatoga neapolitana* Polymerase (Tne). Vertreter der Pol-II-Typ-Polymerase mit 3'-5'-Exonukleaseaktivität sind z.B die *Pyrrococcus woesie* Polymerase (Pwo), *Pyrococcus furiosus* Polymerase (Pfu), *Thermococcus litoralis* Polymerase (Tli), *Pyrodictum abyssi.*

Im folgenden wird auf die beispielhaft genannten Vertreter der Pol-I-Typ und Pol-II-Typ Polymerasen näher eingegangen:

Die *Taq* DNA Polymerase aus *Thermus aquaticus* (*Taq* Polymerase), die *Escherichia coli* DNA Polymerase I (*E. coli* polI) und die *Thermotoga neapolitana* DNA Polymerase (*Tne* Polymerase) sind bakterielle DNA Polymerasen der Familie A. Es sind DNA Polymerasen vom polI-Typ, da die verschiedenen enzymatischen Aktivitäten in relativ gleicher Weise in verschiedenen Domänen lokalisiert sind wie bei der *E. coli* polI. Die *Pyrococcus woesi* DNA Polymerase (*Pwo* Polymerase) ist, wie die *Thermococcus litorales* DNA Polymerase (Vent^{™} Polymerase) und die *Pyrococcus furiosus* DNA Polymerase (*Pfu* Polymerase), eine archaebakterielle DNA Polymerase der Familie B.

Die *Taq* Polymerase ist beschrieben von Chien, A. et al. (1976) J. Bacteriol. 127, 1550-1557, Kaledin, A.S. et al. (1980) Biokhimiya 45, 644-651 und Lawyer, F.C et al. (1989) J. Biol. Chem. 264, 6427-6437. Ursprünglich wurde sie aus dem thermophilen Eubakterium *Thermus aquaticus* isoiert, später in *E. coli* kloniert. Das Enzym hat ein Molekulargewicht von 94 kDa und ist als Monomer aktiv. Die *Taq* Polymerase ist geeignet zum Einsatz in der Polymerasekettenreaktion (PCR), da sie eine hohe Thermostabilität (Halbwertszeit von 40 Minuten bei 95°C / 5 Minuten bei 100°C) und eine hoch prozessive 5'-3'-DNA-Polymerase (Polymerisationsrate: 75 Nukleotide pro Sekunde) aufweist. Neben der Polymeraseaktivität wurde von Longley et al. (1990) Nucl. Acids Res. 18, 7317-7322 eine 5'-Nukleaseaktivität nachgewiesen. Das Enzym zeigt keine 3'-5'-Exonukleaseaktivität, so daß beim Einbau der vier Desoxyribonukleotidtriphosphate zur sukzessiven Verlängerung von Polynukleotidketten Fehler entstehen, die bei der Genamplifikation stören (Fehlerrate: 2 x 10⁻⁴ Fehler/Base, Cha, R. S. und Thilly, W. G. (1993) PCR Methods Applic. 3, 18-29). Die Tertiärstruktur der *Taq* Polymerase ist seit 1995 bekannt (Kim et al., 1995, Korolev et al., 1995).

Die *E. coli* polI ist beschrieben in Kornberg, A. und Baker, T. A. (1992) DNA Replication, 2. Aufl., Freeman, New York, 113-165. Das Enzym hat ein Molekulargewicht von 103 kDa und ist als Monomer aktiv. Die *E. coli* poll besitzt 5'-Nuklease- und 5'-3'-DNA-Polymeraseaktivität. Im Gegensatz zur *Taq* Polymerase besitzt sie zusätzlich eine 3'-5'-Exonukleaseaktivität als Korrekturlesefunktion. Die E. *coli* polI und deren Klenow Fragment (Jacobsen, H. et al. (1974) Eur. J. Biochem. 45, 623-627) wurden vor der Einführung der *Taq* Polymerase für die PCR eingesetzt. Aufgrund ihrer geringen Thermostabilität sind sie jedoch weniger gut geeignet, da sie jeden Zyklus neu zugesetzt werden müssen. Die Tertiärstruktur des Klenow Fragmentes der *E. coli* polI ist seit 1983 bekannt (Brick, P. et al. (1983) J. Mol. Biol. 166, 453-456, Ollis, D.L. et al. (1985) Nature 313, 762-766 und Beese, L.S. et al. (1993) Science 260, 352-355).

Die *Tne* Polymerase wurde aus dem thermophilen Eubakterium *Thermotoga neapolitana* isoliert und später in E. *coli* kloniert. Die Aminosäuresequenz der *Tne* Polymerase ist der *Thermotoga maritima* DNA Polymerase (UITma^{™} Polymerase) ähnlich (persönliche Auskunft Dr. B. Frey). Sie weist hohe Thermostabilität, 5'-Nukleaseaktivität, 3'-5'-Exonukleaseaktivität und 5'-3'-DNA-Polymeraseaktivität auf. Ein Nachteil ist die geringere Polymerisationsrate verglichen mit der der *Taq* Polymerase. Die in der Aminosäuresequenz ähnliche UITma^{™} Polymerase wird für die PCR verwendet, wenn hohe Genauigkeit benötigt wird. Von der Struktur der *Tne* Polymerase ist bisher nur die Aminosäuresequenz bekannt (Boehringer Mannheim). Das Enzym ist jedoch homolog zur *E. coli* polI, so daß, obwohl die Tertiärstruktur nicht bekannt ist, die Möglichkeit des Homologiemodelling besteht.

Die *Pfu* Polymerase wurde aus dem hyperthermophilen, marinen Archaebakterium *Pyrococcus furiosus* isoliert. Sie weist hohe Thermostabilität (95% Aktivität nach einer Stunde bei 95°C), 3'-5'-Exonukleaseaktivität und 5'-3'-DNA-Polymeraseaktivität auf (Lundberg, K. S. et al. (1991) Gene 108, 1-6). Die Genauigkeit der DNA-Synthese ist ca. zehnmal höher als bei der *Taq* Polymerase. Sie wird für die PCR verwendet, wenn hohe Genauigkeit benötigt wird. Von der Struktur ist bisher nur die Aminosäuresequenz bekannt.

Die *Pwo* Polymerase (PCR Applications Manual (1995), Boehringer Mannheim GmbH, Biochemica, 28-32) wurde ursprünglich aus dem hyperthermophilen Archaebakterium *Pyrococcus* woesi isoliert und später in *E. coli* kloniert. Das Enzym hat ein Molekulargewicht von etwa 90 kDa und ist als Monomer aktiv. Die *Pwo* Polymerase besitzt einen höhere Thermostabilität als die *Taq* Polymerase (Halbwertszeit > 2 Stunden bei 100°C), eine hoch prozessive 5'-3'-DNA-Polymeraseaktivität und eine hohe 3'-5'-Exonukleaseaktivität, wodurch die Genauigkeit der DNA-Synthese erhöht wird. Das Enzym hat keine 5'-Nukleaseaktivität. Die Polymerisationsrate (30 Nukleotide pro Sekunde) ist geringer als bei der *Taq* Polymerase. Das Enzym wird in der PCR eingesetzt, wenn hohe Genauigkeit gefordert ist. Die Genauigkeit der DNA-Synthese ist mehr als 10 mal höher als bei Verwendung der *Taq* Polymerase.

Die Ath-Polymerase wurde aus dem thermophilen Archaebakterium Anaerocellum thermophium isoliert und später in E.coli kloniert. Die Ath-Polymerase weist hohe Thermostabilität auf und weist nach einer Inkubation von 30 min bei 80°C in Abwesenheit von stabilisierenden Detergentien wenigestens noch 90% der ursprünglichen Aktivität auf. Die Polymerase weist weiterhin RT-Aktivität in Gegenwart von Magnesium-Ionen auf. Ath-Polymerase ist hinterlegt bei der deutschen Sammlung von Mikroorganismen und Zellkulturen GmbH, Mascheroder Weg 1b, D38124 Braunschweig DSM Accession Nr. 8995. Die Ath-Polymerase weist 5'-3'-Polymerase-aktivität, 5'-3'-Exonukleaseaktivität aber keine 3'-5'-Exonuldeaseaktivität auf.

In die Aminosäuresequenz der Polymerasenchimären können weiterhin Histidin-tags oder andere Reinigungshilfen für die verbesserte Aufreinigung eingebaut sein.

Zum Einfügen einer 3'-5'-Exonukleaseaktivität eine Polymerase in eine andere beispielsweise in die *Taq* Polymerase gibt es hauptsächlich vier Verfahren, die ebenfalls Gegenstand der vorliegenden Erfindung sind:

### 1. Das Einfügen des 3'-5'-Exonukleasebereiches einer anderen DNA Polymerase durch Austausch eines Molekülbereichs der Taq Polymerase.

Dieser Ansatz ist insbesondere geeignet, da die *Taq* Polymerase homolog zur *E. coli* polI ist, die aus funktionell und strukturell voneinander unabhängigen Domänen besteht (Joyce, C.M. und Steitz, T.A. (1987) TIBS 12, 288-292) und als Modell für andere DNA Polymerasen dienen kann (Joyce, C.M. (1991) Curr. Opin. Struct. Biol. 1, 123-129). Geeignet für den Austausch sind DNA Polymerasen, bei denen eine 3'-S' Exonukleaseaktivität nachgewiesen ist, deren DNA-Sequenz bekannt ist und das für die 3'-5'-Exonukleaseaktivität kodierende Gen zugänglich ist. Für ein rationales Proteindesign anhand von Modellstrukturen ist es zusätzlich von Vorteil, daß der 3'-5'-Exonukleasebereich und der Polymerasebereich zur *E. coli* polI homolog ist. Der 3'-5'-Exonukleasebereich sollte sich vorzugsweise gut in die Struktur der *E. coli* polI einfügen und an den Polymerasebereich der *Taq* Polymerase anfügen. Eine aufgeklärte Tertiärstruktur mit zugänglichen Strukturdaten sowie hohe Thermostabilität des Proteins sind weitere Vorteile.

Folgende DNA Polymerasen sind daher beispielsweise geeignet:

### a. E. coli polI

Die *E. coli* polI erfüllt, bis auf die Thermostabilität, alle oben aufgeführten Bedingungen. Die Tertiärstruktur des Klenow Fragmentes ist in der Brookhavendatenbank zugänglich und sie gehört, wie die *Taq* Polymerase, zur Familie A der DNA Polymerasen. Die Identität in der Aminosäuresequenz beträgt 32%. Bei Berücksichtigung der bekannten Domänenstruktur finden sich die größten Übereinstimmungen im N-terminalen und im C-terminalen Bereich der beiden Proteine (32 % Identität in den 5'-Nukleasedomänen, 49% Identität in den Polymerasedomänen). Im Bereich der 3'-5'-Exonukleasedomäne weist die kürzere *Taq* Polymerase mehrere Deletionen auf (14% Identität in 3'-5'-Exonukleasedomäne und Zwischendomäne). Da die *E. coli* polI thermolabil ist und die Wechselwirkungen an der Grenzfläche zwischen den beiden Domänen im chimären Protein nicht mehr optimal sind, ist es wahrscheinlich, daß auch die Proteinchimäre geringere Thermostabilität als die *Taq* Polymerase aufweist. Diese kann durch nachträgliche Modifizierung von Aminosäuren an der Grenzfläche behoben werden.

### b. Thermostabile DNA Polymerasen

Von den thermostabilen DNA Polymerasen mit 3'-5'-Exonuklease, die heute zur PCR eingesetzt werden, scheinen die *Pwo* Polymerase, die *Pfu* Polymerase, die Vent^{™} Polymerase, die *Tne* Polymerase und die UITma^{™} Polymerase zur Kombination mit der *Taq* DNA Polymerase geeignet. Die Gene sind (über die Firma Boehringer Mannheim) zugänglich von der *Pwo* Polymerase und der *Tne* Polymerase. Die Pfu Polymerase ist erhältlich von Stratagene Inc. Für ein rationales Proteindesign ist die *Tne* Polymerase aufgrund ihrer Homologie zur *Taq* Polymerase und *E. coli* poll gut geeignet. Bei der Verwendung der *Pfu* Polymerase sind Planungen nur anhand von Aminosäuresequenzalignments unter Berücksichtigung bekannter konservierter, für die Funktion essentieller Aminosäuren und Motive möglich.

### 2. Die Modifikation der Taq DNA Polymerase in der Zwischendomäne

Zum Einfügen einer 3'-5'-Exonukleaseaktivität müssen alle für die Aktivität essentiellen Aminosäuren in die Struktur eingefügt werden. Nach heutigen Kenntnisstand betrifft das besonders die drei Motive Exo I, Exo II und Exo III. Die essentiellen Motive müssen außerdem in geeigneter Art und Weise verknüpft werden, um in die für die Katalyse notwendige räumliche Lage gebracht zu werden.

Die Veränderung der *Taq* DNA Polymerase im Polymerasebereich ist ebenfalls möglich. Auch ein de novo Design von Polymerasen ist prinzipiell denkbar.

Die erfindungsgemäßen Chimären können weiterhin optimiert werden durch:
1. Entfernung der 5'-Nukleasedomäne (möglich auch proteolytisch) oder nachträgliche Inaktivierung der 5'-Nukleaseaktivität (beschrieben in Merkens, L. S. (1995) Biochem. Biophys. Acta 1264, 243-248)
2. Modifikation durch Punktmutationen oder Fragmentaustausch
3. Optimierung der Strukturen an den Grenzflächen der Chimären
4. Optimierung durch random Mutagenese und/oder random Rekombination mit anderen Polymerasegenen (molekulare Evolution).

Beispiele für erfindungsgemäße Polymerasenchimären sind die folgenden:
- *Taq* DNA Polymerase(M1-V307)*E.coli* DNA Polymerase(D355-D501)*Taq* DNA Polymerase(A406-E832)
- *Taq* DNA Polymerase(M1-P291)*E.coli* DNA Polymerase(Y327-K511)*Taq* DNA Polymerase(L416-E832)
- *Taq* DNA Polymerase(M1-P291) *E.coli* DNA Polymerase(Y327-H519)*Taq* DNA Polymerase(E424-E832): Punktmutation A643G; Ile455Val SEQ ID No.: 1
- *Taq* DNA Polymerase(M1-P291)*E*.*coli* DNA Polymerase(Y327-V536)*Taq* DNA Polymerase(L441-E832)
- *Taq* DNA Polymerase(M1-P291)*E*.*coli* DNA Polymerase(Y327-G544)*Taq* DNA Polymerase(V449-E832); SEQ ID No.: 2
- *Taq* DNA Polymerase(M1-P302)*E.coli* DNA Polymerase(K348-S365)*Taq* DNA Polymerase(A319-E347) *E.coli* DNA Poly(N450-T505)*Taq* DNA Polymerase(E410-E4832);
- *Taq* DNA Polymerase(M1-V307)*Tne* DNA Polymerase(D323-D468)*Taq* DNA Polymerase(A406-E832)
- *Taq* DNA Polymerase(M1-P291)*Tne* DNA Polymerase(P295-I478)*Taq* DNA Polymerase(L416-E832)
- *Taq* DNA Polymerase(M1-P291)*Tne* DNA Polymerase(P295-E485)*Taq* DNA Polymerase(E424-E832); stille Mutation A1449C SEQ ID No.: 3
- *Taq* DNA Polymerase(M1-P291*)Tne* DNA Polymerase(P295-V502)*Taq* DNA Polymerase(L441-E832)
- *Taq* DNA Polymerase(M1-P291)*Tne* DNA Polymerase(P295-G510)*Taq* DNA Polymerase(V449-E832); stille Mutation C1767T SEQ ID No.: 4
- *Taq* DNA Polymerase(M1-P302)*Tne* DNA Polymerase(E316-D333)*Taq* DNA Polymerase(A319-E347)*Tne* DNA Polymerase(1381-M394)*Taq* DNA Polymerase(R362-L380)*Tne* DNA Polymerase(E415-T472)*Taq* DNA Polymerase(E410-E832);
- G308D/V310E/L352N/L356D/E401Y/R305D
- *Taq* DNA Polymerase(1-291)*Pfu* DNA Polymerase(V100-R346)*Taq* DNA Polymerase(E424-E832)
- *Taq* DNA Polymerase(1-291)*Pfu* DNA Polymerase(H103-S334)*Taq* DNA Polymerase(E424-E832); SEQ ID No.: 5
- *Taq* DNA Polymerase(1-291)*Pfu* DNA Polymerase(V100-F389)*Taq* DNA Polymerase(-E424-E832)
- *Taq* DNA Polymerase(1-291)*Pfu* DNA Polymerase(V100-F389)*Taq* DNA Polymerase(-V449-E832); SEQ ID No.: 6
- *Taq* DNA Polymerase(1-291)*Pfu* DNA Polymerase(M1-F389)*Taq* DNA Polymerase(-V449-E832)

Von den oben genannten Polymerasenchimären wurden die folgenden näher untersucht:
- *Taq* DNA Polymerase(M1-P291)*E.coli* DNA Polymerase(Y327-H519)*Taq* DNA Polymerase(E424-E832): Punktmutation A643G; Ile455Val (Taq Ec1) SEQ ID No.: 1
- *Taq* DNA Polymerase(M1-P291)*E.coli* DNA Polymerase(Y327-G544)*Taq* DNA Polymerase(V449-E832), (Taq Ec2) SEQ ID No.: 2
- *Taq* DNA Polymerase(M1-P291)*Tne* DNA Polymerase(P295-E485)*Taq* DNA Polymerase(E424-E832), stille Mutation A1449C (Taq Tnel) SEQ ID No.: 3
- *Taq* DNA Polymerase(M1-P291)*Tne* DNA Polymerase(P295-G510)*Taq* DNA Polymerase(V449-E832), stille Mutation C1767T (Taq Tne2) SEQ ID No.: 4
- *Taq* DNA Polymerase(1-291)*Pfu* DNA Polymerase(V100-R346)*Taq* DNA Polymerase(E424-E832), (Taq Pfu1) SEQ ID No.: 5
- *Taq* DNA Polymerase(1-291)*Pfu* DNA Polymerase(V100-F389)*Taq* DNA Polymerase(-V449-E832), (Taq Pfu2) SEQ ID No.: 6

Zur Auswahl geeigneter DNA Polymerasen werden multiple Aminosäuresequenzalignments verfügbarer Sequenzen von DNA Polymerasen und DNA bindenden Proteinen, zum Beispiel mit dem Programm GCG (Devereux et al., 1984, Nucl. Acids Res. 12, 387-395) erstellt. Zur Erstellung eines guten Alignments sind Sekundärstrukturvorhersagen, bekannte strukturbasierte Sequenzalignments, bekannte Motive und funktionell essentielle Aminosäuren sowie phylogenetische Gesichtspunkte zu berücksichtigen. Bestehen die Proteine aus funktionell und strukturell unabhängigen Domänen, ist es sinnvoll, die Aminosäuresequenzalignments zunächst bezogen auf die einzelnen Domänen zu erstellen und erst danach zu einem vollständigen Sequenzalignments zusammenzufügen.

Werden homologe Sequenzen gefunden, deren Tertiärstruktur bekannt ist, so besteht die Möglichkeit, eine 3D-Modellstruktur aus dem homologen Protein abzuleiten. Zur Modellerstellung kann das Programm BRAGI (Reichelt und Schomburg, 1988, J. Mol. Graph. 6, 161-165) verwendet werden. Zur Energieminimierung der Strukturen einzelner Molekülbereiche sowie ganzer Moleküle kann das Programm AMBER (Weiner et al., 1984, J. Am. Chem. Soc. 106, 765-784) und zur Überprüfung der Güte des Modells, das Programm Procheck verwendet werden. Sind nur die Cα-Koordinaten der Struktur des Ausgangsproteins erhältlich, so kann die Struktur, zum Beispiel mit dem Programm O (Jones et al., 1991, Acta Cryst. A47, 110-119), rekonstruiert werden. Des weiteren besteht die Möglichkeit, in der Proteindatenbank unzugängliche, jedoch bereits als Stereobild veröffentlichte, Cα-Koordinaten, zu erhalten, indem das Stereobild eingescannt wird, die Koordinaten gepickt werden (zum Beispiel mit dem Programm Magick) und die z-Koordinaten berechnet werden (zum Beispiel mit dem Programm Stereo). Die Planung von Varianten kann anhand von Aminosäuresequenzalignments, anhand von 3D-Modellen oder anhand von experimentell ermittelten 3D-Strukturen erfolgen.

Des weiteren wurden Chimärenvarianten hergestellt, bei denen die Polymerasenaktivität aufweisende Domäne Reverse Transkriptase Aktivität aufweist. Beispiele für geeignete Polymerasen sind z.B. die Polymerase aus Anaerocellum thermophilum Ath oder Thermus Thermophilum Tth. Die 3'-5'-Exonukleaseaktivität wird durch eine Domäne eingefügt, die aus einer anderen Polymerase stammt, z.B. die Tne-Polymerase oder die Pfu oder Pwo Polymerase. Diese Chimäre kann zusätzlich 5'-3'-Exonukleaseaktivität aufweisen, wobei die 5'-Exonukleaseaktivität aufweisende Domäne sowohl aus der ersten als auch aus der zweiten Polymerase stammen kann.

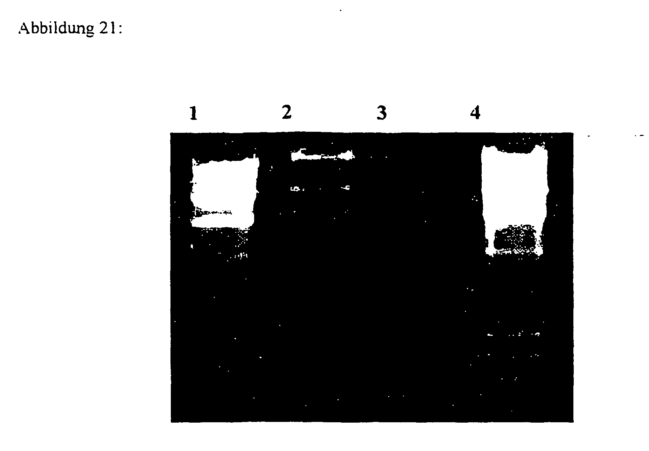

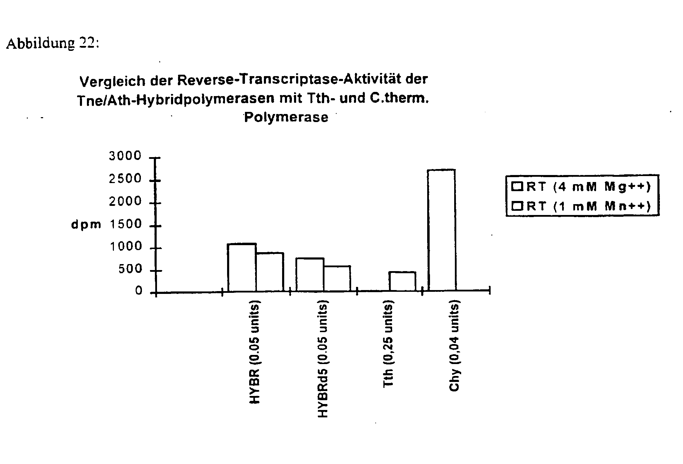

Die rekombinanten Hybridpolymerasen HYB und HYBd5 haben wie die DNA-Polymerase aus Anaerocellum thermophilum eine relativ starke Reverse Transcriptase-Aktivität, sowohl in Gegenwart von Magnesium-, als auch in Gegenwart von Manganionen. Wie in Abbildung 22 ersichtlich, ist das Verhältnis von Polymeraseaktivität zu Reverser Transkriptase-Aktivität günstiger als bei der Tth-Polymerase, dem gebräuchlichsten und bekanntesten Enzym dieser Art. Dieser Befund gilt sowohl für die Magesium-abhängige, als auch für die Mangan-abhängige Reverse Transkriptase-Aktivität. Daraus kann geschlossen werden, daß die Polymerasedomäne, die von der Anaerocellum-Polymerase stammt, auch im Hybridenzym volle Aktivität zeigt. Die Variante HYBd5 zeigt außerdem 3'-5'Exonuclease-Aktivität wie in Abbildung 21 ersichtlich. Diese wird durch die Anwesenheit von Deoxynucleosidtriphosphaten gehemmt, wie es für die typische "Proofreading Aktivität" zu erwarten ist. Die Exonuclease-Domaine, die von der DNA-Polymerase aus Thermotoge neapolitana stammt, ist also im Hybridmolekül ebenfalls aktiv. Die Hemmbarkeit der Exonuclease-Aktivität zeigt ferner, daß beide Domainen des Hybridpolymerasemoleküls interagieren und somit die Hybridpolymerase den natürlichen Enzymen funktionell sehr ähnlich ist.

Die gentechnische Herstellung von Domänenaustauschvarianten kann durch PCR-Mutagenese, nach der SOE-Methode (Horton et al. (1989) Gene 77, 61-68) oder nach der modifizierten Methode (siehe Schema unter Beispiele) mit Hilfe chemisch synthetisierter Oligodesoxynukleotide erfolgen. Die jeweiligen DNA-Fragmente werden auf einem Agarosegel aufgetrennt, isoliert und in den Ausgangsvektor ligiert. Als Ausgangsvektoren können für *E.coli* pUC Derivate mit geeigneten Promotoren verwendet werden wie pTE, pTaq, pPL, Bluescript. Die Plasmid-DNA wird in einen *E. coli*-Stamm transformiert, zum Beispiel XL1-Blue, einige Klone werden gepickt und deren Plasmid-DNA isoliert. Möglich ist aber auch die Verwendung anderer Stämme wie z.B. Nova Blue, BL21(DE), MC1000 etc. Selbstverständlich ist es auch möglich, in anderen Organismen zu klonieren wie in Hefe-, Pflanzen-, Säugerzellen. Durch Restriktionsanalyse wird eine Vorauswahl an Klonen getroffen, deren Plasmid-DNA im modifizierten Bereich sequenziert wird.

Die Genexpression der Zielproteine kann bei vielen Plasmiden, zum Beispiel Pbtaq, durch IPTG induziert werden. Bei der Herstellung vieler verschiedener Varianten ist es sinnvoll, ein universelles Aufreinigungsverfahren zu etablieren. Hierfür ist die Affinitätschromatographie an Ni-NTA (nickel-nitrilotriacetic acid) Agarose gut geeignet, die nach dem Anfügen eines His-Tags an das Protein, zum Beispiel durch PCR, verwendet werden kann. Die Proteinkonzentrationen können mit dem Protein Assay ESL (Boehringer Mannheim) bestimmt werden und kontaminierende Nebenaktivitäten der Präparationen wie für die kommerziell erhältliche *Taq* Polymerase (Boehringer Mannheim) beschriebenen. Zur weiteren Charakterisierung der Varianten werden Polymerase-, Exonukleaseaktivitäts- und Thermostabilitätstests durchgeführt, sowie das jeweilige Temperaturoptimum bestimmt. Die Polymeraseaktivitäten der Chimären können in nicht-radioaktiven Testsystemen, zum Beispiel durch Bestimmung der Einbaurate von Dig-dUTP in DN'ase aktivierte Kalbsthymus-DNA, oder in radioaktiven Testsystemen, zum Beispiel durch Bestimmung der Einbaurate von α-[³²P]dCTP in M13 mp9 ss-DNA, ermittelt werden. Zur Bestimmung der Temperaturoptima der Polymeraseaktivität der Chimären wird die Polymerasereaktion bei unterschiedlichen Temperaturen durchgeführt und es werden die Spezifischen Aktivitäten berechnet. Zur Bestimmung der Thermostabilitäten werden die Restaktivitäten, d.h. Prozent der Ausgangsaktivität ohne Hitzebehandlung, nach Hitzebehandlung ermittelt. Die 3'-5' Exonukleaseaktivität kann durch den Abbau eines 5'-Dig-markierten Primers, der an einen DNA-Matrizenstrang annealt, von seinem 3'-Ende her, gezeigt werden. Die Korrektur von 3'-mismatched Primern und deren Verlängerung (proof-reading) kann gezeigt werden, indem falschgepaarte (mismatched) 5'-Dig-markierte Primer, die in der Erkennungssequenz eines Restriktionsenzymes (z.B. Eco RI) an einen Matrizenstrang annealen, verlängert werden. Nur bei einer Korrektur der Fehlpaarung durch das Enzym, ist eine Spaltung mit dem Restriktionsenzym möglich. Die Prozessivität kann durch Einsatz der Varianten in der PCR untersucht werden. Ist das Enzym nicht thermostabil genug für den Einsatz in der PCR, so kann eine PCR beim Temperaturoptimum als Verlängerungstemperatur unter sukzessiver Enzymzugabe durchgeführt werden. Die Exonucleaseaktivität der Chimären kann in einem radioaktiven Testsystem bestimmt werden. Dazu wurde ein bestimmte Menge der Chimären-Polymerasen (i.d. Regel 2.5 U) bei unterschiedlichen Temperaturen für 4 Stunden mit markierter DNA (5 µg [³H] DNA in den jeweiligen Testpuffern) inkubiert. Gegebenenfalls wurden dNTPs in unterschiedlichen Konzentrationen zugesetzt (0 - 0.2 mM). Nach Abstoppen der Reaktion wird die Freisetzung an radioaktiv markierten Nukleotiden bestimmt.

Gegenstand der vorliegenden Erfindung ist desweiteren die DNA-Sequenz der oben beschriebenen Polymerasenchimären. Insbesondere sind Gegenstand der vorliegenden Erfindung die DNA-Sequenzen der SEQ.ID.No.: 2,4 und 6. Gegenstand der vorliegenden Erfindung sind desweiteren die Aminosäuresequenzen der oben beschriebenen Polymerase-Chimäre. Insbesondere sind Gegenstand der vorliegenden Erfindung die Aminosäuresequenzen der SEQ.ID.No: 8, 10 und 12.

Ein weiterer Gegenstand der vorliegenden Erfindung sind die Vektoren, die die obengenannten DNA Sequenzen enthalten. Ein bevorzugter Vektor ist pBTaq (Plasmid Pbtaq4_oligo 67 (Villbrandt (1995), Dissertation, TU Braunschweig)). Ein weiterer Gegenstand der Erfindung sind die E. coli Stämme, insbesondere der Stamm *Escherichia coli* XL1-Blue, die den Vektor, der das Polymerase-Chimäre-Gen trägt, enthalten. Folgende Stämme wurden bei der DSM hinterlegt, Deutsche Sammlung von Mikroorganismen und Zellkulturen GmbH, Mascheroder Weg 1b, D-38124 Braunschweig:
- *E.coli* XL1 Blue x pBTaqEc1 : TaqEc1 DSM No. 12053
- *E.coli* XL1 Blue x pBTaqTnel: TaqTne1 DSM No. 12050
- *E.coli* XL1 Blue x pBTaqTne2: TaqTne2 DSM No. 12051
- *E.coli* XL1 Blue x pBTaqPful: TaqPful DSM No. 12052

Die erfindungsgemäßen Polymerasenchimären eignen sich insbesondere für die Amplifikation von DNA-Fragmenten, z.B. für die Polymerase-Ketten-Reaktion. Eine weitere Anwendung ist beispielsweise die Sequenzierung von DNA-Fragmenten.

Ein bevorzugter Vektor für die Ath-Tne-Chimäre ist der folgende:
E.coli BL 21(DE3) plysS x pETHYBR : HYBR
E.coli BL 21 (DE3) plysS x pETHYBR d5 : HYBR d5

Ein weiterer Gegenstand der Erfindung sind die E.coli Stämme, die den Vektor, der das Polymerase-Chimäre-Gen trägt, enthalten. Folgende Stämme wurden bei der DSM hinterlegt, Deutsche Sammlung von Mikroorganismen und Zellkulturen GmbH, Mascheroder Weg 1b, D-38129 Braunschweig: HYBR (DSM No. 12720); HYBR d5 (DSM No. 12719).

Die Herstellung der oben genannten Ath-Tne-Chimäre ist beispielsweise in den Beispielen 8-11 beschrieben. Die erfindungsgemäßen Chimären die RT-Aktivität aufweisen, eignen sich insbesondere für Reverse Transkription von RNA.

Ein weiterer Gegenstand der vorliegenden Erfindung ist ein Kit zur Amplifikation von DNA-Fragmenten, der mindestens eine der erfindungsgemäße Polymerasenchimäre enthält.

### Kurze Beschreibung der Abbildungen

### Abbildung 1:

DNA-Sequenz der *Taq* DNA Polymerase(M1-P291*)E.coli* DNA Polymerase(Y327-H519)*Taq* DNA Polymerase(E424-E832): Punktmutation A643G; Ile455Val SEQ ID No.: 1; sowie die entsprechende Aminosäuresequenz SEQ ID No.: 7.

### Abbildung 2:

DNA-Sequenz der *Taq* DNA Polymerase(M1-P291)*E.coli* DNA Polymerase(Y327-G544)*Taq* DNA Polymerase(V449-E832); SEQ ID No.: 2; sowie die entsprechende Aminosäuresequenz SEQ ID No.: 8.

### Abbildung 3:

DNA-Sequenz der *Taq* DNA Polymerase(M1-P291)*Tne* DNA Polymerase(P295-E485)*Taq* DNA Polymerase(E424-E832); stille Mutation A1449C SEQ ID No.: 3; sowie die entsprechende Aminosäuresequenz SEQ ID No.: 9.

### Abbildung 4:

DNA-Sequenz der *Taq* DNA Polymerase(M1-P291)*Tne* DNA Polymerase(P295-G510)*Taq* DNA Polymerase(V449-E832); stille Mutation C1767T SEQ ID No.: 4; sowie die entsprechende Aminosäuresequenz SEQ ID No.: 10.

### Abbildung 5:

DNA-Sequenz der *Taq* DNA Polymerase(1-291)*Pfu* DNA Polymerase(H103-S334)*Taq* DNA Polymerase(E424-E832); SEQ ID No.: 5; sowie die entsprechende Aminosäuresequenz SEQ ID No.: 11.

### Abbildung 6:

DNA-Sequenz der *Taq* DNA Polymerase(1-291)Pfu DNA Polymerase(V100-F389)*Taq* DNA Polymerase(-V449-E832); SEQ ID No.: 6; sowie die entsprechende Aminosäuresequenz SEQ ID No.: 12.

### Abbildung 7:

Aufreinigung der Domänenaustauschvariante TaqEc1 an Ni- NTA-Agarose Analyse auf einem mit Coomassieblau angefärbten 8%igen Polyacrylamidgel.

| | |
|---|---|
| Bahnen 1, 8 | Proteinmolekulargewichtsmarker Broad Range |
| | (200 kDa, 116,25 kDa, 97,4 kDa, 66,2 kDa, 45 kDa, 31 kDa) |
| Bahn 2 | lösliche Proteine |
| Bahn 3 | Säulendurchlauf |
| Bahn 4 | Waschfraktion Puffer B |
| Bahn 5 | Waschfraktion Puffer A |
| Bahnen 6, 7 | Eluatfraktionen Puffer C |
| Proteinausbeute (OD₂₈₀) etwa 7 mg | |

### Abbildung 8:

Bestimmung der Proteinreinheit: SDS-PAGE, Phast-System (10-15%): Silberfärbung MW: Proteinmolekulargewichtsmarker; NHis-TaqPol: Taq DNA Polymerase mit N-terminalem His-Tag; TaqEc1, TaqTne1, TaqTne2: Domänenaustauschvarianten.

### Abbildung 9:

Spezifische Aktivitäten der Domänenaustauschvarianten bei verschiedenen Temperaturen.

### Abbildung 10:

Test der Domänenaustauschvarianten in der PCR unter sukzessiver Enzymzugabe, Extension bei 72°C.
Lambda-DNA (links): Größe der Zielsequenz = 500 bp
Plasmid pa (rechts): Größe der Zielsequenz = 250 bp
Bahn 1: *Taq* DNA Polymerase (Fa. BM), 100 ng, 5 Units
Bahn 2: Domänenaustauschvariante TaqEc1, 500 ng, 1,25 Units/Zyklus
Bahn 3: Domänenaustauschvariante TaqTne1, 50 ng, 3,6 Units/Zyklus
Bahn 4: Domänenaustauschvariante TaqTne2, 50 ng, 3,5 Units/Zyklus
III: DNA-Längenstandard III (Fa. BM)
VI: DNA-Längenstandard VI (Fa. BM)

Ergebnis: Beim Einsatz der Domänenaustauschvariante TaqTne2 entstanden PCR-Produkte der richtigen Größe.

### Abbildung 11:

Test der Domänenaustauschvarianten in der PCR unter sukzessiver Enzymzugabe, Extension bei 55°C.
Lambda-DNA (links): Größe der Zielsequenz = 500 bp
Plasmid pa (rechts): Größe der Zielsequenz = 250 bp
Bahn 1: Domänenaustauschvariante TaqEc1, 500 ng, 6 Units/Zyklus
Bahn 2: Domänenaustauschvariante TaqTne1, 50 ng, 7,5 Units/Zyklus
III: DNA-Längenstandard III (Fa. BM)
VI: DNA-Längenstandard VI (Fa. BM)

Ergebnis: Beim Einsatz der Domänenaustauschvariante TaqEc1 entstanden PCR-Produkte der richtigen Größe.

### Abbildung 12:

3'-5' Exonuklease-Test - Variante TaqEc1, Inkubation bei 72°C, Primer P1.

### Abbildung 13:

3'-5' Exonuklease-Test - Variante TaqEc1, Inkubation bei 50°C, Primer P1 (links), Primer P2 (rechts).

### Abbildung 14:

Korrektur von 3'-mismatched Primern und deren Verlängerung - Variante TaqEc1 (3'-mismatch primer correction assay)
(-) : ohne Restriktionsenzymverdau
(+) : Restriktionsenzymverdau mit Eco RI.

### Abbildung 15:

### Schematische Darstellung

Abbau von Primern am 3'-Ende (3'-5' exonuclease assay) und Korrektur von 3'-mismatched Primern und deren Verlängerung (3'-mismatch primer correction assay).

### Abbildung 16:

Schematische Darstellung: Vereinfachtes Ablaufschema, Abbau von Primern am 3'-Ende und Korrektur von 3'-mismatched Primern und Verlängerung

### Abbildung 17:

CLUSTAL W(1.5) Multiple Sequenz Alignment der Ath, Tne, PoII-Polymerase Gene sowie des vorhergesagten Gens der Polymerasenchimäre. Der Teil der Chimären-Sequenz der aus Tne stammt, ist unterstrichen.

### Abbildung 18:

A. Struktur der Primer, die für die PCR-Amplifikation der Tne-Exo und der Ath-Polymerase-Domänen verwendet wurden.
B. Teil des Aminosäuresequenz-Alignments der zwei Polymerasen, der den gewählten "Kreuzungspunkt" zeigt.
C. Nukleotidsequenz und Position der Primer, die für die Konstruktion des Hybridpolymerase-Gens designed wurden. Die Sequenzen der Primer, die nicht komplementär sind zur Zielsequenz, wurden in kleinen Buchstaben dargestellt. Komplementäre "überlappende" Sequenzen in den TNELOW und ATHUP Primern wurden doppelt unterstrichen.

### Abbildung 19:

A. Teil des Alignments der Ath und Tne Aminosäuresequenzen, die die homologe Region zeigt, die für das Zusammenspleißen der Domänen der zwei Polymerasen verwendet wurde.
B. Nukleotid- und Aminosäuresequenz der zwei Polymerasen in der Splicing Region. Gezeigt ist die einzige BamHI Schnittstelle in der Tne DNA Sequenz und die Sequenz der beiden Oligos, die konstruiert wurden, um die BamHI Schnittstelle in die Ath-Polymerase einzuführen.

### Abbildung 20:

Konstruktion des Gens der Polymerasenchimäre (s. auch Beispiel 8)

### Abbildung 21:

3'-5'-Exonuklease-Aktivität der rekombinanten DNA-Polymerase
1- DNA des Lambda-Phagen, hydrolysiert durch HindIII
2- DNA des Lambda-Phagen, hydrolysiert durch HindIII, und dNTP, und rekombinante DNA-Polymerase
3- DNA des Lambda-Phagen, hydrolysiert durch HindIII, ohne dNTP, mit rekombinanter DNA-Polymerase
4- DNA des Lambda-Phagen, hydrolysiert durch HindIII.

### Abbildung22:

### Reverse Transcriptase-Aktivität der recombinanten Polymerasen HYB und HYBd5.

Die DNA-Polymerase-Aktivität von je 2 µl Extract aus E.coli BL21 (DE3) plysS x pETHYBr und E.coli BL21(DE3) plysS x pETHYBRd5 wurden zu je 0,05 units ermittelt. Diese Mengen wurden verwendet um die Reverse Transcriptase-Akivität der Hybridpolymerasen zu bestimmen in Abhängigkeit von 1 mM Mangan- bzw 4 mM Magnesiumionen. Als Kontrolle wurde Tth (0,25 units) als Mangan-abhängige Reverse Transcriptase und C.therm. Polymerase (Roche Molecular Biochemicals) als Magnesiumabhängige Reverse Transcriptase verwedet.

### Beispiel 1: Konstruktion und Klonierung

### Etablierung eines universellen Aufreinigungsverfahrens

Zur Vereinheitlichung des Aufreinigungsprotokolls der Domänenaustauschvarianten wurde die Affinitätschromatographie an Ni-NTA (nickel-nitrilotriacetic acid) Agarose verwendet. Dazu war es notwendig, vor der Herstellung der Proteinvarianten, ein His-Tag an die/in die *Taq* DNA Polymerase an-/einzufügen. Geplant und hergestellt wurden zwei verschiedene His-Tag-Varianten im Plasmid Pbtaq4_oligo67 (Boehringer Mannheim). Die Variante NHis-TaqPol enthält ein N-terminales His-Tag, eine Enterokinasespaltstelle, um das His-Tag gegebenenfalls abzuspalten und ein Epitop zum Nachweis der His-Tag-Proteine mit Antikörpern (Quiagen). Sie wurde durch PCR von der EcoRI-Site bis zur PstI-Site hergestellt. Bei der N-terminalen Proteinsequenzierung konnten von der Variante NHis-TaqPol die zwanzig N-terminalen Aminosäuren als richtig bestätigt werden.
Sequenz: NHis-TaqPol

Die Variante 5DHis-TaqPol enthält ein His-Tag in einem flexiblen Loop der 5' Nukleasedomäne, zwischen Glycin 79 und Glycin 80 der *Taq* DNA Polymerase und wurde durch PCR-Mutagenese von der EcoRI-Site bis zur Pstl-Site hergestellt.
Sequenz: 5DHis-TaqPol
SEQ ID No.: 15
SEQ ID No.: 16

Die Korrektheit der Plasmid-DNA im jeweils modifizierten Bereich der beiden neuen Gene wurde durch DNA-Sequenzierung bestätigt. Beide modifizierten Gene wurden unter gleichen Bedingungen und in gleicher Höhe wie das Ausgangsprotein ohne His-Tag exprimiert, konnten gut über Ni-NTA-Agarose aufgereinigt werden und verhielten sich in der Standard-PCR wie die *Taq* Polymerase ohne His-Tag. Für die Aufreinigung der Domänenaustauschvarianten wurde das N-terminale His-Tag verwendet

### Aminosäuresequenzalignments

Zur Planung der Domänenaustauschvarianten wurden folgende Aminosäuresequenzalignments erstellt:
1. *Tne-, E.coli I- und Taq DNA Polymerase*
*2. Pfu-, E.coli I- und Taq DNA Polymerase*
3. Multiple Aminosäuresequenzalignments von DNA Polymerasen

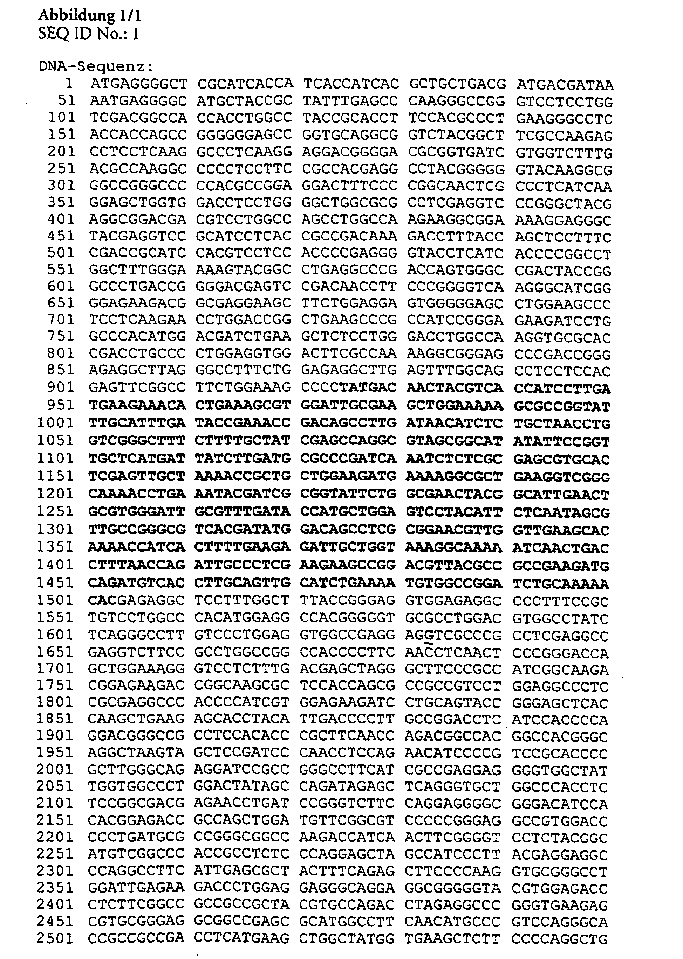

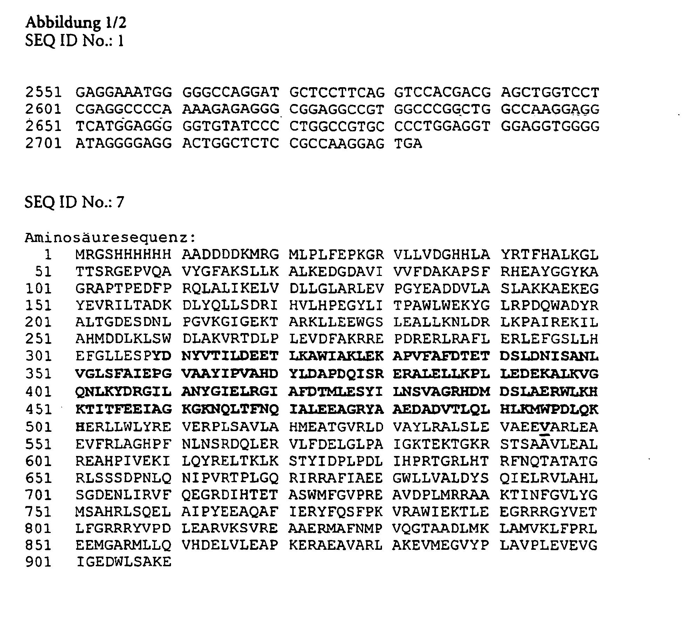

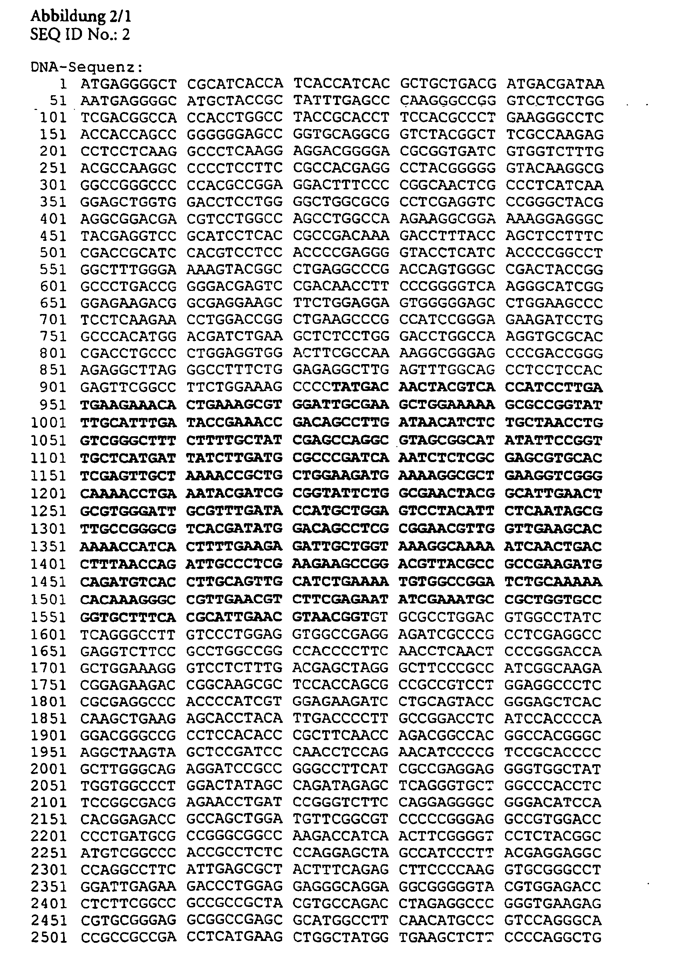

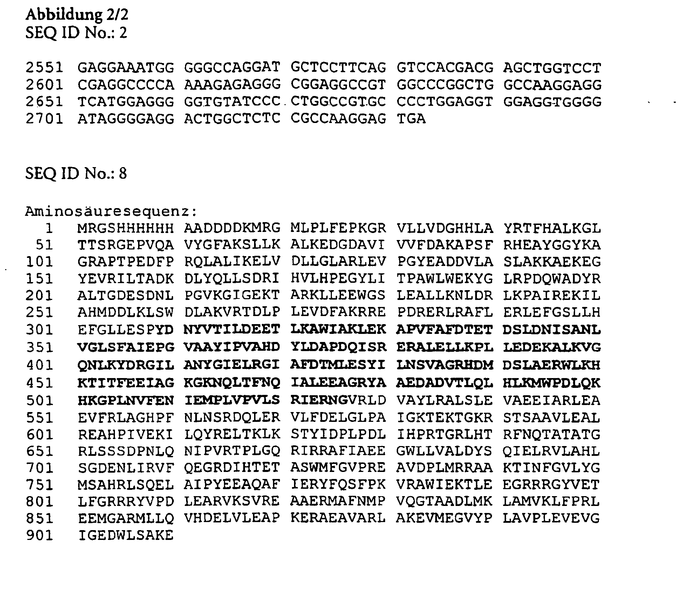

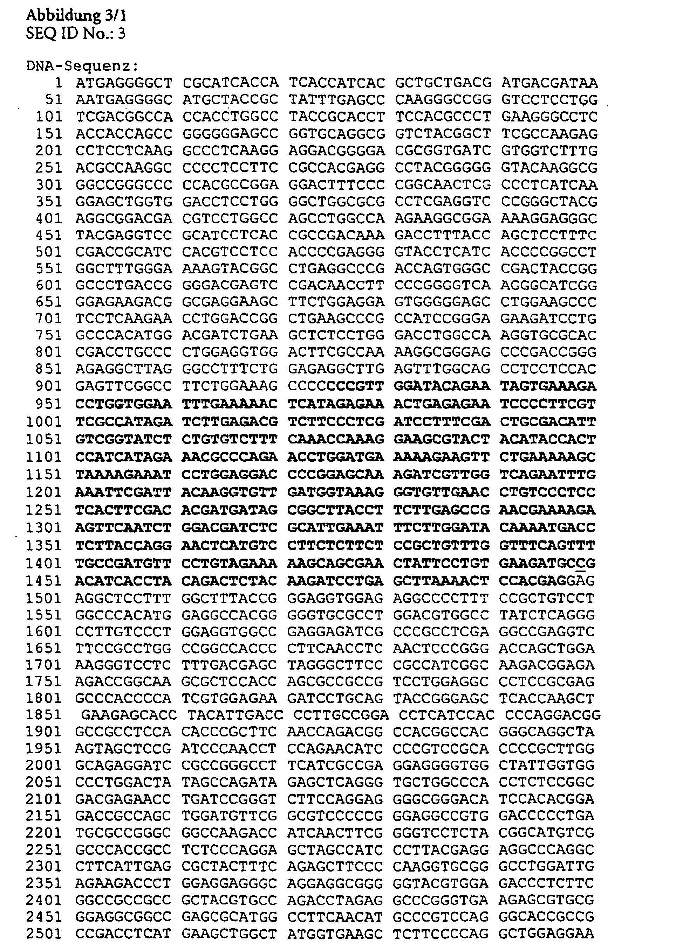

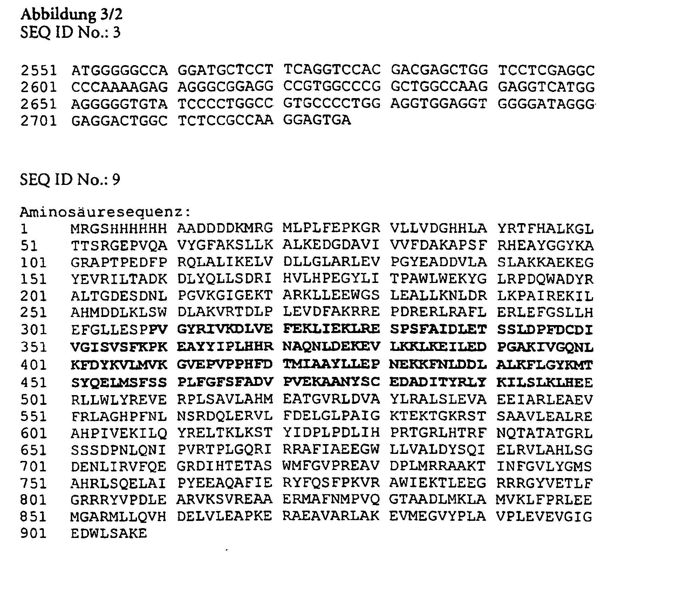

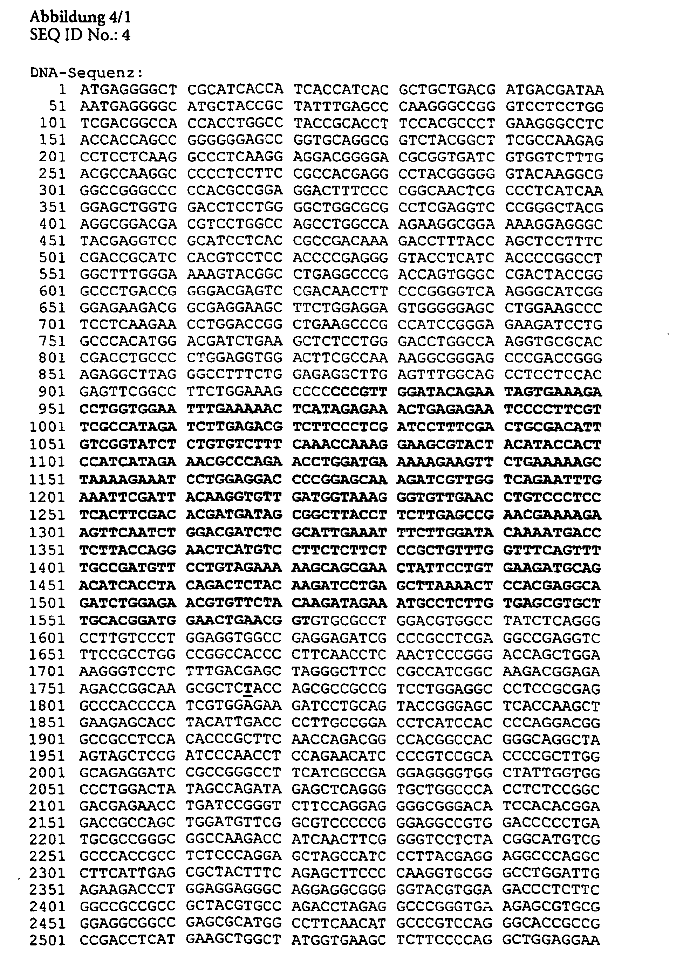

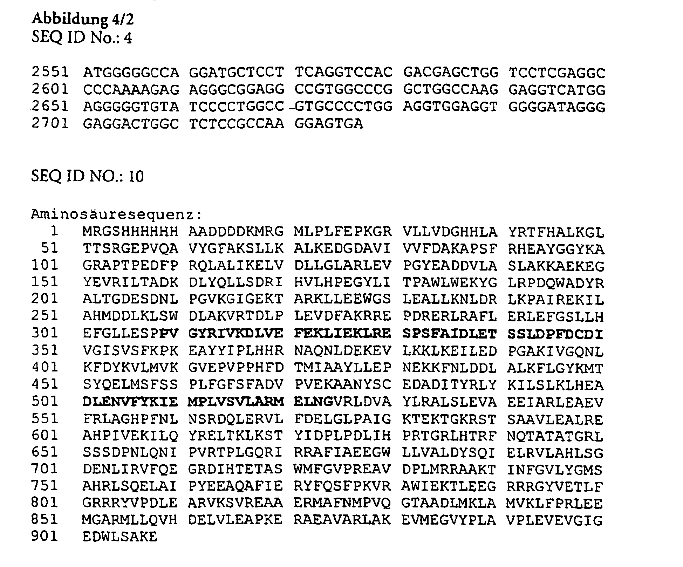

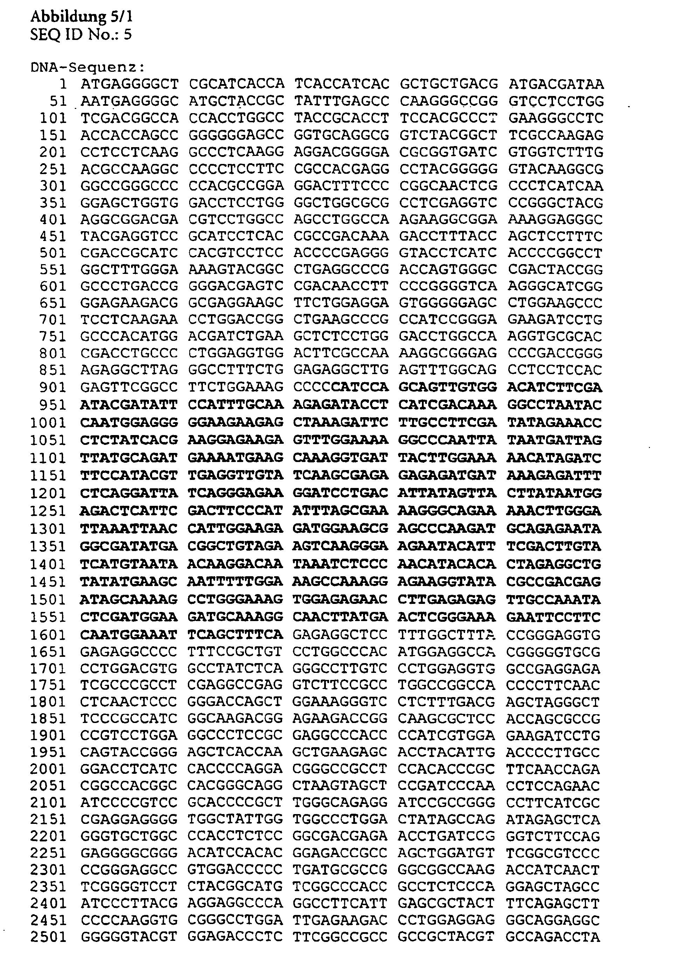

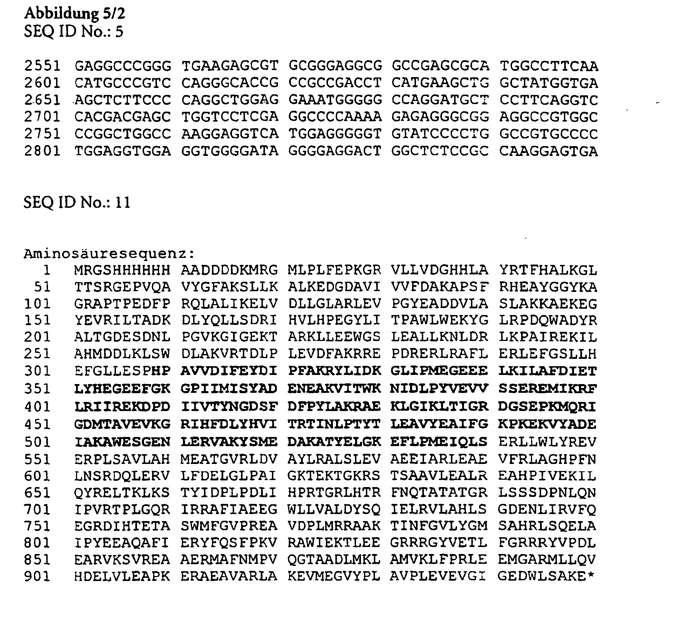

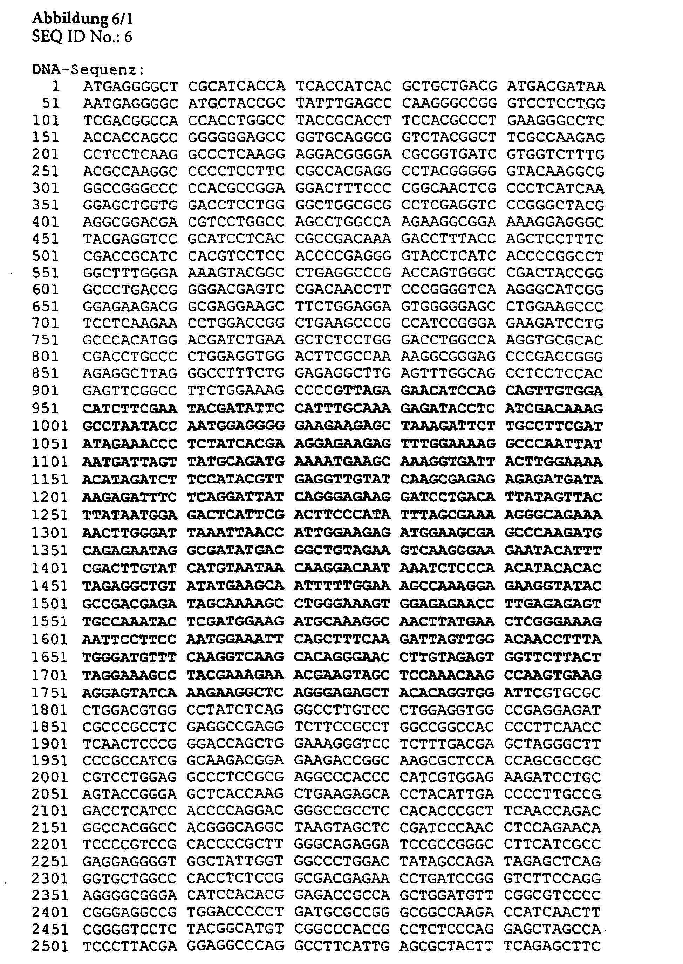

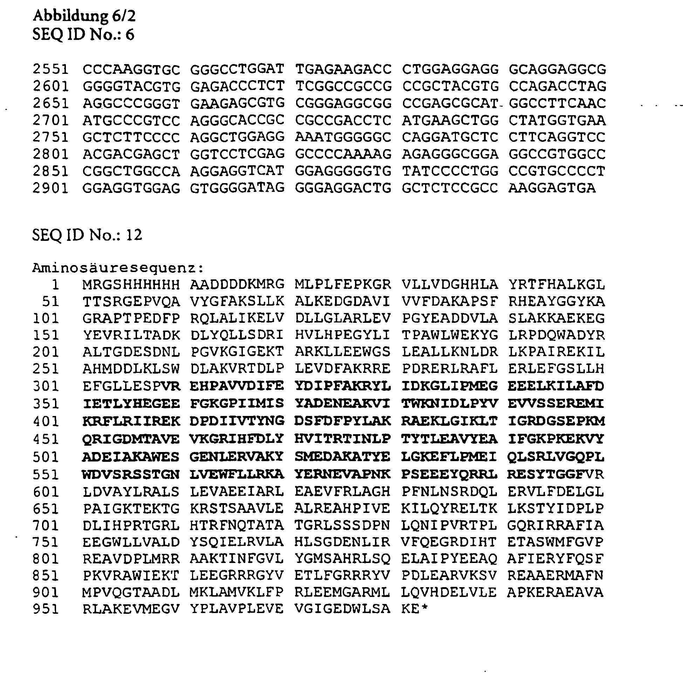

Die Alignments wurden bezogen auf einzelne Molekülbereiche (Domänen) mit dem Programm GCG erstellt und unter Berücksichtigung bekannter Sekundärstrukturen, Motive und essentieller Aminosäuren und unter Verwendung des strukturbasierten Sequenzalignments der Sequenzen der 3'-5' Exonukleasedömane des Klenow Fragmentes mit der entsprechenden Domäne der *Taq* DNA Polymerase (Abbildung 2d in Kim et al. (1995) Nature 376, 612-616) zu dem vollständigen Sequenzalignments zusammengefügt.

Zur Auswahl der Ausgangsstruktur des Klenow Fragmentes für das Homologiemodelling wurden die damals zugänglichen Strukturen der *E. coli* DNA Polymerase I mit dem Programm Bragi im RMS-Fit verglichen:
Klenow Fragment - dCMP Komplex (POB-code:1dpi), 2,8 Å(1987), Klenow Fragment - dCTP-Komplex (PDB-code:1kfd) 3,9 Å (1993) und Klenow Fragment, D355A - DNA-Komplex (PDB-code:1kln) 3,2 Å (1994).
Ausgewählt wurde die Struktur Klenow Fragment (PDB-code:1kln). In den zwei Bereichen, in den Koordinaten fehlten, wurden zwei Loops eingebaut (Programm Bragi) und energieminimiert (Programm Amber). Die Güte der Proteinstruktur wurde überprüft (Programm Procheck).

### Erstellung von 3D-Modellen

Für dem Molekülbereich der *Taq* DNA Polymerase, der die Aminosäuren 292-832 umfaßt, wurde ein 3D-Modell in Homologie zur Struktur des Klenow Fragmentes (PDB-code:1kln) mit dem Programm Bragi erstellt. Die Modellierung umfaßte Aminosäureaustausche, Einführung von Insertionen und Deletionen, Energieminimierung der neuen Loopbereiche und Energieminimierung des gesamten Moleküls (Programm Amber).

Die Struktur der *Taq* DNA Polymerase war zum Zeitpunkt der Modellierungarbeiten schon veröffentlicht, jedoch noch nicht in der Proteindatenbank zugänglich. Zur Erstellung eines Modells der Zwischendomäne der *Taq* DNA Polymerase, die der 3'-5' Exonukleasedomäne des Klenow Fragmentes entspricht (Aminosäuren 292-423) wurde ein Stereobild (Abbildung 2c in Kim et al. (1995) Nature 376, 612-616) eingescanned, die Cα-Koordinaten am Bildschirm (jeweils x- und y-Koordinaten für das linke und rechte Bild) gepickt (Programm Magick, (John Cristy, E. I. du Pont De Nemours and Company Incorporated)), die z-Koordinaten berechnet (Programm Stereo, (Collaborative Computational Project, Number 4 (1994) Acta Cryst. D50, 760-763)), die Proteinhauptkette unter Erzeugung eines poly-Alanins rekonstruiert (Programm O), Aminosäureaustausche durchgeführt (Programm Bragi) und eine Energieminimierung des gesamten Moleküls durchgeführt (Programm Amber). Das Modell der Aminosäurereste 292-423 (s.o.) wurde an das Modell der Polymerasedömane (Aminosäuren 424-832) (s.o.) unter Berücksichtigung der Strukturalignments der *Taq* DNA Polymerase mit dem Klenow Fragment (Abbildung 2b und 2c in Kim et al. (1995) Nature 376, 612-616) angefügt. Die gesamte Modellstruktur wurde energieminimiert (Programm Amber) und die Güte der Modellstruktur überprüft (Programm Procheck, (Laskowski, R., A., et al. (1993) J. Appl. Cryst. 26, 283-291)).

Von der *Tne* DNA Polymerase (Reste 297-893) wurde ein 3D-Modell in Homologie zur Struktur des Klenow Fragmentes (PDB-code:1kln) erstellt. Die Modellierung umfaßte Aminosäureaustausche, Einführung von Insertionen und Deletionen (Programm Bragi), Energieminimierung der neuen Loopbereiche, Energieminimierung des gesamten Moleküls (Programm Amber) und Überprüfung der Güte der Modellstruktur (Programm Procheck).

Es wurden 20 Proteinvarianten geplant.
Bei Verwendung der *E. coli* poll und der *Tne* Polymerase anhand der erstellten 3D-Strukturmodelle, bei Verwendung der *Pfu* Polymerase anhand der erstellten Aminosäurealignments.

Gentechnische Herstellung der Domänenaustauschvarianten
Das N-terminale His-Tag wurde durch PCR eingefügt und die Domänenaustauschvarianten wurden nach der modifizierten SOE-Methode (Horton et al. (1989) Gene 77, 61-68), dargestellt im Schema, mit Hilfe chemisch synthetisierter Oligodesoxynukleotide hergestellt. Die jeweiligen DNA-Fragmente wurden auf einem Agarosegel aufgetrennt, mit dem QIAquick Gel Extraction Kit (Firma Qiagen) nach dem mitgelieferten Protokoll isoliert und bei den PCR-Reaktionen I bis IV in der nachfolgenden PCR-Reaktion eingesetzt oder bei der PCR-Reaktion V mit den beiden Restriktionsenzymen, deren Erkennungssequenz sich in den flankierenden Primern befanden (Eco RI und Pst I) nachgeschnitten. Die Ligation von DNA-Fragmenten und die Herstellung und Transformation von kompetenten XL1-Blue *E. coli*-Zellen durch Elektroporation erfolgte wie von Villbrandt (1995, Dissertation, TU Braunschweig) beschrieben. Es wurden einige Klone gepickt und deren Plasmid-DNA mit dem QIAprep Spin Plasmid Kit (Firma Qiagen) nach dem mitgelieferten Protokoll isoliert. Mikrobiologische Arbeitstechniken und die Rezepturen zur Herstellung von Flüssig- bzw. Plattenmedien sowie das Anlegen von Glyzerinkulturen wurden, wie im Handbuch von Sambrook et al. (1989, Molecular cloning - a laboratory manual, Cold Spring Harbor Laboratory, Cold Spring Harbor, New York) beschrieben, durchgeführt. Die Domänenaustauschvarianten konnten in gleicher Höhe wie das Ausgangsprotein exprimiert werden.

### Beispiel 2: Aufreinigung (für eine Chimäre)

### Aufreinigung der Domänenaustauschvarianten

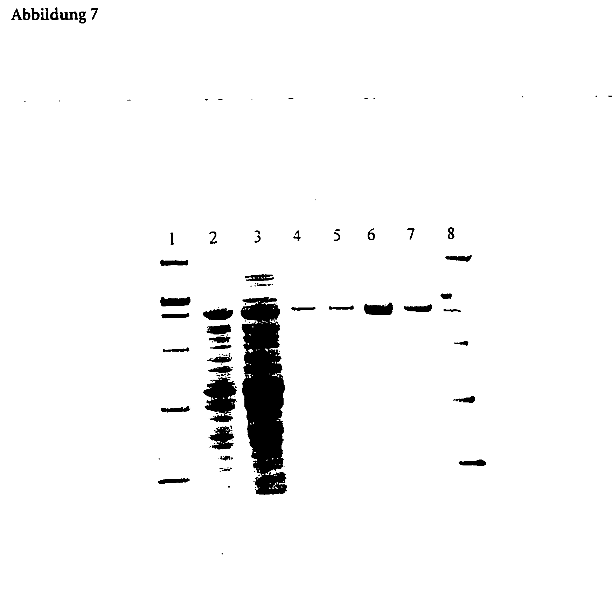

Alle Domänenaustauschvarianten wurden nach dem gleichen Protokoll aus *Escherichiacoli* XL1-Blue isoliert. Die Fermentation erfolgte im 1-Liter-Maßstab in LB-Medium/100 mg/ml Ampicillin/12,5 mg/ml Tetracyclin/1 mM IPTG bei 37°C für 16 Stunden. Die Zellen wurden abzentrifugiert, in 20 ml Lysispuffer (50 mM Tris-HCL, pH 8,5, 10 mM 2-Mercaptoethanol, 1 mM PMSF) aufgenommen, bei -70°C für mindestens 16 Stunden eingefroren und 10 Minuten mit Ultraschall behandelt. Die Zelltrümmer wurden abzentrifugiert und der sterilfiltrierte Überstand auf eine Ni-NTA (nickel-nitrilotriacetic acid)-Agarose-Säule (Qiagen) mit einem Säulenvolumen von 3,5 ml (r = 0,65 cm, h = 2,7 cm) aufgetragen. Es wurde mit 40 ml Puffer A (20 mM Tris-HCL, pH 8,5, 100 mM KCl, 20 mM Imidazol, 10 mM 2-Mercaptoethanol, 10% (v/v) Glyzerin), anschließend mit 10 ml Puffer B (20 mM Tris-HCL, pH 8,5, 1 M KCl, 20 mM Imidazol, 10 mM 2-Mercaptoethanol, 10% (v/v) Glyzerin) und nochmals mit 10 ml Puffer A gewaschen. Die Elution erfolgte mit 15 ml Puffer C (20 mM Tris-HCL, pH 8,5, 100 mM KCl, 100 mM Imidazol, 10 mM 2-Mercaptoethanol, 10% (v/v) Glyzerin). Die Flußrate betrug 0,5 ml/Minute und die Fraktionsgröße 10 ml bei den Waschfraktionen und 1 ml bei den Elutionfraktionen. Die vereinigten Fraktionen wurden gegen Lagerpuffer (20 mM Tris-HCl pH 8.0, 100 mM KCl, 0.1 mM EDTA, 1 mM DTT, 0.5% Tween 20, 50% Glyzerin) dialysiert und 200 µg/ml Gelatine sowie Nonidet P40 in einer Endkonzentration von 0,5% zugesetzt. Die Proteinlösungen wurden bei -20°C aufbewahrt.
Die Analyse der Aufreinigung der Domänenaustauschvariante TaqEc1 an Ni- NTA-Agarose wird in Abb. 7 gezeigt.

### Bestimmung der Proteinkonzentration

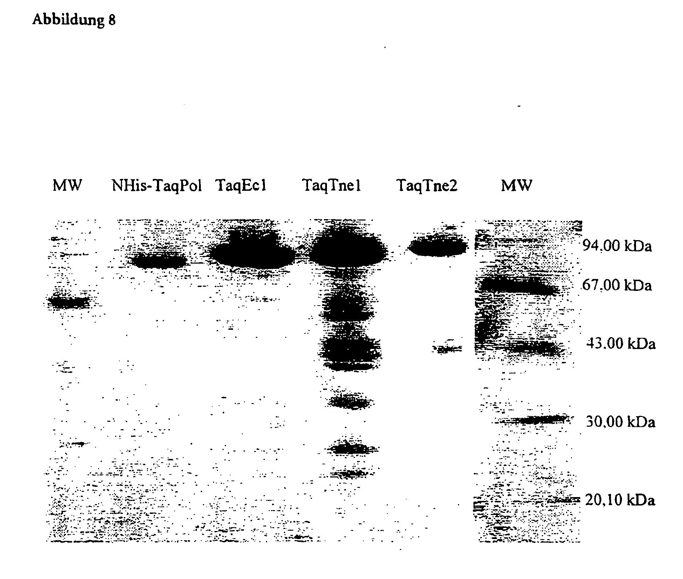

Die Proteinkonzentrationen wurden durch Messung der OD₂₈₀ und mit dem Protein Assay ESL (Boehringer Mannheim) bestimmt. Abb. 8 zeigt die Bestimmung der Proteinreinheit: SDS-PAGE, Phast-System (10-15%): Silberfärbung.

### Beispiel 3: Temperaturoptimum der Polymeraseaktivität der Chimären

Die Polymeraseaktivitäten der Chimären wurden in einem nicht-radioaktiven Testsystem bestimmt. Zum Abgleich der Werte wurde ein radioaktives Testsystem verwendet. Beim nicht-radioaktiven Testsystem wurde die Einbaurate von Dig-dUTP in DN'ase aktivierte Kalbsthymus-DNA bestimmt. Ein 50 µl Testmix enthielt 5 µl Puffermix (500 mM Tris-HCl, 150 mM (NH₄)₂SO₄, 100 mM KCL, 70 mM MgCl₂, 100 mM 2-Mercaptoethanol, pH 8,5), je 100 µM dATP, dCTP, dGTP, dTTP, 36 nM Dig-dUTP (Boehringer Mannheim), 12 µg Kalbsthymus-DNA (DN'ase aktiviert), 10 µg Rinderserumalbumin und 2 µl chimäres Enzym, oder 0,02 Units *Taq* Polymerase (Boehringer Mannheim) als Referenz in Verdünnungspuffer (20 mM Tris-HCl, pH 8,0, 100 mM KCl, 0,1 mM EDTA, 1 mM DTT, 200 µg/ml Gelatine, 0,5% Tween 20, 0,5% Nonidet P40, 50% Glyzerin). Die Inkubation der Reaktionsansätze erfolgte für 30 Minuten bei verschiedenen Temperaturen. Die Reaktionen wurden auf Eis abgestoppt. Je 5 µl der Reaktionsansätze wurden in weiße membranbeschichtete Mikrotiterplatten (Pall BioSupport, SM045BWP) pipetiert und 10 Minuten bei 70°C gebacken. Die Membran der Mikrotiterplatte wurde unter Verwendung der zugehörigen Absaugwanne (Pall BioSupport) wie folgt behandelt: 100 µl Puffer 1 (1%iges Blocking Reagenz (Boehringer Mannheim) in 0,1 M Maleinsäure, 0,15 M NaCl, pH 7,5) auftragen, zwei Minuten inkubieren, durchsaugen, einmal wiederholen; 100 µl Puffer 2 (1: 10000 verdünnter Anti-Dig-AP-Fab-Fragment Antikörpern (Boehringer Mannheim) in Puffer 1) auftragen, zwei Minuten inkubieren, durchsaugen, einmal wiederholen; 200 µl Puffer 3 (Puffer 1 mit 0,3 %Tween 20) unter Vakuum auftragen, einmal wiederholen; 200 µl Puffer 4 (0,1 M Tris-HCL, 0,1 M NaCl, 50 mM MgCl₂, pH 9,5) unter Vakuum auftragen; 50 µl Puffer 5 (1:100 verdünntes CSPD (Boehringer Mannheim) in Puffer 4) auftragen, fünf Minuten inkubieren, durchsaugen. Die Proben wurden im Luminometer (Microlumar LB 96P, Berthold oder Wallac Micro Beta Trilux) vermessen.

Beim radioaktiven Testsystem wurde die Einbaurate von α-[³²P]dCTP in 1 µg M13 mp9 ss-DNA bestimmt. Ein 50 µl Testmix enthielt 5 µl Puffermix (670 mM Tris-HCl, 50 mM MgCl₂, 100 mM 2-Mercaptoethanol, 2% Tesit, 2 mg/ml Gelatine, pH 8.8), je 10 µM dATP, dGTP, dTTP, 5 µM CTP, 0,1 µCi [α-³²P]dCTP, 1 µg M13 mp 9 ss-DNA annealed mit 0,3 µg M13-Primer und 1 µl chimäres Enzym, oder 0.01 Units *Taq* Polymerase (Boehringer Mannheim), als Referenz in Verdünnungspuffer (20 mM Tris-HCl, pH 8,0, 100 mM KCl, 0,1 mM EDTA, 1 mM DTT, 200 µg/ml Gelatine, 0,5% Tween 20, 0,5% Nonidet P40, 50% Glyzerin). Zur Herstellung der DNA-Primermischung wurden 277,2 µg M 13 mp9 ss-DNA (Boehringer Mannheim) und 156 µg M 13-Sequenzierprimer (17 mer) für 30 Minuten auf 55°C erhitzt und 30 Minuten bei Raumtemperatur abgekühlt. Die Inkubation der Reaktionsansätze erfolgte für 30 Minuten bei 65°C. Die Reaktionen wurden auf Eis abgestoppt. Je 25 µl der Reaktionslösungen wurden entnommen und in 250 µl 10% Trichloressigsäure (TCA)/0,01 M Natriumpyrophosphat (PPᵢ) pipettiert, durchmischt und 30 Minuten auf Eis inkubiert. Die Proben wurden über vorgewässerten GFC-Filtern (Whatman) abgesaugt, die Reaktionsgefäße mit 5% TCA/PPi ausgewaschen und die Filter mindestens dreimal mit derselben Lösung gewaschen. Nach den Trocknen wurden die Filter in 5 ml Szintillationsflüssigkeit im β-Counter vermessen. Die Enzymproben wurden in Enzymverdünnungspuffer verdünnt. Von den Verdünnungen wurde jeweils 1 µl eingesetzt. Es wurden Doppel- oder Dreifachbestimmungen vorgenommen. Als Referenz wurde die *Taq* DNA Polymerase der Firma Boehringer Mannheim verwendet.

Eine Einheit (Unit) ist definiert als die Enzymmenge, die notwendig ist, um 10 nM Deoxyribonukleotidtriphosphat in säurefällbare DNA bei 65°C in 30 Minuten einzubauen. Zur Ermittlung der Standardwerte wurden je 2 µl der Gesamtmischung auf einen trockenen Filter pipettiert und getrocknet. Der Null-Wert wurde ermittelt, indem Proben ohne Enzym mitinkubiert und identisch gewaschen wurden.

Die Bestimmung der Temperaturoptima erfolgte mit dem nicht- radioaktiven DNA Polymerasetest bei verschiedenen Temperaturen.

**Spezifische Aktivitäten bei verschiedenen Temperaturen**

| Enzym | Temperatur [°C] | | | | | |
|---|---|---|---|---|---|---|
| | 25 | 37 | 50 | 60 | 72 | 80 |
| TaqPol (BM) | 0,0 | 0,0 | 5764,4 | 8489,1 | 50000,0 | 57986,1 |
| NHis-TaqPol | 0,0 | 0,0 | 5616,1 | 12165,2 | 60843,7 | 74784,4 |
| TaqEc1 | 704,9 | 10353,4 | 50066,5 | 41034,4 | 2677,5 | 1016,2 |
| TaqTne1 | 0,0 | 2559,4 | 15967,0 | 18900,4 | 1100,0 | 0,0 |
| TaqTne2 | 747,2 | 5180,2 | 23549,6 | 30627,3 | 64139,1 | 28727,4 |

### Beispiel 4: Temperaturstabilität der Polymeraseaktivität der Chimären

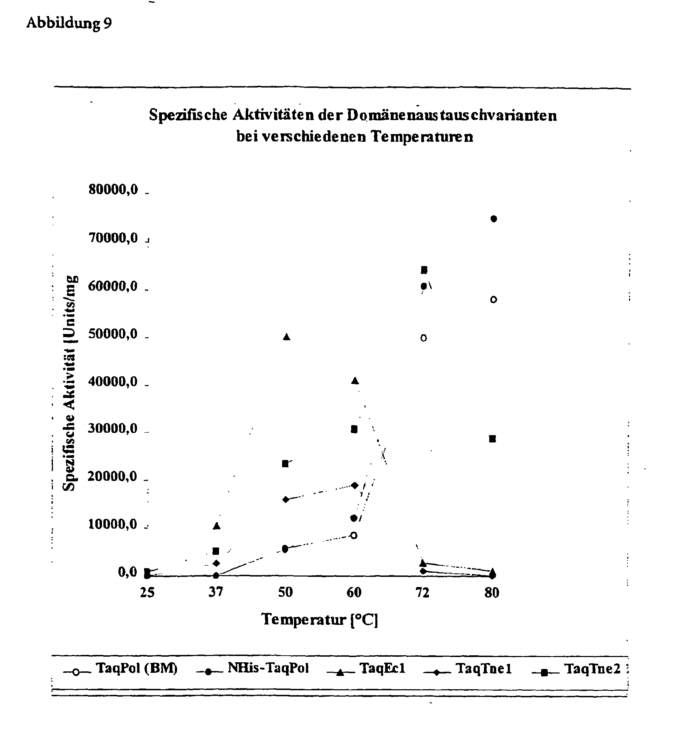

Die Bestimmung der Thermostabilität erfolgte durch Erhitzen der Reaktionsansätze auf 80°C und 95°C für jeweils eine, drei oder sechs Minuten mit anschließender Bestimmung der Restaktivitäten mit dem nicht-radioaktiven DNA Polymerasetest (siehe Abb. 9).

Tabelle: Restaktivitäten [Prozent der Ausgangsaktivität ohne Hitzebehandlung] bei 72°C der Taq DNA Polymerase (TaqPol), der Taq DNA Polymerase mit HisTag (NHis-TaqPol) und der drei Domänenaustauschvarianten (TaqEcl, TaqTne1, TaqTne2) nach der Hitzebehandlung (Einbau von Dig-dUTP in DN'ase aktivierte Kalbsthymus-DNA)

| | 1 Min | 3 Min | 6 Min | 1 Min | 3 Min | 6 Min |
|---|---|---|---|---|---|---|
| Enzym | 80°C | 80°C | 80°C | 95°C | 95°C | 95°C |
| TaqPol | 100 | 100 | 100 | 100 | 100 | 100 |
| NHis-TaqPol | 100 | 100 | 100 | 100 | 100 | 100 |
| TaqEc1 | 0 | 0 | 0 | 0 | 0 | 0 |
| TaqTne1 | 16 | 0 | 0 | 0 | 0 | 0 |
| TaqTne2 | 100 | 100 | 100 | 92 | 0 | 0 |

### Beispiel 5: PCR unter sukzessiver Enzymzugabe

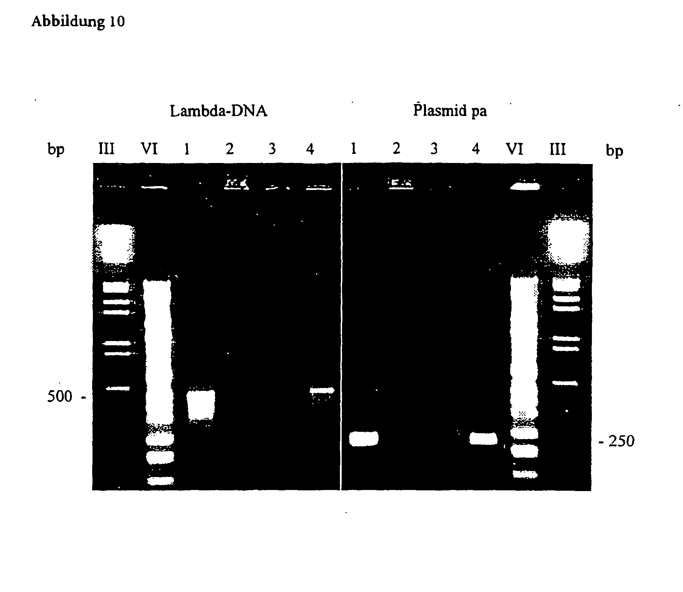

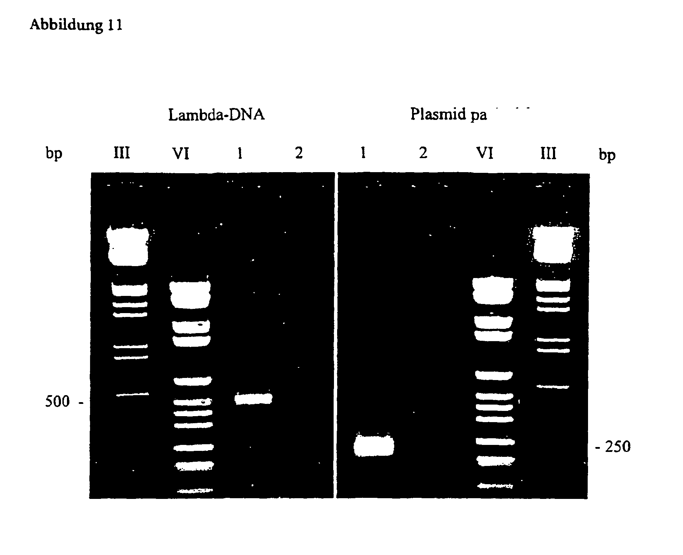

Die Polymerasenchimären wurden in der PCR unter sukzessiver Enzymzugabe getestet. Die Extension erfolgte bei 72°C (Abb. 10) und bei 55°C (Abb. 11). Der Reaktionansätze mit einem Reaktionsvolumen von 100 µl enthielten jeweils 1 ng Lambda-DNA oder pa-Plasmid-DNA (Fa. BM), je 1 µM Primer (25-mer), je 200 µM jedes dNTP's und Standard-PCR-Puffer mit MgCl₂ (Boehringer Mannheim). Die Reaktionsbedingen waren:

Für Extension bei 72°C: 1 Minute 94°C / 30 Sekunden 50°C / 1 Minute 72°C // 25 Zyklen, 2 Minuten 94°C vor und 7 Minuten 72°C nach der PCR-Raktion. Die Zugabe von 0,5 µl der Domänenaustauschvarianten pro Zyklus erfolgte jeweils bei 50°C.

Für Extension bei 55°C: 1 Minute 95°C / 30 Sekunden 50°C / 1 Minute 55°C // 25 Zyklen, 2 Minuten 95°C vor und 7 Minuten 55°C nach der PCR-Raktion. Die Zugabe von 0,5 µl der Domänenaustauschvarianten pro Zyklus erfolgte jeweils bei 50°C.

### Beispiel 6: 3'-5' Exonuklease-Test - Variante TaqEc1

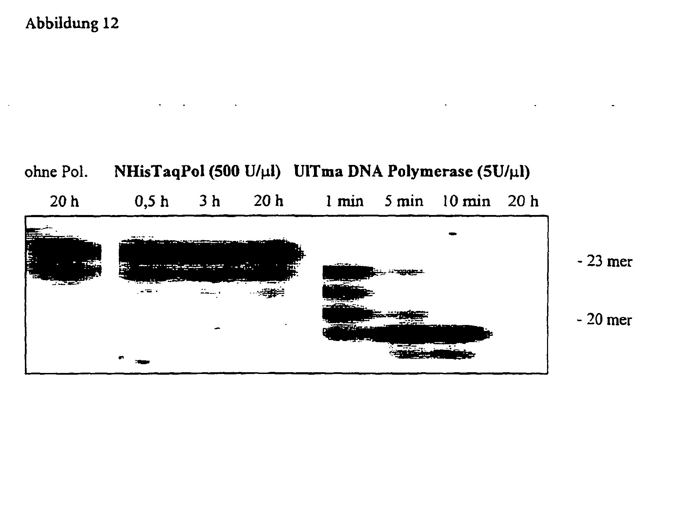

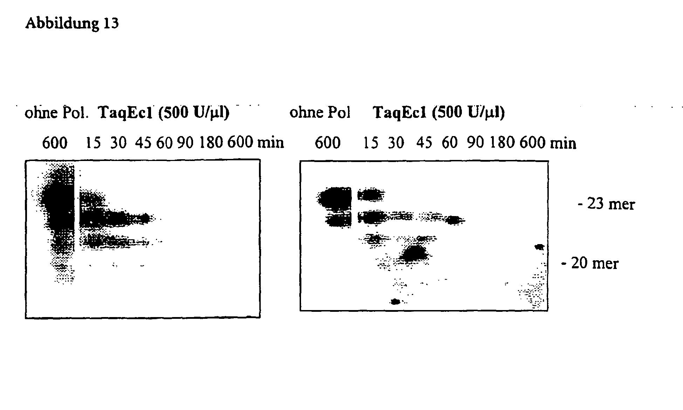

Die Proben wurden mit einem 5'-Dig-markierten Primer, der an einen DNA-Matrizenstrang annealt, in Abwesenheit von Nukleotiden inkubiert. Ein 10 µl Testmix enthielt 1 µl Puffer (100 mM Tris-HCL, 15 mM MgCl₂, 500 mM KCl, 0,1 mg/ml Gelatine, pH 8,3), 1 µl Enzym TaqEc1 (500 Einheiten/µl), 1 pMol Matrizenstrang (50mer, siehe Schema) und je 500 fMol 5'-Dig-markierten Primer P1 (matched, 23mer, siehe Schema) oder P2 (mismatched, 23mer, siehe Schema). Die Inkubation der Reaktionsansätze erfolgte bei 50°C mit unterschiedlicher Inkubationsdauer. Die DNA-Fragmente wurden auf einem 12,5 %igen Acrylamidgel (SequaGel-Kit, Firma Medco) aufgetrennt und durch Kontaktblot auf eine Nylonmembran (Boehringer Mannheim) übertragen. Die Nylonmembran wurde wie folgt behandelt: 100 ml Puffer 1 (1%iges Blocking Reagenz (Boehringer Mannheim) in 0,1 M Maleinsäure, 0,15 M NaCl, pH 7,5), 30 Minuten Inkubation; 100 ml Puffer 2 (1: 10000 verdünnter Anti-Dig-AP-Fab-Fragment Antikörper (Boehringer Mannheim) in Puffer 1), 30 Minuten Inkubation; je 135 ml Puffer 3 (Puffer 1 mit 0,3 %Tween 20), dreimal für 30 Minuten waschen; 50 ml Puffer 4 (0,1 M Tris-HCL, 0,1 M NaCl, 50 mM MgCl₂, pH 9,5), 5 Minuten Inkubation; 50 ml Puffer 5 (1: 1000 verdünntes CPD-Star (Boehringer Mannheim) in Puffer 4), 5 Minuten Inkubation. Die Nylonmembran wurde auf Watman-Papier getrocknet und zur Chemilumineszenz-Detektion für 30 bis 60 Minuten auf einem Chemilumineszenzfilm (Boehringer Mannheim) exponiert. Beim Vorhandensein einer 3'-5 Exonuklease wird der Abbau des Primer am 3'-Ende sichtbar (siehe Abbildungen). Als Negativkontrolle wurde die *Taq* Polymerase mit HisTag (NHis-TaqPol), und als Positivkontrolle die UlTma DNA Polymerase verwendet. Bei beiden Kontrollenzymen erfolgte die Inkubation der Reaktionsansätze bei 72°C. Für die UITma DNA Polymerase wurde der vom Hersteller angegebene Reaktionspuffer verwendet. Abb. 12 und 13 zeigen den 3'-5'-Exonuklease-Test-VarianteTaqEcl.

### Beispiel 7: Korrektur von 3'-mismatched Primern und deren Verlängerung - Variante TaqEc1 (3'-mismatch primer correction assay)

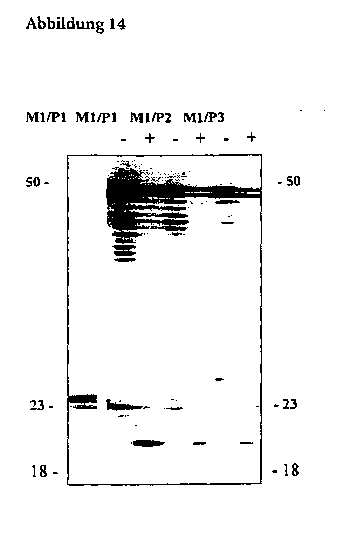

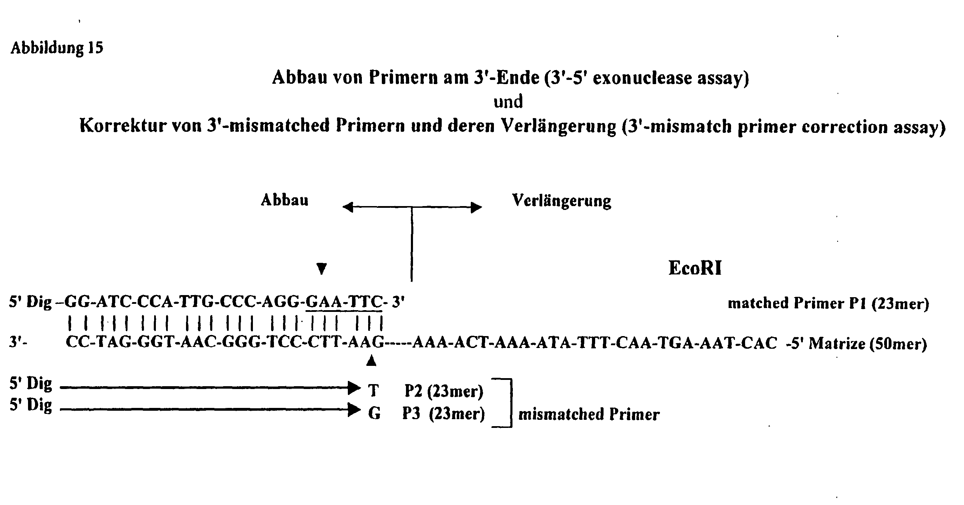

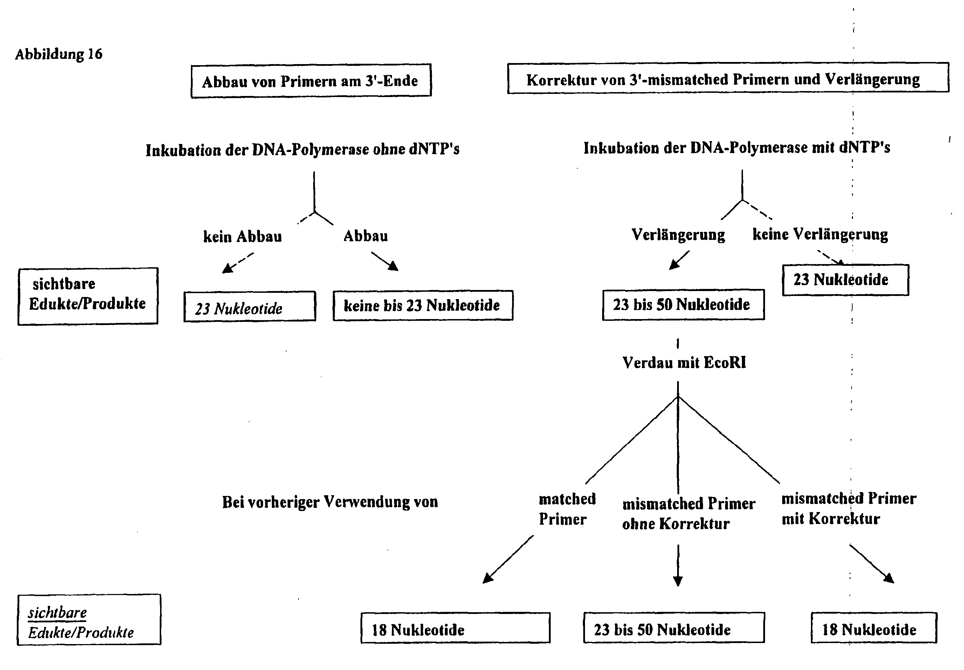

Dig-markierte Primer, die an einen Matrizenstrang (50 mer, siehe Schema) annealen wurden in vier verschiedenen Experimenten verlängert. Primer waren ein matched Primer (P1, 23 mer, siehe Schema) und zwei verschiedene mismatched Primer Primer (P2, P3, 23 mere, siehe Schema), die in der Erkennungssequenz des Restriktionsenzyms Eco RI annealen. Ein 20 µl Testmix enthielt 1 µl Puffer (100 mM Tris-HCL, 15 mM MgCl₂, 500 mM KCl, 0,1 mg/ml Gelatine, pH 8,3), 1 µl Enzym TaqEc1 (500 Einheiten/µl), je 10 µM dATP, dCTP, dGTP, dTTP, 1 pMol Matrizenstrang und je 500 fMol 5'-Dig-markierten Primer P1 (matched) oder P2 (mismatched) oder P3 (mismatched). Die Reaktionsansätze wurden für 60 Minuten bei 50°C inkubiert und danach für 5 Minuten bei 95°C erhitzt. Je 10 µl wurden entnommen und mit je 10 Einheiten Eco RI für 30 Minuten bei 37°C gespalten. Die DNA-Fragmente wurden auf einem 12,5 %igen Acrylamidgel (SequaGel-Kit, Firma Medco) aufgetrennt und durch Kontaktblot auf eine Nylonmembran (Boehringer Mannheim) übertragen. Die Nylonmembran wurde wie oben beschrieben behandelt und für 30 bis 60 Minuten auf einem Chemilumineszenzfilm (Boehringer Mannheim) exponiert. Bei der Verwendung des matched Primer resultiert der Verdau mit Eco RI in einem 28 bp und einem 18 bp Fragment. Die mismatched Primer liefern dieses Ergebnis nur dann, wenn mismatched Nukleotide durch matched Nukleotide ersetzt werden (siehe Abbildung 14).

### Beispiel 8: Modifikation einer rekombinanten DNA Polymerase

Design des hybriden Polymerasegens. Ath Pol und Tne Pol

### Computer Voraussage

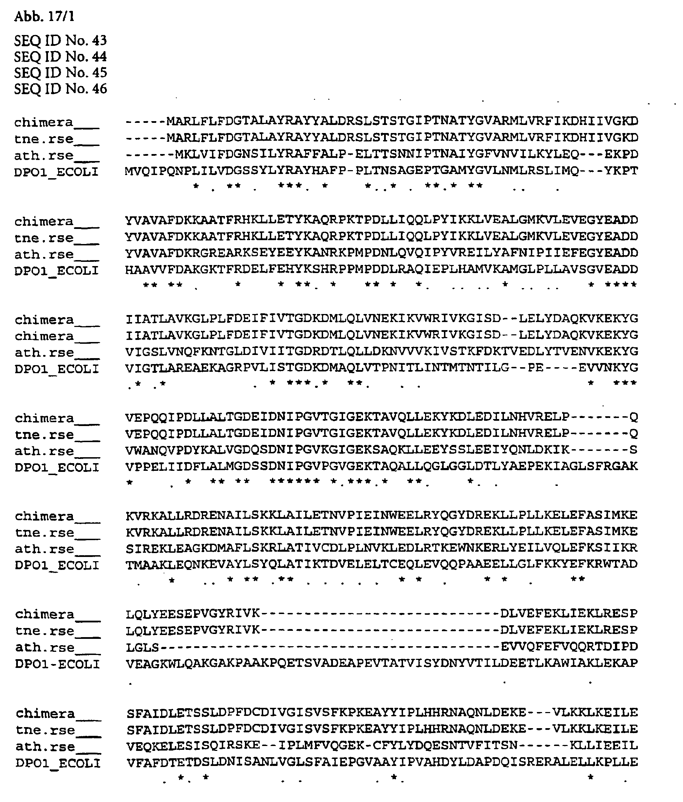

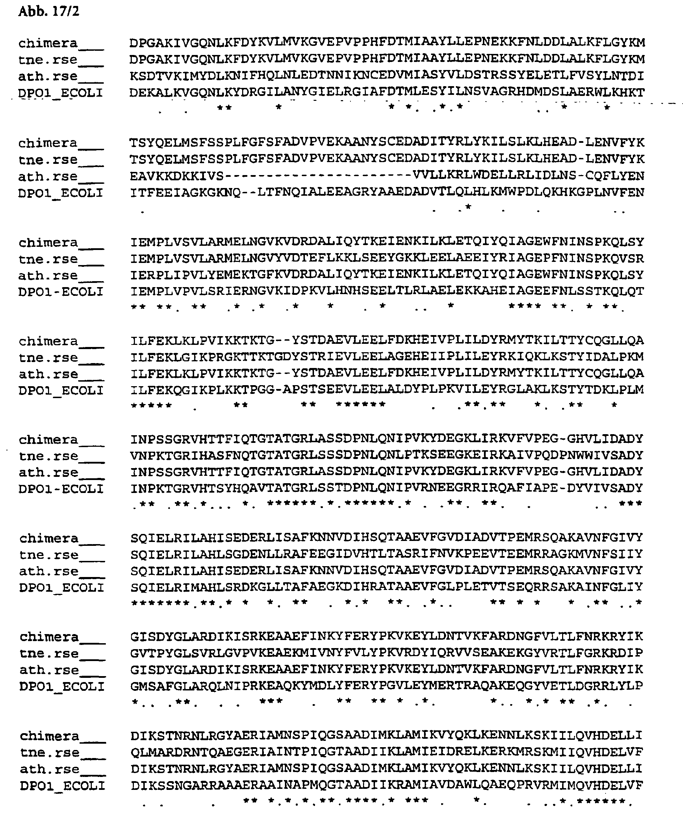

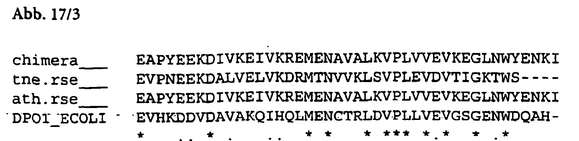

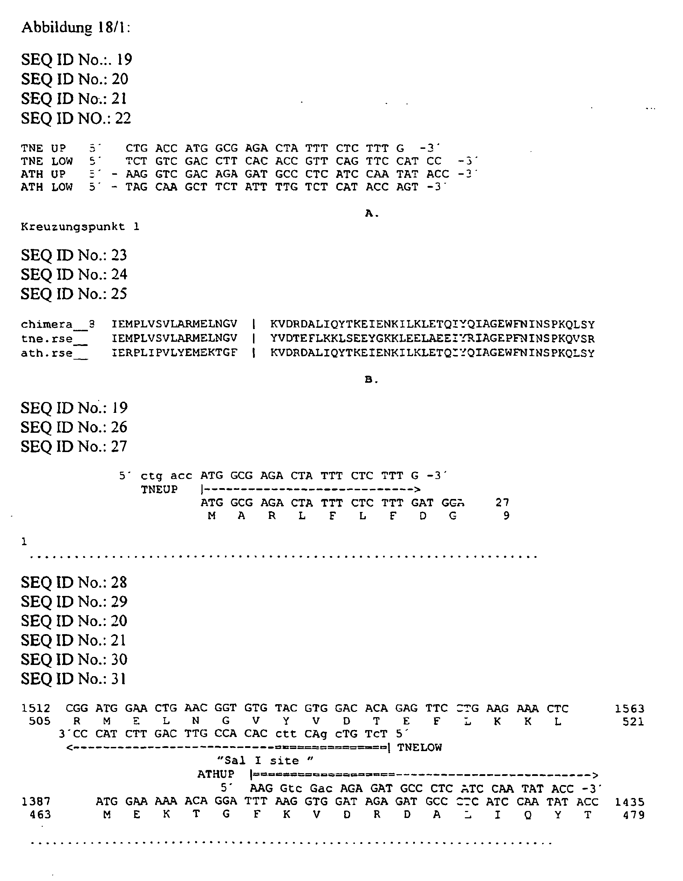

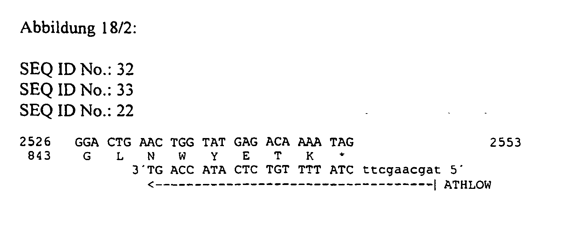

Die Struktur des chimären Polyemerasegens wurde hergeleitet aus dem Sequenz-Alignment (Thompson, J.D., Higgins, D.G. and Gibson, T.J. Nucleic Acids Research, 1994, 22: 4673-4680) zwischen den Polymerasen und dem E.Coli POLI Gen - die Sequenz mit der höchsten Übereinstimmung mit der gelösten 3D Struktur in der Datenbank von Brookhaven (für das Klenow Fragment). Die Paar-Alignments zeigten eine Übereinstimmung von ca. 40%, was zur Annahme Anlaß gibt, daß die 1KLN Struktur als best-möglicher Prototyp anzusehen ist. Um einen reibungslosen Übergang von einer Struktur zu anderen zu gewährleisten, sollte sich der Kreuzungspunkt, vom Gesichtspunkt des multiplen Alignments aus gesehen, in einem Gebiet befinden, welches eine hohe Ähnlichkeit mit allen drei Proteinen besitzt. Der Kreuzungspunkt sollte daher zwischen der Polymerase- und der 3'-5' Exonuklease Domäne liegen (Abbildung 17,18).

Bau eines hybriden Polymerasegens und Expressionsvektoren.
Computervoraussagen und - simulation dienten als Grundlage für den Bau eines hybriden Gens. Zum Erhalt der ATH POL und TNE EXO Domänen wurden PCR Amplifikation and Subcloning als Verfahren verwendet, wobei zwei Primerpaare mit den in Abb. 18 gezeigten Strukturen verwendet wurden. Primer besitzen Sequenzen, die für die N- und C-Enden der jeweiligen Gene sowie der Verbindungssequenz in der Mitte des Gens spezifisch sind, wie in Abb. 2 B, C zu sehen ist. Die Überlappung von 12 Basen in den ATHUP und TNELOW Primern wurde für die folgende Rekonstruktion des Hybdridgens angelegt und überdies in einer eindeutigen SalI Restriktionsstelle eingefügt, welche für weitere Veränderungen mit Polymerase-Domänen verwendet werden kann. Die Überhänge der 5'-Sequenz der TNEUP und ATHLOW Primer kodieren für die Restriktionsstellen NcolI und HindIII zum späteren Subcloning der erforderlichen Fragmente im Expressionsvektor.

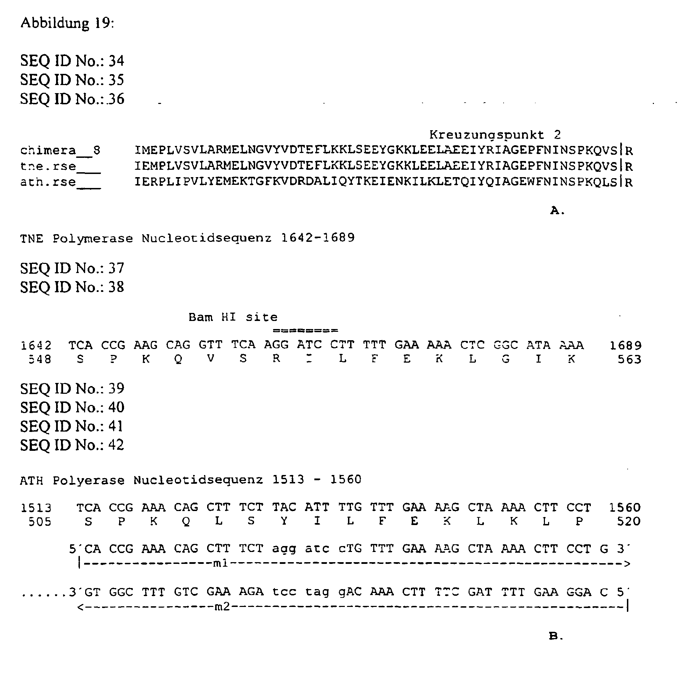

Die Verwendung dieser Strategie erfordert jedoch umfassendes Sequenzieren der subklonierten Regionen. Aus diesem Grunde wurde ein zusätzliches Konstrukt gebaut und die Spleißverbindung zwischen den Genen wurde an eine andere Position verschoben, nämlich 42 Aminosäuren weiter unterhalb der ursprünglichen Verbindungsposition zu einer Region zwischen den Polymerasen, die eine hohe Ähnlichkeit aufweist. Ein Vorteil des neuen Designs ist die eindeutige BamHI Sequenz innerhalb der die vorgeschlagene Spleißverbindung beeinhaltenden TNE Polymerase-Sequenz. Für die Konstruktion des Hybridgens, wurde der Einbau einer BamHI Sequenz in die ATH Polymerase Sequenz, die daraufhin für das Zusammenfügen von Teilen des Gens verwendet wird, mit einer gerichtete Mutagenese durchgeführt. Die Aminosäuren und Nukleotidsequenz der neuen Verbindung ist in Fig. 19 dargestellt.

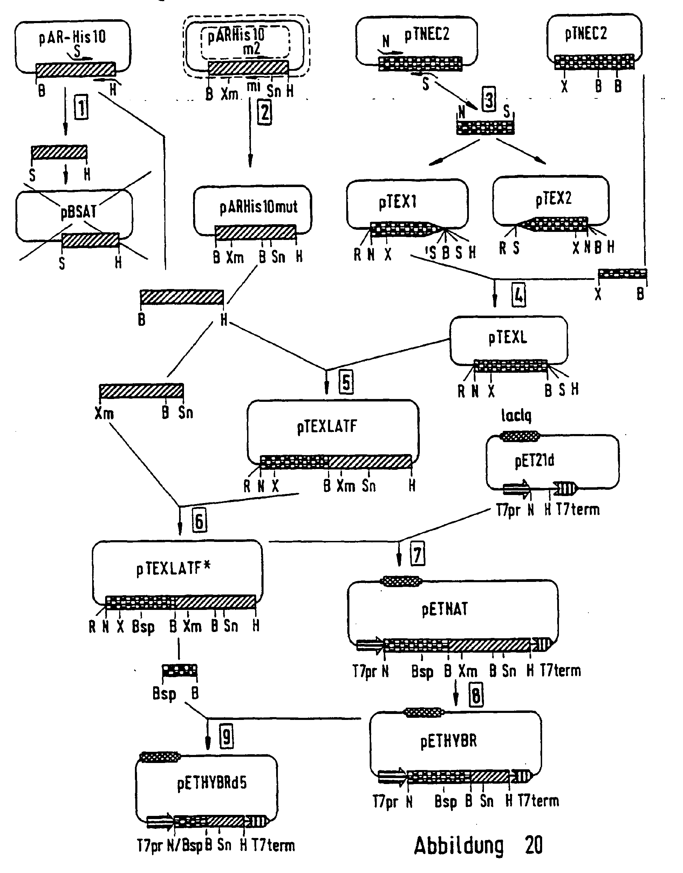

Die Konstruktion des hybriden Polymerasegens erfolgte wie in Fig. 20 beschrieben durch multiples Subkloning, gerichtete Mutagenese und Sequenzierungsschritte.

Alle durch die PCR-Amplifikation erhaltenen Fragmente wurden von den Enden her zu den eindeutigen, in weiteren Subkloningschritten verwendeten Restriktionstellen sequenziert. Um die Genauigkeit der Amplifikation sicherzustellen, wurden die PCR-Reaktionen mit Vent-Polymerase (New England Biolabs) durchgeführt. Die gerichtete Mutagenese erfolgte unter Anwendung des "Quick-Change" Verfahrens (Strategene).

### Beispiel 9: Expression eines hybriden Polymease Gens in E. coli

Die Plasmide pETHYBR und pETHYBRd5 wurden in dem E. coli Stamm BL21(DE3) plySS von Novagene transformiert und führten zur Expression von T7 Polymerase.

Die Expression des hybriden POL Gens wurde durch Messen der DNA Polymerase-Aktivität unter Verwendung des aktivierten DNA Assays in den Extrakten rekombinanten Stämme überwacht. Es galten die folgenden Bedingungen.
1) Die rekombinanten E.coli Stämme wurden in LB Medium mit 100 mcg/ml Ampicilin +30 mcg/ml Chloramphenicol (für pETHYBR und pETHYBRd5 in BL21 (DE3)plysS) oder in 20 ml LB Medium mit 100 mcg/ml Ampicilin + 30 mcg/ml Kanamycin (pARHYBd5 in JM 1 09/pSB 1611) angezüchtet.
2) Die Kulturen wurden bei 37°C auf eine optische Dicht von OD 550 ~ 0,6.0.7 geschüttelt; danach wurden die Kulturen auf 25°-28°C gekühlt, IPTG wurde hinzugefügt bis sich eine Endkonzentation von 1mM einstellte. Die Inkubation wurde dann bei 25-30°C fortgeführt: Für zwei pET Vektoren betrug die Dichte von nicht-induzierten Kulturen nach 4 Stunden Inkubation OD 550 ∼ 2,2 und bei induzierten Kulturen ~ 1,5.
3) Proteinextrakte von BL21(DE3)plysS Stämmen wurden durch Pelletierung von 5 ml Aliquoten der Kulturen hergestellt; die Resuspendierung des Zellpellets erfolgte dann in 100 µl Unterbrechungspuffer mit 40 mM Tris-HCl, pH 8,0, 0,1 mM EDTA, 7 mM 2.mercaptethanol, 0,2 mM PMSF, 0,1% Triton X-100. Die Zell-Extrakte wurden in zwei Zyklen durch Gefrieren und Auftauen der Zellsuspension in flüssigem Stickstoff/Warmwasserbad hergestellt; dann wurde die KCl Lösung hinzugefügt und ergab eine Endkonzentration von 0,75 M und die Extrakte der induzierten und nicht-induzierten Kulturen wurden 15 min lang bei 72° erhitzt, pelletiert und zur Messung der Polymeraseaktivität verwendet; dies erfolgte in dem aktivierten DNA Assay (100 mcg/ml aktivierter DNA, 3 mM MgSO4, 50 mM Tris-HCl, pH 8,9, 0,1%Triton X-100, 70 µM dA-P33, 5-10 µCi/ml) in 20µl Volumen unter Verwendung von 2 µl erhitzten Zellextrakten.

Die Ergebnisse sind in der nachfolgenden Tabelle festgehalten:

**Relative DNA Polymerase Aktivität in extrakten von rekombinanten stämmen (% Einbau von Markierungen, durchschnittlich 3 unabhängige Messungen)**

| Stamm | BL21(DE3)plyS | | | |
|---|---|---|---|---|
| Plasmid | pETHYBR | | pETHYBRd5 | |
| IPTG | - | + | - | + |
| TCA unlöslich r/a | 5 | 40 | 2 | 85 |

Aus diesen Daten geht hervor, daß beide Versionen des hybriden Polymerasegens mit dem pET Vektor System exprimiert werden konnten.

Charakterisierung der rekombinanten huybriden Polymerase.

### Thermostabilität

Die Wärmestabilität rekombinanter Polymerasen wurde duch Erhitzen des Extrakts des E. coli Stammes über verschiedenen Zeiträume (10, 30, 60, 120 Minuten) bei 95°C hinweg bestimmt. Es zeigte sich, daß die voll ausgebildete ebenso wie die verkürzte hybride Polymerase nicht ausreichend stabil waren (100% inaktiv nach 10 minütiger Inkubation bei 95°C. Der Expressionsgrad der rekombinanten Polymerasen wurde durch Analyse der erhitzten Zellextrakte in 10% SDS PAAG evaluiert; da zwischen den induzierten und nicht-induzierten Kulturen kein sichtbare Unterschied festgestellt werden konnte, läßt sich schließen, daß die Produktion hybrider Polymerasen 1% des gesamtem löslichen Proteines nicht überschrietet.

### Korrekturlese-Aktivität

Die Korrekturlese-Aktivität der von pETHYBRd5, z.B. Klenow Fragment, abgeleiteten rekombinanten DNA-Polymerase wurde gemäß dem gleichen Protokoll getestet, das auch für die archaeal DNA verwendet wurde. Es zeigte sich, daß das rekombinante Enzym Korrketurlese-Aktivitäten aufweist.

### Reverse Transkriptase Aktivität

Die folgende Reaktionsmischung wurde zur Bestimmung der reversen Transkriptase Aktivität verwendet: polydA-(dT)₁₅-1µg; TTP - 330 µM, digoxigenin-dUTP - 0,36 µM, BSA - 200µg/ml, Tris HCl, pH 8,5 - 10 mM, KCl - 20 mM. Die Konzentration von MgCl2 im Reaktionsgemisch variierte zwischen 0,5 und 10 mM. DTE wurde bei einer Konzentration 10 mM beigegeben.

2 µl rekombinante DNA-Polymerase (abgeleitet von pETHYBRd5, z.B. Klenow Fragment) wurde dem Reaktionsgemisch beigegeben und bei 50°C 15 min lang inkubiert. Tth DNA-Polymerase mit Mn²⁺ wurde als positive Kontrolle verwendet. Nach Anhalten der Reaktion, wurde das Gemisch auf positive gelandend Nylonmembrane (BM) aufgegeben. Der Nachweis des eingebauten Digoxigenin wurde gemäß BM Protokoll, 1995 durchgeführt.

Es zeigte sich, daß die rekombinanten Enzyme (Klenow-Fragment) reverse Transkriptase-Aktivität besitzen (Abb. 22). Die Aktivität ist abhängig von der Gegenwart von Mn²⁺ (optimale Konzentration 1 mM). Die Gegenwart von Mg²⁺ hatte darüber hinaus einen weiteren stimulierenden Effekt (optimale Mg²⁺ Konzentration 4 mM).

### Beispiel 11: Konstruktion des chimären Polymerasegens (sieh Abb. 20)

Abkürzung der Restriktionssequenzen - B-BamHI, Bsp-BspHI, H-HindIII, N-Ncol, R-EcoRI, S-Sall, Sn-Snal, X-Xhol, Xm-XmaI
1. PCR Amplifikation der ATH POL Domäne mit den Primern ATH UP und ATHLOW unter Verwendung des pARHis10 Plasmids mit dem kompletten Polymerasegen im Vektor pTrcHISB und Subcloning im pSK+ Bluescript Plasmid → pBSAT. Der Einschub wurde aus den flanking Primers sequenziert und stellte sich heraus, daß aufgrund eines Fehlers während der Primersynthese eine einzige Base in der ATHUP Primer Sequen gelöscht worden war.
2. Gerichtete Mutagenese des Plasmids pARHis10 mit den Primern m1 und m2 unter Verwendung der "Quick-change Procedure" (Stratagene) zum Einbau BamHI Sequenz an Position 1535 → pARHis10mut.
3. PCR Amplifikation der TNE EXO Domäne mit den Primern TNEUP und TNELOW auf der Matrize des pTNEC2 Plasmids und Subklonen im SmaI cut puC19 Plasmid → pTEX1 und pTEX2 mit unterschiedlich orientiertem Einbau.
4. Subklonen des 1444 bp XhoI-BamHI Fragmentes aus dem pTNEC2 Plasmid mit der "LONG" EXO Domäne im XhoI-BamHI cut Plasmid pTEX1 → pTEXL.
5. Einbau des kompletten ATH Polymerase Gens als 2553 bp BamHI-HindII Fragment in BamHI-HindIII cut pTEXL → pTEXLATF.
6. Subsitution des XmaI-SnaI Fragmentes des pTEXLATF Plasmids mit dem 1094 bp XmaI-SnaI Fragmentes aus dem pARHis 10mut Plasmids mit der eingebauten BamHI Sequenz → pTEXLATF*.
7. Einbau des 4214 bp NcoIHindII Fragmentes aus pTEXLATF* in den NcoI-HindII cut pET21d Vektor → pETNAT.
8. Löschen des für die N-terminale Domäne der ATH Polymerase kodierenden 1535 bp BamHI Fragmentes aus dem pETNAT Plasmid; dies führt zu einem "in-frame" Joining der TNE EXOL und ATH POL Sequenzen → pETHYBR.
9. Substitution des 1661 bp NcoI-BamHi Fragmentes von pETHYBR mit dem 829 bp BspHI-BamHI Fragment aus pETNAT; dies führt zur Verwendung von Met284 der TNE Polymerase als Anfangscodon und Löschen der N-terminalen Domäne mit der angenommenen 5'-3' Exonuklease Aktivität → pETHYBRd5.

## Patentansprüche

1. Polymerasenchimäre zusammengesetzt aus funktionellen Fragmenten von mindestens zwei unterschiedlichen Polymerasen, wobei die funktionellen Fragmente in der Polymerasenchimäre aktiv sind und, wobei die Polymeraseaktivität aufweisende Domäne von der ersten Polymerase stammt und die 3'-5'-Exonukleaseaktivität aufweisende Domäne von der zweiten Polymerase stammt und wobei die Aminosäuresequenz der Polymerasenchimäre der SEQ ID NO: 8 entspricht.

2. Polymerasenchimäre zusammengesetzt aus funktionellen Fragmenten von mindestens zwei unterschiedlichen Polymerasen, wobei die funktionellen Fragmente in der Polymerasenchimäre aktiv sind und, wobei die Polymeraseaktivität aufweisende Domäne von der ersten Polymerase stammt und die 3'-5'-Exonukleaseaktivität aufweisende Domäne von der zweiten Polymerase stammt und wobei die Aminosäuresequenz der Polymerasenchimäre der SEQ ID NO: 10 entspricht.

3. Polymerasenchimäre zusammengesetzt aus funktionellen Fragmenten von mindestens zwei unterschiedlichen Polymerasen, wobei die funktionellen Fragmente in der Polymerasenchimäre aktiv sind und, wobei die Polymeraseaktivität aufweisende Domäne von der ersten Polymerase stammt und die 3'-5'-Exonukleaseaktivität aufweisende Domäne von der zweiten Polymerase stammt und wobei die Aminosäuresequenz der Polymerasenchimäre der SEQ ID NO: 12 entspricht.

4. Polymerasenchimäre gemäß einem der Ansprüche 1-3, wobei die Chimäre zusätzlich RT-Aktivität aufweist.

5. Polymerasenchimäre gemäß einem der Ansprüche 1-4, wobei in die Aminosäuresequenz der Chimäre Histidin-tags eingebaut wurden.

6. DNA-Molekül einer Polymerasenchimäre gemäß einem der Ansprüche 1-5.

7. DNA-Molekül einer Polymerasenchimäre gemäß SEQ ID NO: 2.

8. DNA-Molekül einer Polymerasenchimäre gemäß SEQ ID NO: 4.

9. DNA-Molekül einer Polymerasenchimäre gemäß SEQ ID NO: 6.

10. Vektor enthaltend ein DNA-Molekül gemäß der Ansprüche 6-9.

11. Transformierte Zelle, die den Vektor gemäß Anspruch 10 enthält.

12. Verfahren zur Herstellung der Polymerasenchimären gemäß einem der Ansprüche 1-5, **dadurch gekennzeichnet, daß** das Verfahren die folgenden Schritte umfaßt:
- Planung von Varianten mit Hilfe von Aminosäuresequenzalignments, von 3D-Modellen oder mit Hilfe von experimentell ermittelten 3D-Strukturen
- gentechnische Herstellung der Domänenaustauschvarianten
- Legierung der DNA-Fragmente in Ausgangsvektoren
- Expression der Chimären in einem Wirt, der durch DNA-Fragment tragende Vektoren transformiert wurde
- Aufreinigung der exprimierten Polymerasenchimäre

13. Verwendung der Polymerasenchimären gemäß einem der Ansprüche 1-5 zu PCR.

14. Verwendung der Polymerasenchimäre gemäß einem der Ansprüche 1-5 zur Sequenzierung von DNA-Fragmenten.

15. Verwendung der Polymerasenchimäre gemäß einem der Ansprüche 1-5 zur RT-PCR ausgehend von einem RNA-template.

16. Kit enthaltend eine Polymerasenchimäre gemäß einem der Ansprüche 1-5.

## Claims

1. Polymerase chimera composed of functional fragments of at least two different polymerases, wherein the functional fragments are active in the polymerase chimera and wherein the domain having polymerase activity is derived from the first polymerase and the domain having 3'-5' exonuclease activity is derived from the second polymerase and wherein the amino acid sequence of the polymerase chimera corresponds to SEQ ID NO:8.

2. Polymerase chimera composed of functional fragments of at least two different polymerases, wherein the functional fragments are active in the polymerase chimera and wherein the domain having polymerase activity is derived from the first polymerase and the domain having 3'-5' exonuclease activity is derived from the second polymerase and wherein the amino acid sequence of the polymerase chimera corresponds to SEQ ID NO:10.

3. Polymerase chimera composed of functional fragments of at least two different polymerases, wherein the functional fragments are active in the polymerase chimera and wherein the domain having polymerase activity is derived from the first polymerase and the domain having 3'-5' exonuclease activity is derived from the second polymerase and wherein the amino acid sequence of the polymerase chimera corresponds to SEQ ID NO:12.

4. Polymerase chimera as claimed in one of the claims 1-3, wherein the chimera additionally has RT activity.

5. Polymerase chimera as claimed in one of the claims 1-4, wherein histidine tags have been incorporated into the amino acid sequence of the chimera.

6. DNA molecule of a polymerase chimera as claimed in one of the claims 1-5.

7. DNA molecule of a polymerase chimera according to SEQ ID NO.2.

8. DNA molecule of a polymerase chimera according to SEQ ID NO.4.

9. DNA molecule of a polymerase chimera according to SEQ ID NO.6.

10. Vector containing a DNA molecule as claimed in claims 6-9.

11. Transformed cell which contains the vector as claimed in claim 10.

12. Process for the production of the polymerase chimeras as claimed in one of the claims 1-5, wherein the process comprises the following steps:
- designing variants with the aid of amino acid sequence alignments, of 3D models or with the aid of experimentally determined 3D structures
- production of domain exchange variants by genetic engineering
- ligating the DNA fragments into starting vectors
- expression of the chimeras in a host which was transformed by vectors carrying DNA fragments
- purifying the expressed polymerase chimera.

13. Use of the polymerase chimeras as claimed in one of the claims 1-5 for PCR.

14. Use of the polymerase chimera as claimed in one of the claims 1-5 to sequence DNA fragments.

15. Use of the polymerase chimera as claimed in one of the claims 1-5 for RT-PCR starting with an RNA template.

16. Kit containing a polymerase chimera as claimed in one of the claims 1-5.

## Revendications

1. Chimère de polymérase constituée de fragments fonctionnels d'au moins deux polymérases différentes, où les fragments fonctionnels dans la chimère de polymérase sont actifs, et le domaine présentant l'activité de polymérase provient de la première polymérase, et le domaine présentant l'activité de 3'-5'-exonucléase provient de la seconde polymérase, et où la séquence d'acides aminés de la chimère de polymérase correspond à la SEQ ID NO : 8.

2. Chimère de polymérase constituée de fragments fonctionnels d'au moins deux polymérases différentes, où les fragments fonctionnels dans la chimère de polymérase sont actifs, et où le domaine présentant l'activité de polymérase provient de la première polymérase et le domaine présentant l'activité de 3'-5'-exonucléase provient de la seconde polymérase, et où la séquence d'acides aminés de la chimère de polymérase correspond à la SEQ ID NO : 10.

3. Chimère de polymérase constituée de fragments fonctionnels d'au moins deux polymérases différentes, où les fragments fonctionnels dans la chimère de polymérase sont actifs, et où le domaine présentant l'activité de polymérase provient de la première polymérase et le domaine présentant l'activité de 3'-5'-exonucléase provient de la seconde polymérase, et où la séquence d'acides aminés de la chimère de polymérase correspond à la SEQ ID NO : 12.

4. Chimère de polymérase selon l'une des revendications 1 à 3, dans laquelle la chimère présente en outre une activité de RT.

5. Chimère de polymérase selon l'une des revendications 1 à 4, dans laquelle, dans la séquence d'acides aminés de la chimère, des marqueurs histidine ont été intégrés.

6. Molécule d'ADN d'une chimère de polymérase selon l'une des revendications 1 à 5.

7. Molécule d'ADN d'une chimère de polymérase selon la SEQ ID NO : 2.

8. Molécule d'ADN d'une chimère de polymérase selon la SEQ ID NO : 4.

9. Molécule d'ADN d'une chimère de polymérase selon la SEQ ID NO : 6.

10. Vecteur contenant une molécule d'ADN selon les revendications 6 à 9.

11. Cellule transformée qui contient le vecteur selon la revendication 10.

12. Procédé de préparation de la chimère de polymérase selon l'une des revendications 1 à 5, **caractérisé en ce que** le procédé comprend les étapes suivantes :
- organisation de variantes à l'aide d'alignements de séquences d'acide aminés, de modèles 3D ou à l'aide de structures 3D déterminées expérimentalement ;
- production par génie génétique des variantes d'échange de domaine ;
- alliage des fragments d'ADN dans des vecteurs de départ ;
- expression des chimères chez un hôte qui a été transformé par des vecteurs portant un fragment d'ADN ;
- purification de la chimère de polymérase exprimée.

13. Utilisation de la chimère de polymérase selon l'une des revendications 1 à 5 pour la PCR.

14. Utilisation de la chimère de polymérase selon l'une des revendications 1 à 5 pour le séquençage de fragments d'ADN.

15. Utilisation de la chimère de polymérase selon l'une des revendications 1 à 5 pour la PCR RT en partant d'une matrice d'ARN.

16. Nécessaire contenant une chimère de polymérase selon l'une des revendications 1 à 5.
